# EUROPEAN PATENT APPLICATION

(11) **EP 4 477 669 A1**
(43) Date of publication of application: **18.12.2024**
(21) Application number: 23749363.0
(22) Date of filing: 07.02.2023
(51) Int. Cl.: C07K 16/46, C07K 16/28, C12N 15/13, A61K 39/395, A61P 35/00

(54) **ANTIGEN-BINDING MOLECULE SPECIFICALLY BINDING TO PSMA AND CD3, AND PHARMACEUTICAL USE THEREOF**

(30) Priority: 07.02.2022 CN 202210116684
(71) Applicant: Jiangsu Hengrui Pharmaceuticals Co., Ltd., Lianyungang, Jiangsu 222047 (CN); Shanghai Hengrui Pharmaceutical Co., Ltd., Shanghai 200245 (CN)
(72) Inventor: YING, Hua, Shanghai 200245 (CN); SHI, Jinping, Shanghai 200245 (CN); YANG, Hao, Shanghai 200245 (CN); ZHANG, Xiaomin, Shanghai 200245 (CN); JIN, Xinsheng, Shanghai 200245 (CN); TAO, Weikang, Shanghai 200245 (CN)
(74) Representative: Dragotti & Associati S.R.L.
(86) International application number: PCT/CN2023/074735
(87) International publication number: WO 2023/147784

(57) **Abstract**

Provided are an antigen-binding molecule specifically binding to PSMA and CD3, and a pharmaceutical use thereof. The antigen-binding molecule can be used for treating tumor-related diseases.

## Description

The present application claims priority to Chinese Patent Application No. 202210116684.6 filed on Feb. 7, 2022.

### TECHNICAL FIELD

The present disclosure belongs to the field of biotechnology, and specifically, the present disclosure relates to an antigen-binding molecule and use thereof.

### BACKGROUND

The statements herein merely provide background information related to the present disclosure and may not necessarily constitute the prior art.

PSMA, belonging to glutamate carboxypeptidase II (GCPII), consists of 750 amino acids, including 19 intracellular amino acids, 24 transmembrane amino acids, and 707 extracellular amino acids. The extracellular portion of PSMA consists of 3 domains: a protease-like domain, an apical domain, and a C-terminal domain. All three domains are involved in substrate binding. The protease-like and apical domains directly bind to the substrate, and the C-terminal domain causes PSMA to form a dimer to fulfill its function. In prostate cancer patients, the expression level of PSMA in tumor tissues is 100-1000 times higher than in the normal prostate. PSMA is also expressed on the surface of endometrial cancer cells, in the neovasculature of gastric cancer (66.4%) and colorectal cancer (84.6%), in the neovasculature of non-small cell lung cancer, and on some tumor cells. Therefore, PSMA can serve as a development target for prostate cancer and may also apply to other tumor types.

CD3 is a homodimeric or heterodimeric antigen expressed on T cells. Functional CD3 is formed by dimer binding of two of four different chains: ε, ζ, δ, and γ. CD3 dimer arrangements include γ/ε, δ/ε, and ζ/ζ. CD3 binds to the T-cell receptor complex (TCR) and is essential for T-cell activation. Therefore, anti-CD3 antibodies that activate T cells have been proposed for therapeutic purposes. However, administration of anti-CD3 antibodies may trigger T-cell activation and the release of related cytokines. Excessive cytokine release leads to severe cytokine release syndrome (CRS), which is a significant challenge in the clinical use of anti-CD3 antibodies. Therefore, there is an unmet need for PSMA/CD3 bispecific antibodies with high activity and low cytokine release.

### SUMMARY

The present disclosure provides an antigen-binding molecule that specifically binds to PSMA and CD3 and an antibody that specifically binds to PSMA.

In one aspect, the present disclosure provides an antigen-binding molecule comprising at least one antigen-binding moiety that specifically binds to PSMA and at least one antigen-binding moiety that specifically binds to CD3, wherein the antigen-binding moiety that specifically binds to PSMA comprises a heavy chain variable region (PSMA-VH) and a light chain variable region (PSMA-VL), and the antigen-binding moiety that specifically binds to CD3 comprises a heavy chain variable region (CD3-VH) and a light chain variable region (CD3-VL).

In some embodiments, provided is the antigen-binding molecule according to the above, wherein
(i) PSMA-HCDR1, PSMA-HCDR2, and PSMA-HCDR3 in the PSMA-VH comprise the amino acid sequences of PSMA-HCDR1, PSMA-HCDR2, and PSMA-HCDR3 in SEQ ID NO: 77, respectively, and PSMA-LCDR1, PSMA-LCDR2, and PSMA-LCDR3 in the PSMA-VL comprise the amino acid sequences of PSMA-LCDR1, PSMA-LCDR2, and PSMA-LCDR3 in SEQ ID NO: 78, respectively, or
(ii) PSMA-HCDR1, PSMA-HCDR2, and PSMA-HCDR3 in the PSMA-VH comprise the amino acid sequences of PSMA-HCDR1, PSMA-HCDR2, and PSMA-HCDR3 in SEQ ID NO: 73, respectively, and PSMA-LCDR1, PSMA-LCDR2, and PSMA-LCDR3 in the PSMA-VL comprise the amino acid sequences of PSMA-LCDR1, PSMA-LCDR2, and PSMA-LCDR3 in SEQ ID NO: 74, respectively, or
(iii) PSMA-HCDR1, PSMA-HCDR2, and PSMA-HCDR3 in the PSMA-VH comprise the amino acid sequences of PSMA-HCDR1, PSMA-HCDR2, and PSMA-HCDR3 in SEQ ID NO: 75, respectively, and PSMA-LCDR1, PSMA-LCDR2, and PSMA-LCDR3 in the PSMA-VL comprise the amino acid sequences of PSMA-LCDR1, PSMA-LCDR2, and PSMA-LCDR3 in SEQ ID NO: 76, respectively, or
(iv) PSMA-HCDR1, PSMA-HCDR2, and PSMA-HCDR3 in the PSMA-VH comprise the amino acid sequences of PSMA-HCDR1, PSMA-HCDR2, and PSMA-HCDR3 in SEQ ID NO: 79, respectively, and PSMA-LCDR1, PSMA-LCDR2, and PSMA-LCDR3 in the PSMA-VL comprise the amino acid sequences of PSMA-LCDR1, PSMA-LCDR2, and PSMA-LCDR3 in SEQ ID NO: 80, respectively, or
(v) PSMA-HCDR1, PSMA-HCDR2, and PSMA-HCDR3 in the PSMA-VH comprise the amino acid sequences of PSMA-HCDR1, PSMA-HCDR2, and PSMA-HCDR3 in SEQ ID NO: 81, respectively, and PSMA-LCDR1, PSMA-LCDR2, and PSMA-LCDR3 in the PSMA-VL comprise the amino acid sequences of PSMA-LCDR1, PSMA-LCDR2, and PSMA-LCDR3 in SEQ ID NO: 82, respectively, or
(vi) PSMA-HCDR1, PSMA-HCDR2, and PSMA-HCDR3 in the PSMA-VH comprise the amino acid sequences of PSMA-HCDR1, PSMA-HCDR2, and PSMA-HCDR3 in SEQ ID NO: 83, respectively, and PSMA-LCDR1, PSMA-LCDR2, and PSMA-LCDR3 in the PSMA-VL comprise the amino acid sequences of PSMA-LCDR1, PSMA-LCDR2, and PSMA-LCDR3 in SEQ ID NO: 84, respectively, or
(vii) PSMA-HCDR1, PSMA-HCDR2, and PSMA-HCDR3 in the PSMA-VH comprise the amino acid sequences of PSMA-HCDR1, PSMA-HCDR2, and PSMA-HCDR3 in SEQ ID NO: 85, respectively, and PSMA-LCDR1, PSMA-LCDR2, and PSMA-LCDR3 in the PSMA-VL comprise the amino acid sequences of PSMA-LCDR1, PSMA-LCDR2, and PSMA-LCDR3 in SEQ ID NO: 86, respectively, or
(viii) PSMA-HCDR1, PSMA-HCDR2, and PSMA-HCDR3 in the PSMA-VH comprise the amino acid sequences of PSMA-HCDR1, PSMA-HCDR2, and PSMA-HCDR3 in SEQ ID NO: 87, respectively, and PSMA-LCDR1, PSMA-LCDR2, and PSMA-LCDR3 in the PSMA-VL comprise the amino acid sequences of PSMA-LCDR1, PSMA-LCDR2, and PSMA-LCDR3 in SEQ ID NO: 88, respectively, or
(ix) PSMA-HCDR1, PSMA-HCDR2, and PSMA-HCDR3 in the PSMA-VH comprise the amino acid sequences of PSMA-HCDR1, PSMA-HCDR2, and PSMA-HCDR3 in SEQ ID NO: 89, respectively, and PSMA-LCDR1, PSMA-LCDR2, and PSMA-LCDR3 in the PSMA-VL comprise the amino acid sequences of PSMA-LCDR1, PSMA-LCDR2, and PSMA-LCDR3 in SEQ ID NO: 90, respectively, or
(x) PSMA-HCDR1, PSMA-HCDR2, and PSMA-HCDR3 in the PSMA-VH comprise the amino acid sequences of PSMA-HCDR1, PSMA-HCDR2, and PSMA-HCDR3 in SEQ ID NO: 91, respectively, and PSMA-LCDR1, PSMA-LCDR2, and PSMA-LCDR3 in the PSMA-VL comprise the amino acid sequences of PSMA-LCDR1, PSMA-LCDR2, and PSMA-LCDR3 in SEQ ID NO: 92 or 152, respectively, or
(xi) PSMA-HCDR1, PSMA-HCDR2, and PSMA-HCDR3 in the PSMA-VH comprise the amino acid sequences of PSMA-HCDR1, PSMA-HCDR2, and PSMA-HCDR3 in SEQ ID NO: 93, respectively, and PSMA-LCDR1, PSMA-LCDR2, and PSMA-LCDR3 in the PSMA-VL comprise the amino acid sequences of PSMA-LCDR1, PSMA-LCDR2, and PSMA-LCDR3 in SEQ ID NO: 94, respectively, or
(xii) PSMA-HCDR1, PSMA-HCDR2, and PSMA-HCDR3 in the PSMA-VH comprise the amino acid sequences of PSMA-HCDR1, PSMA-HCDR2, and PSMA-HCDR3 in SEQ ID NO: 95, respectively, and PSMA-LCDR1, PSMA-LCDR2, and PSMA-LCDR3 in the PSMA-VL comprise the amino acid sequences of PSMA-LCDR1, PSMA-LCDR2, and PSMA-LCDR3 in SEQ ID NO: 96, respectively.

In some embodiments, the PSMA-HCDR1, PSMA-HCDR2, PSMA-HCDR3, PSMA-LCDR1, PSMA-LCDR2, and PSMA-LCDR3 are defined according to the Kabat, IMGT, Chothia, AbM, or Contact numbering scheme.

In some embodiments, provided is the antigen-binding molecule according to any one of the above, wherein
(i) the PSMA-VH has: a PSMA-HCDR1 comprising the amino acid sequence of SEQ ID NO: 13, a PSMA-HCDR2 comprising the amino acid sequence of SEQ ID NO: 14, and a PSMA-HCDR3 comprising the amino acid sequence of SEQ ID NO: 15, and the PSMA-VL has: a PSMA-LCDR1 comprising the amino acid sequence of SEQ ID NO: 16, a PSMA-LCDR2 comprising the amino acid sequence of SEQ ID NO: 17, and a PSMA-LCDR3 comprising the amino acid sequence of SEQ ID NO: 18, or
(ii) the PSMA-VH has: a PSMA-HCDR1 comprising the amino acid sequence of SEQ ID NO: 1, a PSMA-HCDR2 comprising the amino acid sequence of SEQ ID NO: 2, and a PSMA-HCDR3 comprising the amino acid sequence of SEQ ID NO: 3, and the PSMA-VL has: a PSMA-LCDR1 comprising the amino acid sequence of SEQ ID NO: 4, a PSMA-LCDR2 comprising the amino acid sequence of SEQ ID NO: 5, and a PSMA-LCDR3 comprising the amino acid sequence of SEQ ID NO: 6, or
(iii) the PSMA-VH has: a PSMA-HCDR1 comprising the amino acid sequence of SEQ ID NO: 7, a PSMA-HCDR2 comprising the amino acid sequence of SEQ ID NO: 8, and a PSMA-HCDR3 comprising the amino acid sequence of SEQ ID NO: 9, and the PSMA-VL has: a PSMA-LCDR1 comprising the amino acid sequence of SEQ ID NO: 10, a PSMA-LCDR2 comprising the amino acid sequence of SEQ ID NO: 11, and a PSMA-LCDR3 comprising the amino acid sequence of SEQ ID NO: 12, or
(iv) the PSMA-VH has: a PSMA-HCDR1 comprising the amino acid sequence of SEQ ID NO: 19, a PSMA-HCDR2 comprising the amino acid sequence of SEQ ID NO: 20, and a PSMA-HCDR3 comprising the amino acid sequence of SEQ ID NO: 21, and the PSMA-VL has: a PSMA-LCDR1 comprising the amino acid sequence of SEQ ID NO: 22, a PSMA-LCDR2 comprising the amino acid sequence of SEQ ID NO: 23, and a PSMA-LCDR3 comprising the amino acid sequence of SEQ ID NO: 24, or
(v) the PSMA-VH has: a PSMA-HCDR1 comprising the amino acid sequence of SEQ ID NO: 25, a PSMA-HCDR2 comprising the amino acid sequence of SEQ ID NO: 26, and a PSMA-HCDR3 comprising the amino acid sequence of SEQ ID NO: 27, and the PSMA-VL has: a PSMA-LCDR1 comprising the amino acid sequence of SEQ ID NO: 28, a PSMA-LCDR2 comprising the amino acid sequence of SEQ ID NO: 29, and a PSMA-LCDR3 comprising the amino acid sequence of SEQ ID NO: 30, or
(vi) the PSMA-VH has: a PSMA-HCDR1 comprising the amino acid sequence of SEQ ID NO: 31, a PSMA-HCDR2 comprising the amino acid sequence of SEQ ID NO: 32, and a PSMA-HCDR3 comprising the amino acid sequence of SEQ ID NO: 33, and the PSMA-VL has: a PSMA-LCDR1 comprising the amino acid sequence of SEQ ID NO: 34, a PSMA-LCDR2 comprising the amino acid sequence of SEQ ID NO: 35, and a PSMA-LCDR3 comprising the amino acid sequence of SEQ ID NO: 36, or
(vii) the PSMA-VH has: a PSMA-HCDR1 comprising the amino acid sequence of SEQ ID NO: 37, a PSMA-HCDR2 comprising the amino acid sequence of SEQ ID NO: 38, and a PSMA-HCDR3 comprising the amino acid sequence of SEQ ID NO: 39, and the PSMA-VL has: a PSMA-LCDR1 comprising the amino acid sequence of SEQ ID NO: 40, a PSMA-LCDR2 comprising the amino acid sequence of SEQ ID NO: 41, and a PSMA-LCDR3 comprising the amino acid sequence of SEQ ID NO: 42, or
(viii) the PSMA-VH has: a PSMA-HCDR1 comprising the amino acid sequence of SEQ ID NO: 43, a PSMA-HCDR2 comprising the amino acid sequence of SEQ ID NO: 44, and a PSMA-HCDR3 comprising the amino acid sequence of SEQ ID NO: 45, and the PSMA-VL has: a PSMA-LCDR1 comprising the amino acid sequence of SEQ ID NO: 46, a PSMA-LCDR2 comprising the amino acid sequence of SEQ ID NO: 47, and a PSMA-LCDR3 comprising the amino acid sequence of SEQ ID NO: 48, or
(ix) the PSMA-VH has: a PSMA-HCDR1 comprising the amino acid sequence of SEQ ID NO: 49, a PSMA-HCDR2 comprising the amino acid sequence of SEQ ID NO: 50, and a PSMA-HCDR3 comprising the amino acid sequence of SEQ ID NO: 51, and the PSMA-VL has: a PSMA-LCDR1 comprising the amino acid sequence of SEQ ID NO: 52, a PSMA-LCDR2 comprising the amino acid sequence of SEQ ID NO: 53, and a PSMA-LCDR3 comprising the amino acid sequence of SEQ ID NO: 54, or
(x) the PSMA-VH has: a PSMA-HCDR1 comprising the amino acid sequence of SEQ ID NO: 55, a PSMA-HCDR2 comprising the amino acid sequence of SEQ ID NO: 56, and a PSMA-HCDR3 comprising the amino acid sequence of SEQ ID NO: 57, and the PSMA-VL has: a PSMA-LCDR1 comprising the amino acid sequence of SEQ ID NO: 58 or 159, a PSMA-LCDR2 comprising the amino acid sequence of SEQ ID NO: 59, and a PSMA-LCDR3 comprising the amino acid sequence of SEQ ID NO: 60, or
(xi) the PSMA-VH has: a PSMA-HCDR1 comprising the amino acid sequence of SEQ ID NO: 61, a PSMA-HCDR2 comprising the amino acid sequence of SEQ ID NO: 62, and a PSMA-HCDR3 comprising the amino acid sequence of SEQ ID NO: 63, and the PSMA-VL has: a PSMA-LCDR1 comprising the amino acid sequence of SEQ ID NO: 64, a PSMA-LCDR2 comprising the amino acid sequence of SEQ ID NO: 65, and a PSMA-LCDR3 comprising the amino acid sequence of SEQ ID NO: 66, or
(xii) the PSMA-VH has: a PSMA-HCDR1 comprising the amino acid sequence of SEQ ID NO: 67, a PSMA-HCDR2 comprising the amino acid sequence of SEQ ID NO: 68, and a PSMA-HCDR3 comprising the amino acid sequence of SEQ ID NO: 69, and the PSMA-VL has: a PSMA-LCDR1 comprising the amino acid sequence of SEQ ID NO: 70, a PSMA-LCDR2 comprising the amino acid sequence of SEQ ID NO: 71, and a PSMA-LCDR3 comprising the amino acid sequence of SEQ ID NO: 72.

In some embodiments, provided is the antigen-binding molecule according to any one of the above, wherein the PSMA-HCDR1, PSMA-HCDR2, PSMA-HCDR3, PSMA-LCDR1, PSMA-LCDR2, and PSMA-LCDR3 are defined according to the Kabat numbering scheme.

In some embodiments, provided is the antigen-binding molecule according to any one of the above, wherein the antigen-binding molecule binds to human PSMA at 25 °C with a KD of less than 1 × 10⁻⁷ M, 5 × 10⁻⁸ M, or 1 × 10⁻⁸ M, as measured by surface plasmon resonance.

In some specific embodiments, the antigen-binding molecule according to any one of the above binds to human PSMA at 25 °C with a KD of less than 9 × 10⁻⁹ M, 8 × 10⁻⁹ M, 7 × 10⁻⁹ M, 6 × 10⁻⁹ M, 5 × 10⁻⁹ M, 4 × 10⁻⁹ M, 3 × 10⁻⁹ M, 2.9 × 10⁻⁹ M, 2.8 × 10⁻⁹ M, 2.7 × 10⁻⁹ M, 2.6 × 10⁻⁹ M, 2.5 × 10⁻⁹ M, 2.4 × 10⁻⁹ M, 2.3 × 10⁻⁹ M, 2.2 × 10⁻⁹ M, 2.1 × 10⁻⁹ M, 2.0 × 10⁻⁹ M, 1.9 × 10⁻⁹ M, 1.8 × 10⁻⁹ M, 1.7 × 10⁻⁹ M, 1.6 × 10⁻⁹ M, 1.5 × 10⁻⁹ M, 1.4 × 10⁻⁹ M, as measured by surface plasmon resonance.

In some embodiments, provided is the antigen-binding molecule according to any one of the above, wherein:
(i) the PSMA-VH comprises the amino acid sequence of SEQ ID NO: 77, 107, 108, 109, 110, 111, or 112, and the PSMA-VL comprises the amino acid sequence of SEQ ID NO: 78, 113, 114, or 115, or
(ii) the PSMA-VH comprises the amino acid sequence of SEQ ID NO: 73 or 99, and the PSMA-VL comprises the amino acid sequence of SEQ ID NO: 74 or 100, or
(iii) the PSMA-VH comprises the amino acid sequence of SEQ ID NO: 75, 101, 102, or 103, and the PSMA-VL comprises the amino acid sequence of SEQ ID NO: 76, 104, 105, or 106, or
(iv) the PSMA-VH comprises the amino acid sequence of SEQ ID NO: 79, 116, or 117, and the PSMA-VL comprises the amino acid sequence of SEQ ID NO: 80, 118, or 119, or
(v) the PSMA-VH comprises the amino acid sequence of SEQ ID NO: 81 or 120, and the PSMA-VL comprises the amino acid sequence of SEQ ID NO: 82, 121, 122, 123, 124, 125, or 126, or
(vi) the PSMA-VH comprises the amino acid sequence of SEQ ID NO: 83 or 127, and the PSMA-VL comprises the amino acid sequence of SEQ ID NO: 84, 128, 129, or 130, or
(vii) the PSMA-VH comprises the amino acid sequence of SEQ ID NO: 85, 131, or 132, and the PSMA-VL comprises the amino acid sequence of SEQ ID NO: 86, 133, 134, or 135, or
(viii) the PSMA-VH comprises the amino acid sequence of SEQ ID NO: 87, 136, 137, or 138, and the PSMA-VL comprises the amino acid sequence of SEQ ID NO: 88, 139, or 140, or
(ix) the PSMA-VH comprises the amino acid sequence of SEQ ID NO: 89, 141, 142, 143, or 144, and the PSMA-VL comprises the amino acid sequence of SEQ ID NO: 90, 145, 146, or 147, or
(x) the PSMA-VH comprises the amino acid sequence of SEQ ID NO: 91, 148, or 149, and the PSMA-VL comprises the amino acid sequence of SEQ ID NO: 92, 150, 151, or 152, or
(xi) the PSMA-VH comprises the amino acid sequence of SEQ ID NO: 93, 153, 154, 155, or 156, and the PSMA-VL comprises the amino acid sequence of SEQ ID NO: 94, 157, or 158, or
(xii) the PSMA-VH comprises the amino acid sequence of SEQ ID NO: 95, 277, 278, or 279, and the PSMA-VL comprises the amino acid sequence of SEQ ID NO: 96, 280, 281, 282, or 283.

In some embodiments, provided is the antigen-binding molecule according to any one of the above, wherein
(i) the PSMA-VH comprises the amino acid sequence of SEQ ID NO: 112, and the PSMA-VL comprises the amino acid sequence of SEQ ID NO: 115, or
   the PSMA-VH comprises the amino acid sequence of SEQ ID NO: 110, and the PSMA-VL comprises the amino acid sequence of SEQ ID NO: 114, or
   the PSMA-VH comprises the amino acid sequence of SEQ ID NO: 77, and the PSMA-VL comprises the amino acid sequence of SEQ ID NO: 78, or
   the PSMA-VH comprises the amino acid sequence of SEQ ID NO: 107, and the PSMA-VL comprises the amino acid sequence of SEQ ID NO: 113, or
   the PSMA-VH comprises the amino acid sequence of SEQ ID NO: 108, and the PSMA-VL comprises the amino acid sequence of SEQ ID NO: 113, or
   the PSMA-VH comprises the amino acid sequence of SEQ ID NO: 109, and the PSMA-VL comprises the amino acid sequence of SEQ ID NO: 113, or
   the PSMA-VH comprises the amino acid sequence of SEQ ID NO: 110, and the PSMA-VL comprises the amino acid sequence of SEQ ID NO: 113, or
   the PSMA-VH comprises the amino acid sequence of SEQ ID NO: 111, and the PSMA-VL comprises the amino acid sequence of SEQ ID NO: 113, or
   the PSMA-VH comprises the amino acid sequence of SEQ ID NO: 107, and the PSMA-VL comprises the amino acid sequence of SEQ ID NO: 114, or
   the PSMA-VH comprises the amino acid sequence of SEQ ID NO: 108, and the PSMA-VL comprises the amino acid sequence of SEQ ID NO: 114, or
   the PSMA-VH comprises the amino acid sequence of SEQ ID NO: 109, and the PSMA-VL comprises the amino acid sequence of SEQ ID NO: 114, or
   the PSMA-VH comprises the amino acid sequence of SEQ ID NO: 111, and the PSMA-VL comprises the amino acid sequence of SEQ ID NO: 114, or
(ii) the PSMA-VH comprises the amino acid sequence of SEQ ID NO: 73, and the PSMA-VL comprises the amino acid sequence of SEQ ID NO: 74, or
   the PSMA-VH comprises the amino acid sequence of SEQ ID NO: 99, and the PSMA-VL comprises the amino acid sequence of SEQ ID NO: 100, or
(iii) the PSMA-VH comprises the amino acid sequence of SEQ ID NO: 75, and the PSMA-VL comprises the amino acid sequence of SEQ ID NO: 76, or
   the PSMA-VH comprises the amino acid sequence of SEQ ID NO: 101, and the PSMA-VL comprises the amino acid sequence of SEQ ID NO: 104, or
   the PSMA-VH comprises the amino acid sequence of SEQ ID NO: 102, and the PSMA-VL comprises the amino acid sequence of SEQ ID NO: 104, or
   the PSMA-VH comprises the amino acid sequence of SEQ ID NO: 103, and the PSMA-VL comprises the amino acid sequence of SEQ ID NO: 104, or
   the PSMA-VH comprises the amino acid sequence of SEQ ID NO: 101, and the PSMA-VL comprises the amino acid sequence of SEQ ID NO: 105, or
   the PSMA-VH comprises the amino acid sequence of SEQ ID NO: 102, and the PSMA-VL comprises the amino acid sequence of SEQ ID NO: 105, or
   the PSMA-VH comprises the amino acid sequence of SEQ ID NO: 103, and the PSMA-VL comprises the amino acid sequence of SEQ ID NO: 105, or
   the PSMA-VH comprises the amino acid sequence of SEQ ID NO: 101, and the PSMA-VL comprises the amino acid sequence of SEQ ID NO: 106, or
   the PSMA-VH comprises the amino acid sequence of SEQ ID NO: 102, and the PSMA-VL comprises the amino acid sequence of SEQ ID NO: 106, or
   the PSMA-VH comprises the amino acid sequence of SEQ ID NO: 103, and the PSMA-VL comprises the amino acid sequence of SEQ ID NO: 106, or
(iv) the PSMA-VH comprises the amino acid sequence of SEQ ID NO: 79, and the PSMA-VL comprises the amino acid sequence of SEQ ID NO: 80, or
   the PSMA-VH comprises the amino acid sequence of SEQ ID NO: 116, and the PSMA-VL comprises the amino acid sequence of SEQ ID NO: 118, or
   the PSMA-VH comprises the amino acid sequence of SEQ ID NO: 117, and the PSMA-VL comprises the amino acid sequence of SEQ ID NO: 118, or
   the PSMA-VH comprises the amino acid sequence of SEQ ID NO: 116, and the PSMA-VL comprises the amino acid sequence of SEQ ID NO: 119, or
   the PSMA-VH comprises the amino acid sequence of SEQ ID NO: 117, and the PSMA-VL comprises the amino acid sequence of SEQ ID NO: 119, or
(v) the PSMA-VH comprises the amino acid sequence of SEQ ID NO: 81, and the PSMA-VL comprises the amino acid sequence of SEQ ID NO: 82, or
   the PSMA-VH comprises the amino acid sequence of SEQ ID NO: 120, and the PSMA-VL comprises the amino acid sequence of SEQ ID NO: 121, or
   the PSMA-VH comprises the amino acid sequence of SEQ ID NO: 120, and the PSMA-VL comprises the amino acid sequence of SEQ ID NO: 122, or
   the PSMA-VH comprises the amino acid sequence of SEQ ID NO: 120, and the PSMA-VL comprises the amino acid sequence of SEQ ID NO: 123, or
   the PSMA-VH comprises the amino acid sequence of SEQ ID NO: 120, and the PSMA-VL comprises the amino acid sequence of SEQ ID NO: 124, or
   the PSMA-VH comprises the amino acid sequence of SEQ ID NO: 120, and the PSMA-VL comprises the amino acid sequence of SEQ ID NO: 125, or
   the PSMA-VH comprises the amino acid sequence of SEQ ID NO: 120, and the PSMA-VL comprises the amino acid sequence of SEQ ID NO: 126, or
(vi) the PSMA-VH comprises the amino acid sequence of SEQ ID NO: 83, and the PSMA-VL comprises the amino acid sequence of SEQ ID NO: 84, or
   the PSMA-VH comprises the amino acid sequence of SEQ ID NO: 127, and the PSMA-VL comprises the amino acid sequence of SEQ ID NO: 128, or
   the PSMA-VH comprises the amino acid sequence of SEQ ID NO: 127, and the PSMA-VL comprises the amino acid sequence of SEQ ID NO: 129, or
   the PSMA-VH comprises the amino acid sequence of SEQ ID NO: 127, and the PSMA-VL comprises the amino acid sequence of SEQ ID NO: 130, or
(vii) the PSMA-VH comprises the amino acid sequence of SEQ ID NO: 85, and the PSMA-VL comprises the amino acid sequence of SEQ ID NO: 86, or
   the PSMA-VH comprises the amino acid sequence of SEQ ID NO: 131, and the PSMA-VL comprises the amino acid sequence of SEQ ID NO: 133, or
   the PSMA-VH comprises the amino acid sequence of SEQ ID NO: 131, and the PSMA-VL comprises the amino acid sequence of SEQ ID NO: 134, or
   the PSMA-VH comprises the amino acid sequence of SEQ ID NO: 131, and the PSMA-VL comprises the amino acid sequence of SEQ ID NO: 135, or
   the PSMA-VH comprises the amino acid sequence of SEQ ID NO: 132, and the PSMA-VL comprises the amino acid sequence of SEQ ID NO: 133, or
   the PSMA-VH comprises the amino acid sequence of SEQ ID NO: 132, and the PSMA-VL comprises the amino acid sequence of SEQ ID NO: 134, or
   the PSMA-VH comprises the amino acid sequence of SEQ ID NO: 132, and the PSMA-VL comprises the amino acid sequence of SEQ ID NO: 135, or
(viii) the PSMA-VH comprises the amino acid sequence of SEQ ID NO: 87, and the PSMA-VL comprises the amino acid sequence of SEQ ID NO: 88, or
   the PSMA-VH comprises the amino acid sequence of SEQ ID NO: 136, and the PSMA-VL comprises the amino acid sequence of SEQ ID NO: 139, or
   the PSMA-VH comprises the amino acid sequence of SEQ ID NO: 137, and the PSMA-VL comprises the amino acid sequence of SEQ ID NO: 139, or
   the PSMA-VH comprises the amino acid sequence of SEQ ID NO: 138, and the PSMA-VL comprises the amino acid sequence of SEQ ID NO: 139, or
   the PSMA-VH comprises the amino acid sequence of SEQ ID NO: 138, and the PSMA-VL comprises the amino acid sequence of SEQ ID NO: 140, or
(ix) the PSMA-VH comprises the amino acid sequence of SEQ ID NO: 89, and the PSMA-VL comprises the amino acid sequence of SEQ ID NO: 90, or
   the PSMA-VH comprises the amino acid sequence of SEQ ID NO: 141, and the PSMA-VL comprises the amino acid sequence of SEQ ID NO: 145, or
   the PSMA-VH comprises the amino acid sequence of SEQ ID NO: 142, and the PSMA-VL comprises the amino acid sequence of SEQ ID NO: 145, or
   the PSMA-VH comprises the amino acid sequence of SEQ ID NO: 143, and the PSMA-VL comprises the amino acid sequence of SEQ ID NO: 145, or
   the PSMA-VH comprises the amino acid sequence of SEQ ID NO: 144, and the PSMA-VL comprises the amino acid sequence of SEQ ID NO: 145, or
   the PSMA-VH comprises the amino acid sequence of SEQ ID NO: 141, and the PSMA-VL comprises the amino acid sequence of SEQ ID NO: 146, or
   the PSMA-VH comprises the amino acid sequence of SEQ ID NO: 142, and the PSMA-VL comprises the amino acid sequence of SEQ ID NO: 146, or
   the PSMA-VH comprises the amino acid sequence of SEQ ID NO: 143, and the PSMA-VL comprises the amino acid sequence of SEQ ID NO: 146, or
   the PSMA-VH comprises the amino acid sequence of SEQ ID NO: 144, and the PSMA-VL comprises the amino acid sequence of SEQ ID NO: 146, or
   the PSMA-VH comprises the amino acid sequence of SEQ ID NO: 141, and the PSMA-VL comprises the amino acid sequence of SEQ ID NO: 147, or
   the PSMA-VH comprises the amino acid sequence of SEQ ID NO: 142, and the PSMA-VL comprises the amino acid sequence of SEQ ID NO: 147, or
   the PSMA-VH comprises the amino acid sequence of SEQ ID NO: 143, and the PSMA-VL comprises the amino acid sequence of SEQ ID NO: 147, or
(x) the PSMA-VH comprises the amino acid sequence of SEQ ID NO: 91, and the PSMA-VL comprises the amino acid sequence of SEQ ID NO: 92, or
   the PSMA-VH comprises the amino acid sequence of SEQ ID NO: 148, and the PSMA-VL comprises the amino acid sequence of SEQ ID NO: 150, or
   the PSMA-VH comprises the amino acid sequence of SEQ ID NO: 149, and the PSMA-VL comprises the amino acid sequence of SEQ ID NO: 150, or
   the PSMA-VH comprises the amino acid sequence of SEQ ID NO: 148, and the PSMA-VL comprises the amino acid sequence of SEQ ID NO: 151, or
   the PSMA-VH comprises the amino acid sequence of SEQ ID NO: 149, and the PSMA-VL comprises the amino acid sequence of SEQ ID NO: 151, or
   the PSMA-VH comprises the amino acid sequence of SEQ ID NO: 148, and the PSMA-VL comprises the amino acid sequence of SEQ ID NO: 152, or
   the PSMA-VH comprises the amino acid sequence of SEQ ID NO: 149, and the PSMA-VL comprises the amino acid sequence of SEQ ID NO: 152, or
(xi) the PSMA-VH comprises the amino acid sequence of SEQ ID NO: 93, and the PSMA-VL comprises the amino acid sequence of SEQ ID NO: 94; or
   the PSMA-VH comprises the amino acid sequence of SEQ ID NO: 153, and the PSMA-VL comprises the amino acid sequence of SEQ ID NO: 157; or
   the PSMA-VH comprises the amino acid sequence of SEQ ID NO: 154, and the PSMA-VL comprises the amino acid sequence of SEQ ID NO: 157; or
   the PSMA-VH comprises the amino acid sequence of SEQ ID NO: 155, and the PSMA-VL comprises the amino acid sequence of SEQ ID NO: 157; or
   the PSMA-VH comprises the amino acid sequence of SEQ ID NO: 156, and the PSMA-VL comprises the amino acid sequence of SEQ ID NO: 157; or
   the PSMA-VH comprises the amino acid sequence of SEQ ID NO: 153, and the PSMA-VL comprises the amino acid sequence of SEQ ID NO: 158; or
   the PSMA-VH comprises the amino acid sequence of SEQ ID NO: 154, and the PSMA-VL comprises the amino acid sequence of SEQ ID NO: 158; or
   the PSMA-VH comprises the amino acid sequence of SEQ ID NO: 155, and the PSMA-VL comprises the amino acid sequence of SEQ ID NO: 158; or
   the PSMA-VH comprises the amino acid sequence of SEQ ID NO: 156, and the PSMA-VL comprises the amino acid sequence of SEQ ID NO: 158; or
(xii) the PSMA-VH comprises the amino acid sequence of SEQ ID NO: 95, and the PSMA-VL comprises the amino acid sequence of SEQ ID NO: 96.

In some embodiments, provided is the antigen-binding molecule according to any one of the above, wherein
(i) the PSMA-VH comprises the amino acid sequence of SEQ ID NO: 112, and the PSMA-VL comprises the amino acid sequence of SEQ ID NO: 115, or
the PSMA-VH comprises the amino acid sequence of SEQ ID NO: 110, and the PSMA-VL comprises the amino acid sequence of SEQ ID NO: 114, or
(vi) the PSMA-VH comprises the amino acid sequence of SEQ ID NO: 127, and the PSMA-VL comprises the amino acid sequence of SEQ ID NO: 128.

In some embodiments, provided is the antigen-binding molecule according to any one of the above, wherein the antigen-binding molecule comprises an Fc region (including an IgG Fc region or an IgG₁ Fc region). In some embodiments, the Fc region comprises one or more amino acid substitutions capable of reducing binding to Fc receptors, particularly Fcy receptors, as compared to a wild-type Fc region. In some embodiments, the Fc region is a human IgG₁ Fc region, and the amino acid residues at positions 234 and 235 are A, as numbered according to the EU index. In some embodiments, the Fc region comprises the amino acid sequence of SEQ ID NO: 175.

In some embodiments, provided is the antigen-binding molecule according to any one of the above, wherein the antigen-binding molecule also comprises an Fc region comprising a first subunit (Fc1) and a second subunit (Fc2) capable of associating with each other, and the Fc1 and Fc2 each independently have one or more amino acid substitutions that reduce homodimerization of the Fc region.

The ordinal numbers "first", "second", "third", "1", "2", "3", etc. (e.g., "first subunit", "Fc2", "third chain", and "linker 2") in the present disclosure are used only to distinguish between different features, elements, components, or steps and are not intended to limit the number, order, or level.

In some embodiments, the Fc1 and Fc2 each independently have one or more amino acid substitutions according to the knob-into-hole technique. In some embodiments, the Fc1 has a knob structure according to the knob-into-hole technique, and the Fc2 has a hole structure according to the knob-into-hole technique. Vice versa.

In some embodiments, the amino acid residue at position 366 of the Fc1 is W; and the amino acid residue at position 366 of the Fc2 is S, the amino acid residue at position 368 is A, and the amino acid residue at position 407 is V, as numbered according to the EU index. Vice versa.

In some embodiments, the amino acid residue at position 354 of the Fc1 is C, and the amino acid residue at position 349 of the Fc2 is C, as numbered according to the EU index. Vice versa.

In some embodiments, the Fc1 has the amino acid sequence of SEQ ID NO: 169, and the Fc2 has the amino acid sequence of SEQ ID NO: 170.

In some embodiments, provided is the antigen-binding molecule according to any one of the above, wherein the antigen-binding molecule comprises one antigen-binding moiety that specifically binds to PSMA and one antigen-binding moiety that specifically binds to CD3. In some embodiments, the antigen-binding moiety that specifically binds to PSMA and the antigen-binding moiety that specifically binds to CD3 are scFvs.

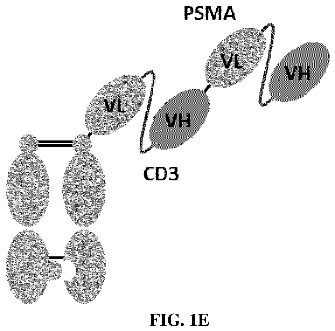

In some embodiments, provided is the antigen-binding molecule according to any one of the above, wherein the antigen-binding molecule comprises one first chain having a structure represented by formula (a) and one second chain having a structure represented by formula (b):
formula (a) [PSMA-VH]-[linker 1]-[PSMA-VL]-[linker 2]-[CD3-VH]-[linker 3]-[CD3-VL]-[linker 4]-[Fc2], and
formula (b) [Fc1];
   the structures represented by formula (a) and formula (b) are arranged from N-terminus to C-terminus, and the linker 1, linker 2, linker 3, and linker 4 are identical or different peptide linkers (as a non-limiting example, the structure shown in FIG. 1E). It should be understood that the Fc1 and Fc2 function herein to form a dimer and are therefore interchangeable.

In some embodiments, provided is the antigen-binding molecule according to any one of the above, wherein
the antigen-binding molecule has: one first chain comprising the amino acid sequence of SEQ ID NO: 191 and one second chain comprising the amino acid sequence of SEQ ID NO: 169; or
the antigen-binding molecule has: one first chain comprising the amino acid sequence of SEQ ID NO: 192 and one second chain comprising the amino acid sequence of SEQ ID NO: 169; or
the antigen-binding molecule has: one first chain comprising the amino acid sequence of SEQ ID NO: 198 and one second chain comprising the amino acid sequence of SEQ ID NO: 169.

In some embodiments, provided is the antigen-binding molecule according to any one of the above, wherein the antigen-binding moiety that specifically binds to PSMA or the antigen-binding moiety that specifically binds to CD3 comprises a Titin chain and an Obscurin chain (capable of forming a dimer).

In some embodiments, the Titin chain comprises an amino acid sequence selected from the group consisting of SEQ ID NO: 210 to SEQ ID NO: 228, and the Obscurin chain comprises an amino acid sequence selected from the group consisting of SEQ ID NO: 229 to SEQ ID NO: 269. In some embodiments, the Titin chain comprises the amino acid sequence of SEQ ID NO: 226, and the Obscurin chain comprises the amino acid sequence of SEQ ID NO: 264.

In some embodiments, provided is the antigen-binding molecule according to any one of the above, wherein the antigen-binding molecule comprises one antigen-binding moiety that specifically binds to PSMA and one antigen-binding moiety that specifically binds to CD3. In some embodiments, the antigen-binding moiety that specifically binds to PSMA is a Fab; the antigen-binding moiety that specifically binds to CD3 is a substituted Fab comprising a Titin chain and an Obscurin chain (capable of forming a dimer).

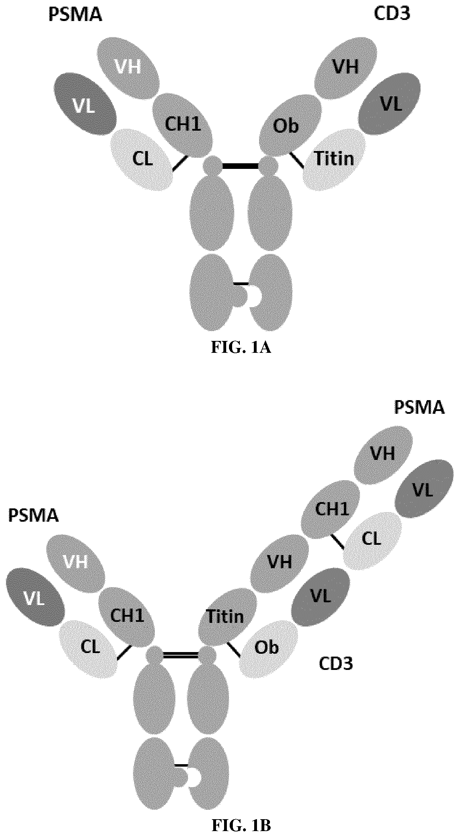

In some embodiments, provided is the antigen-binding molecule according to any one of the above, wherein the antigen-binding molecule comprises one first chain having a structure represented by formula (c), one second chain having a structure represented by formula (d), one third chain having a structure represented by formula (e), and one fourth chain having a structure represented by formula (f):
formula (c) [PSMA-VH]-[CH1]-[Fc1],
formula (d) [PSMA-VL]-[CL],
formula (e) [CD3-VH]-[linker 5]-[Obscurin chain]-[Fc2], and
formula (f) [CD3-VL]-[linker 6]-[Titin chain];
   the structures represented by formulas (c), (d), (e), and (f) are arranged from N-terminus to C-terminus, and the linker 5 and the linker 6 are identical or different peptide linkers (as a non-limiting example, the structure shown in FIG. 1A). It should be understood that the Fc1 and Fc2 function herein to form a dimer and are therefore interchangeable.

In some embodiments, provided is the antigen-binding molecule according to any one of the above, wherein
the antigen-binding molecule has: one first chain comprising the amino acid sequence of SEQ ID NO: 193, one second chain comprising the amino acid sequence of SEQ ID NO: 194, one third chain comprising the amino acid sequence of SEQ ID NO: 171, and one fourth chain comprising the amino acid sequence of SEQ ID NO: 172, or,
the antigen-binding molecule has: one first chain comprising the amino acid sequence of SEQ ID NO: 201, one second chain comprising the amino acid sequence of SEQ ID NO: 202, one third chain comprising the amino acid sequence of SEQ ID NO: 171, and one fourth chain comprising the amino acid sequence of SEQ ID NO: 172, or
the antigen-binding molecule has: one first chain comprising the amino acid sequence of SEQ ID NO: 206, one second chain comprising the amino acid sequence of SEQ ID NO: 207, one third chain comprising the amino acid sequence of SEQ ID NO: 171, and one fourth chain comprising the amino acid sequence of SEQ ID NO: 172, or
the antigen-binding molecule has: one first chain comprising the amino acid sequence of SEQ ID NO: 284, one second chain comprising the amino acid sequence of SEQ ID NO: 285, one third chain comprising the amino acid sequence of SEQ ID NO: 171, and one fourth chain comprising the amino acid sequence of SEQ ID NO: 172.

In some embodiments, provided is the antigen-binding molecule according to any one of the above, wherein the antigen-binding molecule comprises two antigen-binding moieties that specifically bind to PSMA and one antigen-binding moiety that specifically binds to CD3. In some embodiments, the antigen-binding moieties that specifically bind to PSMA are Fabs; the antigen-binding moiety that specifically binds to CD3 is a substituted Fab comprising a Titin chain and an Obscurin chain (capable of forming a dimer).

In some embodiments, provided is the antigen-binding molecule according to any one of the above, wherein the antigen-binding molecule comprises one first chain having a structure represented by formula (c), two second chains having a structure represented by formula (d), one third chain having a structure represented by formula (g), and one fourth chain having a structure represented by formula (h):
formula (c) [PSMA-VH]-[CH1]-[Fc1],
formula (d) [PSMA-VL]-[CL],
formula (g) [PSMA-VH]-[CH1]-[CD3-VH]-[linker 7]-[Titin chain]-[Fc2], and
formula (h) [CD3-VL]-[linker 8]-[Obscurin chain];
the structures represented by formulas (c), (d), (g), and (h) are arranged from N-terminus to C-terminus, and the linker 7 and the linker 8 are identical or different peptide linkers. (As a non-limiting example, the structure shown in FIG. 1B). It should be understood that the Fc1 and Fc2 function herein to form a dimer and are therefore interchangeable.

In some embodiments, provided is the antigen-binding molecule according to any one of the above, wherein
the antigen-binding molecule has: one first chain comprising the amino acid sequence of SEQ ID NO: 180, two second chains comprising the amino acid sequence of SEQ ID NO: 181, one third chain comprising the amino acid sequence of SEQ ID NO: 182, and one fourth chain comprising the amino acid sequence of SEQ ID NO: 174, or
the antigen-binding molecule has: one first chain comprising the amino acid sequence of SEQ ID NO: 187, two second chains comprising the amino acid sequence of SEQ ID NO: 188, one third chain comprising the amino acid sequence of SEQ ID NO: 189, and one fourth chain comprising the amino acid sequence of SEQ ID NO: 174, or
the antigen-binding molecule has: one first chain comprising the amino acid sequence of SEQ ID NO: 193, two second chains comprising the amino acid sequence of SEQ ID NO: 194, one third chain comprising the amino acid sequence of SEQ ID NO: 195, and one fourth chain comprising the amino acid sequence of SEQ ID NO: 174, or
the antigen-binding molecule has: one first chain comprising the amino acid sequence of SEQ ID NO: 206, two second chains comprising the amino acid sequence of SEQ ID NO: 207, one third chain comprising the amino acid sequence of SEQ ID NO: 208, and one fourth chain comprising the amino acid sequence of SEQ ID NO: 174.

In some embodiments, provided is the antigen-binding molecule according to any one of the above, wherein the antigen-binding molecule comprises two antigen-binding moieties that specifically bind to PSMA and two antigen-binding moieties that specifically bind to CD3. In some embodiments, the antigen-binding moieties that specifically bind to PSMA are Fabs; the antigen-binding moieties that specifically bind to CD3 are substituted Fabs comprising a Titin chain and an Obscurin chain (capable of forming a dimer).

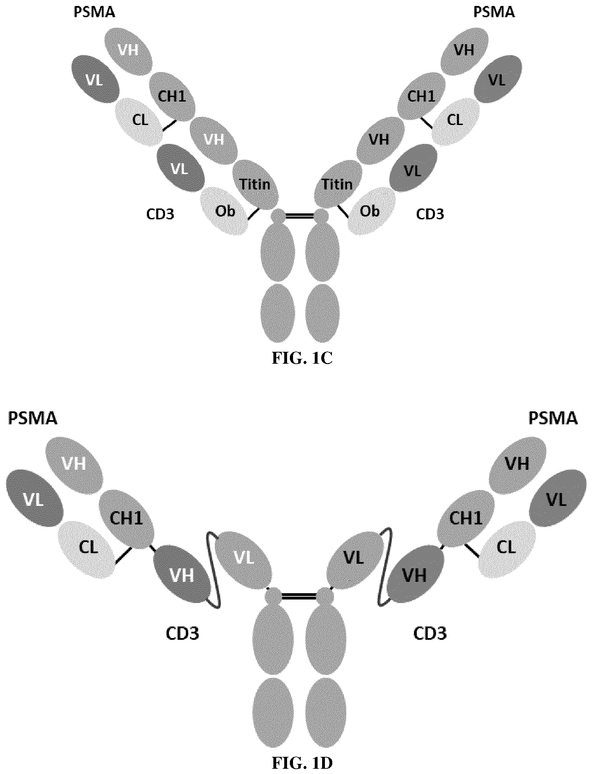

In some embodiments, provided is the antigen-binding molecule according to any one of the above, wherein the antigen-binding molecule comprises two first chains having a structure represented by formula (j), two second chains having a structure represented by formula (d), and two third chains having a structure represented by formula (h):
formula (j) [PSMA-VH]-[CH1]-[CD3-VH]-[linker 9]-[Titin chain]-[one subunit of Fc],
formula (d) [PSMA-VL]-[CL], and
formula (h) [CD3-VL]-[linker 8]-[Obscurin chain];
the structures represented by formulas (j), (d), and (h) are arranged from N-terminus to C-terminus, and the linker 8 and the linker 9 are identical or different peptide linkers. (As a non-limiting example, the structure shown in FIG. 1C). It should be understood that the Fc1 and Fc2 function herein to form a dimer and are therefore interchangeable.

In some embodiments, provided is the antigen-binding molecule according to any one of the above, wherein
the antigen-binding molecule has: two first chains comprising the amino acid sequence of SEQ ID NO: 183, two second chains comprising the amino acid sequence of SEQ ID NO: 181, and two third chains comprising the amino acid sequence of SEQ ID NO: 174, or
the antigen-binding molecule has: two first chains comprising the amino acid sequence of SEQ ID NO: 185, two second chains comprising the amino acid sequence of SEQ ID NO: 186, and two third chains comprising the amino acid sequence of SEQ ID NO: 174, or
the antigen-binding molecule has: two first chains comprising the amino acid sequence of SEQ ID NO: 196, two second chains comprising the amino acid sequence of SEQ ID NO: 194, and two third chains comprising the amino acid sequence of SEQ ID NO: 174, or
the antigen-binding molecule has: two first chains comprising the amino acid sequence of SEQ ID NO: 203, two second chains comprising the amino acid sequence of SEQ ID NO: 204, and two third chains comprising the amino acid sequence of SEQ ID NO: 174, or
the antigen-binding molecule has: two first chains comprising the amino acid sequence of SEQ ID NO: 286, two second chains comprising the amino acid sequence of SEQ ID NO: 285, and two third chains comprising the amino acid sequence of SEQ ID NO: 174.

In some embodiments, provided is the antigen-binding molecule according to any one of the above, wherein the antigen-binding molecule comprises two antigen-binding moieties that specifically bind to PSMA and two antigen-binding moieties that specifically bind to CD3. In some embodiments, the antigen-binding moieties that specifically bind to PSMA are Fabs, and the antigen-binding moieties that specifically bind to CD3 are scFvs.

In some embodiments, provided is the antigen-binding molecule according to any one of the above, wherein the antigen-binding molecule comprises two first chains having a structure represented by formula (k) and two second chains having a structure represented by formula (d):
formula (k) [PSMA-VH]-[CH1]-[CD3-VH]-[linker 9]-[CD3-VL]-[linker 10]-[one subunit of Fc], and
formula (d) [PSMA-VL]-[CL];
the structures represented by formulas (k) and (d) are arranged from N-terminus to C-terminus, and the linker 9 and the linker 10 are identical or different peptide linkers. (As a non-limiting example, the structure shown in FIG. 1D). It should be understood that the Fc1 and Fc2 function herein to form a dimer and are therefore interchangeable.

In some embodiments, provided is the antigen-binding molecule according to any one of the above, wherein
the antigen-binding molecule has: two first chains comprising the amino acid sequence of SEQ ID NO: 184 and two second chains comprising the amino acid sequence of SEQ ID NO: 181, or
the antigen-binding molecule has: two first chains comprising the amino acid sequence of SEQ ID NO: 190 and two second chains comprising the amino acid sequence of SEQ ID NO: 188, or
the antigen-binding molecule has: two first chains comprising the amino acid sequence of SEQ ID NO: 197 and two second chains comprising the amino acid sequence of SEQ ID NO: 194, or
the antigen-binding molecule has: two first chains comprising the amino acid sequence of SEQ ID NO: 199 and two second chains comprising the amino acid sequence of SEQ ID NO: 200, or
the antigen-binding molecule has: two first chains comprising the amino acid sequence of SEQ ID NO: 205 and two second chains comprising the amino acid sequence of SEQ ID NO: 204, or
the antigen-binding molecule has: two first chains comprising the amino acid sequence of SEQ ID NO: 209 and two second chains comprising the amino acid sequence of SEQ ID NO: 207.

In another aspect, the present disclosure also provides an isolated antibody capable of specifically binding to PSMA, wherein the antibody comprises a heavy chain variable region PSMA-VH and a light chain variable region PSMA-VL, wherein
(i) PSMA-HCDR1, PSMA-HCDR2, and PSMA-HCDR3 in the PSMA-VH comprise the amino acid sequences of PSMA-HCDR1, PSMA-HCDR2, and PSMA-HCDR3 in SEQ ID NO: 77, respectively, and PSMA-LCDR1, PSMA-LCDR2, and PSMA-LCDR3 in the PSMA-VL comprise the amino acid sequences of PSMA-LCDR1, PSMA-LCDR2, and PSMA-LCDR3 in SEQ ID NO: 78, respectively, or
(ii) PSMA-HCDR1, PSMA-HCDR2, and PSMA-HCDR3 in the PSMA-VH comprise the amino acid sequences of PSMA-HCDR1, PSMA-HCDR2, and PSMA-HCDR3 in SEQ ID NO: 73, respectively, and PSMA-LCDR1, PSMA-LCDR2, and PSMA-LCDR3 in the PSMA-VL comprise the amino acid sequences of PSMA-LCDR1, PSMA-LCDR2, and PSMA-LCDR3 in SEQ ID NO: 74, respectively, or
(iii) PSMA-HCDR1, PSMA-HCDR2, and PSMA-HCDR3 in the PSMA-VH comprise the amino acid sequences of PSMA-HCDR1, PSMA-HCDR2, and PSMA-HCDR3 in SEQ ID NO: 75, respectively, and PSMA-LCDR1, PSMA-LCDR2, and PSMA-LCDR3 in the PSMA-VL comprise the amino acid sequences of PSMA-LCDR1, PSMA-LCDR2, and PSMA-LCDR3 in SEQ ID NO: 76, respectively, or
(iv) PSMA-HCDR1, PSMA-HCDR2, and PSMA-HCDR3 in the PSMA-VH comprise the amino acid sequences of PSMA-HCDR1, PSMA-HCDR2, and PSMA-HCDR3 in SEQ ID NO: 79, respectively, and PSMA-LCDR1, PSMA-LCDR2, and PSMA-LCDR3 in the PSMA-VL comprise the amino acid sequences of PSMA-LCDR1, PSMA-LCDR2, and PSMA-LCDR3 in SEQ ID NO: 80, respectively, or
(v) PSMA-HCDR1, PSMA-HCDR2, and PSMA-HCDR3 in the PSMA-VH comprise the amino acid sequences of PSMA-HCDR1, PSMA-HCDR2, and PSMA-HCDR3 in SEQ ID NO: 81, respectively, and PSMA-LCDR1, PSMA-LCDR2, and PSMA-LCDR3 in the PSMA-VL comprise the amino acid sequences of PSMA-LCDR1, PSMA-LCDR2, and PSMA-LCDR3 in SEQ ID NO: 82, respectively, or
(vi) PSMA-HCDR1, PSMA-HCDR2, and PSMA-HCDR3 in the PSMA-VH comprise the amino acid sequences of PSMA-HCDR1, PSMA-HCDR2, and PSMA-HCDR3 in SEQ ID NO: 83, respectively, and PSMA-LCDR1, PSMA-LCDR2, and PSMA-LCDR3 in the PSMA-VL comprise the amino acid sequences of PSMA-LCDR1, PSMA-LCDR2, and PSMA-LCDR3 in SEQ ID NO: 84, respectively, or
(vii) PSMA-HCDR1, PSMA-HCDR2, and PSMA-HCDR3 in the PSMA-VH comprise the amino acid sequences of PSMA-HCDR1, PSMA-HCDR2, and PSMA-HCDR3 in SEQ ID NO: 85, respectively, and PSMA-LCDR1, PSMA-LCDR2, and PSMA-LCDR3 in the PSMA-VL comprise the amino acid sequences of PSMA-LCDR1, PSMA-LCDR2, and PSMA-LCDR3 in SEQ ID NO: 86, respectively, or
(viii) PSMA-HCDR1, PSMA-HCDR2, and PSMA-HCDR3 in the PSMA-VH comprise the amino acid sequences of PSMA-HCDR1, PSMA-HCDR2, and PSMA-HCDR3 in SEQ ID NO: 87, respectively, and PSMA-LCDR1, PSMA-LCDR2, and PSMA-LCDR3 in the PSMA-VL comprise the amino acid sequences of PSMA-LCDR1, PSMA-LCDR2, and PSMA-LCDR3 in SEQ ID NO: 88, respectively, or
(ix) PSMA-HCDR1, PSMA-HCDR2, and PSMA-HCDR3 in the PSMA-VH comprise the amino acid sequences of PSMA-HCDR1, PSMA-HCDR2, and PSMA-HCDR3 in SEQ ID NO: 89, respectively, and PSMA-LCDR1, PSMA-LCDR2, and PSMA-LCDR3 in the PSMA-VL comprise the amino acid sequences of PSMA-LCDR1, PSMA-LCDR2, and PSMA-LCDR3 in SEQ ID NO: 90, respectively, or
(x) PSMA-HCDR1, PSMA-HCDR2, and PSMA-HCDR3 in the PSMA-VH comprise the amino acid sequences of PSMA-HCDR1, PSMA-HCDR2, and PSMA-HCDR3 in SEQ ID NO: 91, respectively, and PSMA-LCDR1, PSMA-LCDR2, and PSMA-LCDR3 in the PSMA-VL comprise the amino acid sequences of PSMA-LCDR1, PSMA-LCDR2, and PSMA-LCDR3 in SEQ ID NO: 92 or 152, respectively, or
(xi) PSMA-HCDR1, PSMA-HCDR2, and PSMA-HCDR3 in the PSMA-VH comprise the amino acid sequences of PSMA-HCDR1, PSMA-HCDR2, and PSMA-HCDR3 in SEQ ID NO: 93, respectively, and PSMA-LCDR1, PSMA-LCDR2, and PSMA-LCDR3 in the PSMA-VL comprise the amino acid sequences of PSMA-LCDR1, PSMA-LCDR2, and PSMA-LCDR3 in SEQ ID NO: 94, respectively, or
(xii) PSMA-HCDR1, PSMA-HCDR2, and PSMA-HCDR3 in the PSMA-VH comprise the amino acid sequences of PSMA-HCDR1, PSMA-HCDR2, and PSMA-HCDR3 in SEQ ID NO: 95, respectively, and PSMA-LCDR1, PSMA-LCDR2, and PSMA-LCDR3 in the PSMA-VL comprise the amino acid sequences of PSMA-LCDR1, PSMA-LCDR2, and PSMA-LCDR3 in SEQ ID NO: 96, respectively.

In some embodiments, the PSMA-HCDR1, PSMA-HCDR2, PSMA-HCDR3, PSMA-LCDR1, PSMA-LCDR2, and PSMA-LCDR3 are defined according to the Kabat, IMGT, Chothia, AbM, or Contact numbering scheme.

In some embodiments, provided is the isolated antibody according to the above, wherein
(i) the PSMA-VH has: a PSMA-HCDR1 comprising the amino acid sequence of SEQ ID NO: 13, a PSMA-HCDR2 comprising the amino acid sequence of SEQ ID NO: 14, and a PSMA-HCDR3 comprising the amino acid sequence of SEQ ID NO: 15, and the PSMA-VL has: a PSMA-LCDR1 comprising the amino acid sequence of SEQ ID NO: 16, a PSMA-LCDR2 comprising the amino acid sequence of SEQ ID NO: 17, and a PSMA-LCDR3 comprising the amino acid sequence of SEQ ID NO: 18, or
(ii) the PSMA-VH has: a PSMA-HCDR1 comprising the amino acid sequence of SEQ ID NO: 1, a PSMA-HCDR2 comprising the amino acid sequence of SEQ ID NO: 2, and a PSMA-HCDR3 comprising the amino acid sequence of SEQ ID NO: 3, and the PSMA-VL has: a PSMA-LCDR1 comprising the amino acid sequence of SEQ ID NO: 4, a PSMA-LCDR2 comprising the amino acid sequence of SEQ ID NO: 5, and a PSMA-LCDR3 comprising the amino acid sequence of SEQ ID NO: 6, or
(iii) the PSMA-VH has: a PSMA-HCDR1 comprising the amino acid sequence of SEQ ID NO: 7, a PSMA-HCDR2 comprising the amino acid sequence of SEQ ID NO: 8, and a PSMA-HCDR3 comprising the amino acid sequence of SEQ ID NO: 9, and the PSMA-VL has: a PSMA-LCDR1 comprising the amino acid sequence of SEQ ID NO: 10, a PSMA-LCDR2 comprising the amino acid sequence of SEQ ID NO: 11, and a PSMA-LCDR3 comprising the amino acid sequence of SEQ ID NO: 12, or
(iv) the PSMA-VH has: a PSMA-HCDR1 comprising the amino acid sequence of SEQ ID NO: 19, a PSMA-HCDR2 comprising the amino acid sequence of SEQ ID NO: 20, and a PSMA-HCDR3 comprising the amino acid sequence of SEQ ID NO: 21, and the PSMA-VL has: a PSMA-LCDR1 comprising the amino acid sequence of SEQ ID NO: 22, a PSMA-LCDR2 comprising the amino acid sequence of SEQ ID NO: 23, and a PSMA-LCDR3 comprising the amino acid sequence of SEQ ID NO: 24, or
(v) the PSMA-VH has: a PSMA-HCDR1 comprising the amino acid sequence of SEQ ID NO: 25, a PSMA-HCDR2 comprising the amino acid sequence of SEQ ID NO: 26, and a PSMA-HCDR3 comprising the amino acid sequence of SEQ ID NO: 27, and the PSMA-VL has: a PSMA-LCDR1 comprising the amino acid sequence of SEQ ID NO: 28, a PSMA-LCDR2 comprising the amino acid sequence of SEQ ID NO: 29, and a PSMA-LCDR3 comprising the amino acid sequence of SEQ ID NO: 30, or
(vi) the PSMA-VH has: a PSMA-HCDR1 comprising the amino acid sequence of SEQ ID NO: 31, a PSMA-HCDR2 comprising the amino acid sequence of SEQ ID NO: 32, and a PSMA-HCDR3 comprising the amino acid sequence of SEQ ID NO: 33, and the PSMA-VL has: a PSMA-LCDR1 comprising the amino acid sequence of SEQ ID NO: 34, a PSMA-LCDR2 comprising the amino acid sequence of SEQ ID NO: 35, and a PSMA-LCDR3 comprising the amino acid sequence of SEQ ID NO: 36, or
(vii) the PSMA-VH has: a PSMA-HCDR1 comprising the amino acid sequence of SEQ ID NO: 37, a PSMA-HCDR2 comprising the amino acid sequence of SEQ ID NO: 38, and a PSMA-HCDR3 comprising the amino acid sequence of SEQ ID NO: 39, and the PSMA-VL has: a PSMA-LCDR1 comprising the amino acid sequence of SEQ ID NO: 40, a PSMA-LCDR2 comprising the amino acid sequence of SEQ ID NO: 41, and a PSMA-LCDR3 comprising the amino acid sequence of SEQ ID NO: 42, or
(viii) the PSMA-VH has: a PSMA-HCDR1 comprising the amino acid sequence of SEQ ID NO: 43, a PSMA-HCDR2 comprising the amino acid sequence of SEQ ID NO: 44, and a PSMA-HCDR3 comprising the amino acid sequence of SEQ ID NO: 45, and the PSMA-VL has: a PSMA-LCDR1 comprising the amino acid sequence of SEQ ID NO: 46, a PSMA-LCDR2 comprising the amino acid sequence of SEQ ID NO: 47, and a PSMA-LCDR3 comprising the amino acid sequence of SEQ ID NO: 48, or
(ix) the PSMA-VH has: a PSMA-HCDR1 comprising the amino acid sequence of SEQ ID NO: 49, a PSMA-HCDR2 comprising the amino acid sequence of SEQ ID NO: 50, and a PSMA-HCDR3 comprising the amino acid sequence of SEQ ID NO: 51, and the PSMA-VL has: a PSMA-LCDR1 comprising the amino acid sequence of SEQ ID NO: 52, a PSMA-LCDR2 comprising the amino acid sequence of SEQ ID NO: 53, and a PSMA-LCDR3 comprising the amino acid sequence of SEQ ID NO: 54, or
(x) the PSMA-VH has: a PSMA-HCDR1 comprising the amino acid sequence of SEQ ID NO: 55, a PSMA-HCDR2 comprising the amino acid sequence of SEQ ID NO: 56, and a PSMA-HCDR3 comprising the amino acid sequence of SEQ ID NO: 57, and the PSMA-VL has: a PSMA-LCDR1 comprising the amino acid sequence of SEQ ID NO: 58 or 159, a PSMA-LCDR2 comprising the amino acid sequence of SEQ ID NO: 59, and a PSMA-LCDR3 comprising the amino acid sequence of SEQ ID NO: 60, or
(xi) the PSMA-VH has: a PSMA-HCDR1 comprising the amino acid sequence of SEQ ID NO: 61, a PSMA-HCDR2 comprising the amino acid sequence of SEQ ID NO: 62, and a PSMA-HCDR3 comprising the amino acid sequence of SEQ ID NO: 63, and the PSMA-VL has: a PSMA-LCDR1 comprising the amino acid sequence of SEQ ID NO: 64, a PSMA-LCDR2 comprising the amino acid sequence of SEQ ID NO: 65, and a PSMA-LCDR3 comprising the amino acid sequence of SEQ ID NO: 66, or
(xii) the PSMA-VH has: a PSMA-HCDR1 comprising the amino acid sequence of SEQ ID NO: 67, a PSMA-HCDR2 comprising the amino acid sequence of SEQ ID NO: 68, and a PSMA-HCDR3 comprising the amino acid sequence of SEQ ID NO: 69, and the PSMA-VL has: a PSMA-LCDR1 comprising the amino acid sequence of SEQ ID NO: 70, a PSMA-LCDR2 comprising the amino acid sequence of SEQ ID NO: 71, and a PSMA-LCDR3 comprising the amino acid sequence of SEQ ID NO: 72.

In some embodiments, the PSMA-HCDR1, PSMA-HCDR2, PSMA-HCDR3, PSMA-LCDR1, PSMA-LCDR2, and PSMA-LCDR3 are defined according to the Kabat numbering scheme.

In some embodiments, the antibody binds to PSMA with an EC₅₀ of less than 10 µg/mL, 1.5 µg/mL, 1.3 µg/mL, 1.1 µg/mL, 1 µg/mL, 0.7 µg/mL, 0.6 µg/mL, 0.5 µg/mL, 0.4 µg/mL, 0.3 µg/mL, 0.2 µg/mL, 0.1 µg/mL, 0.09 µg/mL, 0.08 µg/mL, 0.07 µg/mL, 0.06 µg/mL, 0.05 µg/mL, 0.04 µg/mL, or 0.03 µg/mL, as measured by ELISA. In some embodiments, provided is the antibody according to any one of the above, wherein
(i) the PSMA-VH comprises the amino acid sequence of SEQ ID NO: 77, 107, 108, 109, 110, 111, or 112, and the PSMA-VL comprises the amino acid sequence of SEQ ID NO: 78, 113, 114, or 115, or
(ii) the PSMA-VH comprises the amino acid sequence of SEQ ID NO: 73 or 99, and the PSMA-VL comprises the amino acid sequence of SEQ ID NO: 74 or 100, or
(iii) the PSMA-VH comprises the amino acid sequence of SEQ ID NO: 75, 101, 102, or 103, and the PSMA-VL comprises the amino acid sequence of SEQ ID NO: 76, 104, 105, or 106, or
(iv) the PSMA-VH comprises the amino acid sequence of SEQ ID NO: 79, 116, or 117, and the PSMA-VL comprises the amino acid sequence of SEQ ID NO: 80, 118, or 119, or
(v) the PSMA-VH comprises the amino acid sequence of SEQ ID NO: 81 or 120, and the PSMA-VL comprises the amino acid sequence of SEQ ID NO: 82, 121, 122, 123, 124, 125, or 126, or
(vi) the PSMA-VH comprises the amino acid sequence of SEQ ID NO: 83 or 127, and the PSMA-VL comprises the amino acid sequence of SEQ ID NO: 84, 128, 129, or 130, or
(vii) the PSMA-VH comprises the amino acid sequence of SEQ ID NO: 85, 131, or 132, and the PSMA-VL comprises the amino acid sequence of SEQ ID NO: 86, 133, 134, or 135, or
(viii) the PSMA-VH comprises the amino acid sequence of SEQ ID NO: 87, 136, 137, or 138, and the PSMA-VL comprises the amino acid sequence of SEQ ID NO: 88, 139, or 140, or
(ix) the PSMA-VH comprises the amino acid sequence of SEQ ID NO: 89, 141, 142, 143, or 144, and the PSMA-VL comprises the amino acid sequence of SEQ ID NO: 90, 145, 146, or 147, or
(x) the PSMA-VH comprises the amino acid sequence of SEQ ID NO: 91, 148, or 149, and the PSMA-VL comprises the amino acid sequence of SEQ ID NO: 92, 150, 151, or 152, or
(xi) the PSMA-VH comprises the amino acid sequence of SEQ ID NO: 93, 153, 154, 155, or 156, and the PSMA-VL comprises the amino acid sequence of SEQ ID NO: 94, 157, or 158; or
(xii) the PSMA-VH comprises the amino acid sequence of SEQ ID NO: 95, 277, 278, or 279, and the PSMA-VL comprises the amino acid sequence of SEQ ID NO: 96, 280, 281, 282, or 283.

In some embodiments, provided is the antibody according to any one of the above, wherein
(i) the PSMA-VH comprises the amino acid sequence of SEQ ID NO: 112, and the PSMA-VL comprises the amino acid sequence of SEQ ID NO: 115, or
   the PSMA-VH comprises the amino acid sequence of SEQ ID NO: 110, and the PSMA-VL comprises the amino acid sequence of SEQ ID NO: 114, or
   the PSMA-VH comprises the amino acid sequence of SEQ ID NO: 77, and the PSMA-VL comprises the amino acid sequence of SEQ ID NO: 78, or
   the PSMA-VH comprises the amino acid sequence of SEQ ID NO: 107, and the PSMA-VL comprises the amino acid sequence of SEQ ID NO: 113, or
   the PSMA-VH comprises the amino acid sequence of SEQ ID NO: 108, and the PSMA-VL comprises the amino acid sequence of SEQ ID NO: 113, or
   the PSMA-VH comprises the amino acid sequence of SEQ ID NO: 109, and the PSMA-VL comprises the amino acid sequence of SEQ ID NO: 113, or
   the PSMA-VH comprises the amino acid sequence of SEQ ID NO: 110, and the PSMA-VL comprises the amino acid sequence of SEQ ID NO: 113, or
   the PSMA-VH comprises the amino acid sequence of SEQ ID NO: 111, and the PSMA-VL comprises the amino acid sequence of SEQ ID NO: 113, or
   the PSMA-VH comprises the amino acid sequence of SEQ ID NO: 107, and the PSMA-VL comprises the amino acid sequence of SEQ ID NO: 114, or
   the PSMA-VH comprises the amino acid sequence of SEQ ID NO: 108, and the PSMA-VL comprises the amino acid sequence of SEQ ID NO: 114, or
   the PSMA-VH comprises the amino acid sequence of SEQ ID NO: 109, and the PSMA-VL comprises the amino acid sequence of SEQ ID NO: 114, or
   the PSMA-VH comprises the amino acid sequence of SEQ ID NO: 111, and the PSMA-VL comprises the amino acid sequence of SEQ ID NO: 114, or
(ii) the PSMA-VH comprises the amino acid sequence of SEQ ID NO: 73, and the PSMA-VL comprises the amino acid sequence of SEQ ID NO: 74, or
   the PSMA-VH comprises the amino acid sequence of SEQ ID NO: 99, and the PSMA-VL comprises the amino acid sequence of SEQ ID NO: 100, or
(iii) the PSMA-VH comprises the amino acid sequence of SEQ ID NO: 75, and the PSMA-VL comprises the amino acid sequence of SEQ ID NO: 76, or
   the PSMA-VH comprises the amino acid sequence of SEQ ID NO: 101, and the PSMA-VL comprises the amino acid sequence of SEQ ID NO: 104, or
   the PSMA-VH comprises the amino acid sequence of SEQ ID NO: 102, and the PSMA-VL comprises the amino acid sequence of SEQ ID NO: 104, or
   the PSMA-VH comprises the amino acid sequence of SEQ ID NO: 103, and the PSMA-VL comprises the amino acid sequence of SEQ ID NO: 104, or
   the PSMA-VH comprises the amino acid sequence of SEQ ID NO: 101, and the PSMA-VL comprises the amino acid sequence of SEQ ID NO: 105, or
   the PSMA-VH comprises the amino acid sequence of SEQ ID NO: 102, and the PSMA-VL comprises the amino acid sequence of SEQ ID NO: 105, or
   the PSMA-VH comprises the amino acid sequence of SEQ ID NO: 103, and the PSMA-VL comprises the amino acid sequence of SEQ ID NO: 105, or
   the PSMA-VH comprises the amino acid sequence of SEQ ID NO: 101, and the PSMA-VL comprises the amino acid sequence of SEQ ID NO: 106, or
   the PSMA-VH comprises the amino acid sequence of SEQ ID NO: 102, and the PSMA-VL comprises the amino acid sequence of SEQ ID NO: 106, or
   the PSMA-VH comprises the amino acid sequence of SEQ ID NO: 103, and the PSMA-VL comprises the amino acid sequence of SEQ ID NO: 106, or
(iv) the PSMA-VH comprises the amino acid sequence of SEQ ID NO: 79, and the PSMA-VL comprises the amino acid sequence of SEQ ID NO: 80, or
   the PSMA-VH comprises the amino acid sequence of SEQ ID NO: 116, and the PSMA-VL comprises the amino acid sequence of SEQ ID NO: 118, or
   the PSMA-VH comprises the amino acid sequence of SEQ ID NO: 117, and the PSMA-VL comprises the amino acid sequence of SEQ ID NO: 118, or
   the PSMA-VH comprises the amino acid sequence of SEQ ID NO: 116, and the PSMA-VL comprises the amino acid sequence of SEQ ID NO: 119, or
   the PSMA-VH comprises the amino acid sequence of SEQ ID NO: 117, and the PSMA-VL comprises the amino acid sequence of SEQ ID NO: 119, or
(v) the PSMA-VH comprises the amino acid sequence of SEQ ID NO: 81, and the PSMA-VL comprises the amino acid sequence of SEQ ID NO: 82, or
   the PSMA-VH comprises the amino acid sequence of SEQ ID NO: 120, and the PSMA-VL comprises the amino acid sequence of SEQ ID NO: 121, or
   the PSMA-VH comprises the amino acid sequence of SEQ ID NO: 120, and the PSMA-VL comprises the amino acid sequence of SEQ ID NO: 122, or
   the PSMA-VH comprises the amino acid sequence of SEQ ID NO: 120, and the PSMA-VL comprises the amino acid sequence of SEQ ID NO: 123, or
   the PSMA-VH comprises the amino acid sequence of SEQ ID NO: 120, and the PSMA-VL comprises the amino acid sequence of SEQ ID NO: 124, or
   the PSMA-VH comprises the amino acid sequence of SEQ ID NO: 120, and the PSMA-VL comprises the amino acid sequence of SEQ ID NO: 125, or
   the PSMA-VH comprises the amino acid sequence of SEQ ID NO: 120, and the PSMA-VL comprises the amino acid sequence of SEQ ID NO: 126, or
(vi) the PSMA-VH comprises the amino acid sequence of SEQ ID NO: 83, and the PSMA-VL comprises the amino acid sequence of SEQ ID NO: 84, or
   the PSMA-VH comprises the amino acid sequence of SEQ ID NO: 127, and the PSMA-VL comprises the amino acid sequence of SEQ ID NO: 128, or
   the PSMA-VH comprises the amino acid sequence of SEQ ID NO: 127, and the PSMA-VL comprises the amino acid sequence of SEQ ID NO: 129, or
   the PSMA-VH comprises the amino acid sequence of SEQ ID NO: 127, and the PSMA-VL comprises the amino acid sequence of SEQ ID NO: 130, or
(vii) the PSMA-VH comprises the amino acid sequence of SEQ ID NO: 85, and the PSMA-VL comprises the amino acid sequence of SEQ ID NO: 86, or
   the PSMA-VH comprises the amino acid sequence of SEQ ID NO: 131, and the PSMA-VL comprises the amino acid sequence of SEQ ID NO: 133, or
   the PSMA-VH comprises the amino acid sequence of SEQ ID NO: 131, and the PSMA-VL comprises the amino acid sequence of SEQ ID NO: 134, or
   the PSMA-VH comprises the amino acid sequence of SEQ ID NO: 131, and the PSMA-VL comprises the amino acid sequence of SEQ ID NO: 135, or
   the PSMA-VH comprises the amino acid sequence of SEQ ID NO: 132, and the PSMA-VL comprises the amino acid sequence of SEQ ID NO: 133, or
   the PSMA-VH comprises the amino acid sequence of SEQ ID NO: 132, and the PSMA-VL comprises the amino acid sequence of SEQ ID NO: 134, or
   the PSMA-VH comprises the amino acid sequence of SEQ ID NO: 132, and the PSMA-VL comprises the amino acid sequence of SEQ ID NO: 135, or
(viii) the PSMA-VH comprises the amino acid sequence of SEQ ID NO: 87, and the PSMA-VL comprises the amino acid sequence of SEQ ID NO: 88, or
   the PSMA-VH comprises the amino acid sequence of SEQ ID NO: 136, and the PSMA-VL comprises the amino acid sequence of SEQ ID NO: 139, or
   the PSMA-VH comprises the amino acid sequence of SEQ ID NO: 137, and the PSMA-VL comprises the amino acid sequence of SEQ ID NO: 139, or
   the PSMA-VH comprises the amino acid sequence of SEQ ID NO: 138, and the PSMA-VL comprises the amino acid sequence of SEQ ID NO: 139, or
   the PSMA-VH comprises the amino acid sequence of SEQ ID NO: 138, and the PSMA-VL comprises the amino acid sequence of SEQ ID NO: 140, or
(ix) the PSMA-VH comprises the amino acid sequence of SEQ ID NO: 89, and the PSMA-VL comprises the amino acid sequence of SEQ ID NO: 90, or
   the PSMA-VH comprises the amino acid sequence of SEQ ID NO: 141, and the PSMA-VL comprises the amino acid sequence of SEQ ID NO: 145, or
   the PSMA-VH comprises the amino acid sequence of SEQ ID NO: 142, and the PSMA-VL comprises the amino acid sequence of SEQ ID NO: 145, or
   the PSMA-VH comprises the amino acid sequence of SEQ ID NO: 143, and the PSMA-VL comprises the amino acid sequence of SEQ ID NO: 145, or
   the PSMA-VH comprises the amino acid sequence of SEQ ID NO: 144, and the PSMA-VL comprises the amino acid sequence of SEQ ID NO: 145, or
   the PSMA-VH comprises the amino acid sequence of SEQ ID NO: 141, and the PSMA-VL comprises the amino acid sequence of SEQ ID NO: 146, or
   the PSMA-VH comprises the amino acid sequence of SEQ ID NO: 142, and the PSMA-VL comprises the amino acid sequence of SEQ ID NO: 146, or
   the PSMA-VH comprises the amino acid sequence of SEQ ID NO: 143, and the PSMA-VL comprises the amino acid sequence of SEQ ID NO: 146, or
   the PSMA-VH comprises the amino acid sequence of SEQ ID NO: 144, and the PSMA-VL comprises the amino acid sequence of SEQ ID NO: 146, or
   the PSMA-VH comprises the amino acid sequence of SEQ ID NO: 141, and the PSMA-VL comprises the amino acid sequence of SEQ ID NO: 147, or
   the PSMA-VH comprises the amino acid sequence of SEQ ID NO: 142, and the PSMA-VL comprises the amino acid sequence of SEQ ID NO: 147, or
   the PSMA-VH comprises the amino acid sequence of SEQ ID NO: 143, and the PSMA-VL comprises the amino acid sequence of SEQ ID NO: 147, or
(x) the PSMA-VH comprises the amino acid sequence of SEQ ID NO: 91, and the PSMA-VL comprises the amino acid sequence of SEQ ID NO: 92, or
   the PSMA-VH comprises the amino acid sequence of SEQ ID NO: 148, and the PSMA-VL comprises the amino acid sequence of SEQ ID NO: 150, or
   the PSMA-VH comprises the amino acid sequence of SEQ ID NO: 149, and the PSMA-VL comprises the amino acid sequence of SEQ ID NO: 150, or
   the PSMA-VH comprises the amino acid sequence of SEQ ID NO: 148, and the PSMA-VL comprises the amino acid sequence of SEQ ID NO: 151, or
   the PSMA-VH comprises the amino acid sequence of SEQ ID NO: 149, and the PSMA-VL comprises the amino acid sequence of SEQ ID NO: 151, or
   the PSMA-VH comprises the amino acid sequence of SEQ ID NO: 148, and the PSMA-VL comprises the amino acid sequence of SEQ ID NO: 152, or
   the PSMA-VH comprises the amino acid sequence of SEQ ID NO: 149, and the PSMA-VL comprises the amino acid sequence of SEQ ID NO: 152, or
(xi) the PSMA-VH comprises the amino acid sequence of SEQ ID NO: 93, and the PSMA-VL comprises the amino acid sequence of SEQ ID NO: 94; or
   the PSMA-VH comprises the amino acid sequence of SEQ ID NO: 153, and the PSMA-VL comprises the amino acid sequence of SEQ ID NO: 157; or
   the PSMA-VH comprises the amino acid sequence of SEQ ID NO: 154, and the PSMA-VL comprises the amino acid sequence of SEQ ID NO: 157; or
   the PSMA-VH comprises the amino acid sequence of SEQ ID NO: 155, and the PSMA-VL comprises the amino acid sequence of SEQ ID NO: 157; or
   the PSMA-VH comprises the amino acid sequence of SEQ ID NO: 156, and the PSMA-VL comprises the amino acid sequence of SEQ ID NO: 157; or
   the PSMA-VH comprises the amino acid sequence of SEQ ID NO: 153, and the PSMA-VL comprises the amino acid sequence of SEQ ID NO: 158; or
   the PSMA-VH comprises the amino acid sequence of SEQ ID NO: 154, and the PSMA-VL comprises the amino acid sequence of SEQ ID NO: 158; or
   the PSMA-VH comprises the amino acid sequence of SEQ ID NO: 155, and the PSMA-VL comprises the amino acid sequence of SEQ ID NO: 158; or
   the PSMA-VH comprises the amino acid sequence of SEQ ID NO: 156, and the PSMA-VL comprises the amino acid sequence of SEQ ID NO: 158; or
   (xii) the PSMA-VH comprises the amino acid sequence of SEQ ID NO: 95, and the PSMA-VL comprises the amino acid sequence of SEQ ID NO: 96.

In some embodiments, provided is the antibody according to any one of the above, wherein
(i) the PSMA-VH comprises the amino acid sequence of SEQ ID NO: 112, and the PSMA-VL comprises the amino acid sequence of SEQ ID NO: 115, or
the PSMA-VH comprises the amino acid sequence of SEQ ID NO: 110, and the PSMA-VL comprises the amino acid sequence of SEQ ID NO: 114, or
(vi) the PSMA-VH comprises the amino acid sequence of SEQ ID NO: 127, and the PSMA-VL comprises the amino acid sequence of SEQ ID NO: 128.

In some embodiments, provided is the antibody according to any one of the above, wherein the antibody is a bispecific antibody. In some embodiments, the bispecific antibody specifically binds to PSMA and CD3.

In another aspect, the present disclosure provides a pharmaceutical composition comprising: a therapeutically effective amount of the antigen-binding molecule according to any one of the above or the antibody according to any one of the above, and one or more pharmaceutically acceptable carriers, diluents, buffers, or excipients. In some embodiments, the pharmaceutical composition also comprises at least one second therapeutic agent. In some embodiments, the second therapeutic agent is an antibody capable of specifically binding to CD28 or an antibody capable of specifically binding to PSMA and CD28.

In another aspect, the present disclosure also provides an isolated nucleic acid encoding the antigen-binding molecule according to any one of the above or the antibody according to any one of the above.

In another aspect, the present disclosure also provides a host cell comprising the aforementioned nucleic acid. The host cell cannot develop into a plant or animal.

In another aspect, the present disclosure also provides a method for treating a disease, wherein the method comprises administering to a subject a therapeutically effective amount of the antigen-binding molecule according to any one of the above or the antibody according to any one of the above or the composition thereof.

In another aspect, the present disclosure also provides use of the antigen-binding molecule according to any one of the above or the antibody according to any one of the above or the composition thereof in the preparation of a medicament for treating or preventing a disease.

In another aspect, the present disclosure also provides the antigen-binding molecule according to any one of the above or the antibody according to any one of the above or the composition thereof for use as a medicament. In some embodiments, the medicament is used for treating a disease.

In some embodiments, the disease according to any one of the above is a proliferative disease, a tumor, or an immune disease. In some embodiments, the disease according to any one of the above is selected from the group consisting of any one of the following: prostate cancer, lung cancer, endometrial cancer, kidney cancer, bladder cancer, colorectal cancer (including colon cancer and rectal cancer), and gastric cancer. In some embodiments, the disease is prostate cancer. In some embodiments, the disease is castration-resistant prostate cancer.

In some embodiments, the aforementioned disease is a PSMA-associated disease. In some embodiments, the aforementioned disease is a disease in which PSMA expression is abnormal.

The antigen-binding molecule provided by the present disclosure has the following characteristics: good therapeutic activity, safety, pharmacokinetic properties and druggability (e.g., stability).

### BRIEF DESCRIPTION OF THE DRAWINGS

FIG. 1A: a structural schematic diagram of Format 1.
FIG. 1B: a structural schematic diagram of Format 2.
FIG. 1C: a structural schematic diagram of Format 3.
FIG. 1D: a structural schematic diagram of Format 4.
FIG. 1E: a structural schematic diagram of Format 5.

### DETAILED DESCRIPTION

### Terminology

The terminology used herein is for the purpose of describing embodiments only and is not intended to be limiting. Unless defined otherwise, all technical and scientific terms used herein have the same meaning as commonly understood by one of ordinary skill in the art to which the present disclosure pertains.

Unless the context clearly requires otherwise, throughout the description and the claims, the words "comprise", "have", "include", and the like are to be understood in an inclusive sense as opposed to an exclusive or exhaustive sense; that is to say, in the sense of "including, but not limited to". Unless otherwise specified, "comprise" includes "consist of'. For example, for a PSMA-HCDR1 comprising the amino acid sequence of SEQ ID NO: 1, it specifically encompasses a PSMA-HCDR1 the amino acid sequence of which is set forth in SEQ ID NO: 1.

The three-letter and single-letter codes used in the present disclosure for amino acids are as described in J. Biol. Chem, 243, p3558 (1968).

The term "and/or", e.g., "X and/or Y" should be understood to mean "X and Y" or "X or Y" and should be used to provide explicit support for both meanings or either meaning.

The term "amino acid" refers to naturally occurring and synthetic amino acids, as well as amino acid analogs and amino acid mimics that function in a manner similar to the naturally occurring amino acids. Naturally occurring amino acids are those encoded by genetic codes and those amino acids later modified, e.g., hydroxyproline, γ-carboxyglutamic acid, and O-phosphoserine. Amino acid analogs refer to compounds that have a substantially identical chemical structure (i.e., an α carbon that binds to hydrogen, carboxyl, amino, and an R group) to naturally occurring amino acids, e.g., homoserine, norleucine, methionine sulfoxide, and methioninemethyl sulfonium. Such analogs have a modified R group (e.g., norleucine) or a modified peptide skeleton, but retain a substantially identical chemical structure to naturally occurring amino acids. Amino acid mimics refer to chemical compounds that have a different structure from amino acids, but function in a manner similar to naturally occurring amino acids.

The term "amino acid mutation" includes amino acid substitutions, deletions, insertions and modifications. Any combination of substitutions, deletions, insertions, and modifications can be made to obtain a final construct, as long as the final construct possesses the desired properties, such as reduced binding to the Fc receptor. Amino acid sequence deletions and insertions include deletions and insertions at the amino terminus and/or the carboxyl terminus of a polypeptide chain. Specific amino acid mutations may be amino acid substitutions. In one embodiment, the amino acid mutation is a non-conservative amino acid substitution, i.e., a replacement of one amino acid with another amino acid having different structural and/or chemical properties. Amino acid substitutions include replacement with non-naturally occurring amino acids or with derivatives of the 20 native amino acids (e.g., 4-hydroxyproline, 3-methylhistidine, ornithine, homoserine, and 5-hydroxylysine). Amino acid mutations can be generated using genetic or chemical methods well known in the art. Genetic methods may include site-directed mutagenesis, PCR, gene synthesis, and the like. It is contemplated that methods for altering amino acid side chain groups other than genetic engineering, such as chemical modification, may also be used. Various names may be used herein to indicate the same amino acid mutation. Herein, the expression of "position + amino acid residue" may be used to denote an amino acid residue at a specific position. For example, 366W indicates that an amino acid residue at position 366 is W. T366W indicates that an amino acid residue at position 366 is mutated from the original T to W.

The term "antigen-binding molecule" is used in the broadest sense and encompasses a variety of molecules that specifically bind to an antigen, including but not limited to antibodies, other polypeptides having antigen-binding activity, and antibody fusion proteins in which the two are fused, as long as they exhibit desired antigen-binding activity. The antigen-binding molecule herein comprises a variable region (VH) and a variable region (VL) which together comprise an antigen-binding domain. Illustratively, the antigen-binding molecules herein are bispecific antigen-binding molecules (e.g., bispecific antibodies).

The term "antibody" is used in the broadest sense and encompasses a variety of antibody structures, including but not limited to monoclonal antibodies, polyclonal antibodies; monospecific antibodies, multispecific antibodies (e.g., bispecific antibodies), and full-length antibodies, and antibody fragments (or antigen-binding fragments, or antigen-binding moieties), as long as they exhibit the desired antigen-binding activity. For example, a native IgG antibody is a heterotetrameric protein of about 150,000 Daltons composed of two light chains and two heavy chains linked by a disulfide bond. From the N-terminus to the C-terminus, each heavy chain has one variable region (VH), also known as variable heavy domain or heavy chain variable region, followed by three constant domains (CH1, CH2, and CH3). Similarly, from the N-terminus to the C-terminus, each light chain has one variable region (VL), also known as variable light domain or light chain variable domain, followed by one constant light domain (light chain constant region, CL).

The term "bispecific antibody" refers to an antibody (including an antibody or an antigen-binding fragment thereof, such as a single-chain antibody) capable of specifically binding to two different antigens or at least two different epitopes of the same antigen. Bispecific antibodies of various structures have been disclosed in the prior art. The bispecific antibodies can be classified into IgG-like bispecific antibodies and antibody-fragment-type bispecific antibodies according to the integrity of IgG molecules. The bispecific antibodies can be classified into bivalent, trivalent, tetravalent, or higher-valent bispecific antibodies according to the number of the antigen-binding regions. The bispecific antibodies can be classified into symmetric bispecific antibodies and asymmetric bispecific antibodies according to whether their structures are symmetric or asymmetric. Among them, the antibody fragment-type bispecific antibodies, such as Fab fragments lacking Fc fragments, formed by linking 2 or more Fab fragments in one molecule, have relatively low immunogenicity, small molecular weight, and relatively high tumor tissue permeability; typical antibody structures of this type are, e.g., F(ab)2, scFv-Fab, and (scFv)2-Fab. IgG-like bispecific antibodies (e.g., having Fc fragments) have relatively large molecular weight, and the Fc fragments facilitate the purification of the antibody and increase their solubility and stability, and the Fc fragments may further bind to the receptor FcRn and increase the serum half-life of the antibody; typical structural models of bispecific antibodies are, e.g., KiH, CrossMAb, Triomab quadroma, FcΔAdp, ART-Ig, BiMAb, Biclonics, BEAT, DuoBody, Azymetric, XmAb, 2:1 TCBs, 1Fab-IgG TDB, FynomAb, two-in-one/DAF, scFv-Fab-IgG, DART-Fc, LP- DART, CODV-Fab-TL, HLE-BiTE, F(ab)2-CrossMAb, IgG-(scFv)2, Bs4Ab, DVD-Ig, Tetravalent-DART-Fc, (scFv)4-Fc, CODV-Ig, mAb2, and F(ab)4-CrossMAb (see Aran F. Labrijn et al., Nature Reviews Drug Discovery, volume 18, pages 585-608 (2019); Chen S1 et al., J Immunol Res., Feb. 11, 2019; 2019:4516041).

The term "variable region" or "variable domain" refers to a domain in an antigen-binding molecule that binds to an antigen. Herein, the heavy chain variable region in the antigen-binding moiety that specifically binds to PSMA is denoted by PSMA-VH, and the light chain variable region is denoted by PSMA-VL. The heavy chain variable region in the antigen-binding moiety that specifically binds to CD3 is denoted by CD3-VH, and the light chain variable region is denoted by CD3-VL. VH and VL each comprise four conserved framework regions (FRs) and three complementarity determining regions (CDRs). The term "complementarity determining region" or "CDR" refers to a region in the variable domain that primarily contributes to antigen binding; "framework" or "FR" refers to variable domain residues other than CDR residues. A VH comprises 3 CDRs: HCDR1, HCDR2, and HCDR3; a VL comprises 3 CDRs: LCDR1, LCDR2, and LCDR3. Herein, the 3 CDRs in PSMA-VH are denoted by PSMA-HCDR1, PSMA-HCDR2, and PSMA-HCDR3, respectively; the 3 CDRs in PSMA-VL are denoted by PSMA-LCDR1, PSMA-LCDR2, and PSMA-LCDR3, respectively; the 3 CDRs in CD3-VH are denoted by CD3-HCDR1, CD3-HCDR2, and CD3-HCDR3, respectively; the 3 CDRs in CD3-VL are denoted by CD3-LCDR1, CD3-LCDR2, and CD3-LCDR3, respectively. Each VH and VL is, from N-terminus to C-terminus: FR1, CDR1, FR2, CDR2, FR3, CDR3, FR4. A single VH or VL may be sufficient to provide antigen-binding specificity.

The amino acid sequence boundaries of the CDRs can be determined by a variety of well-known schemes, for example, the "Kabat" numbering scheme (see Kabat et al. (1991), "Sequences of Proteins of Immunological Interest", 5th ed., Public Health Service, National Institutes of Health, Bethesda, MD), the "Chothia" numbering scheme, the "ABM" numbering scheme, the "Contact" numbering scheme (see Martin, ACR. Protein Sequence and Structure Analysis of Antibody Variable Domains[J]. 2001), and the ImMunoGenTics (IMGT) numbering scheme (Lefranc, M.P. et al., Dev. Comp. Immunol., 27, 55-77 (2003); Front Immunol., Oct. 16, 2018; 9: 2278), and the like. The corresponding relationships between the various numbering schemes are well known to those skilled in the art. The numbering schemes of the present disclosure are shown in Table 1 below.

**Table 1. Relationships between CDR numbering schemes**

| CDR | IMGT | Kabat | AbM | Chothia | Contact |
|---|---|---|---|---|---|
| HCDR1 | 27-38 | 31-35 | 26-35 | 26-32 | 30-35 |
| HCDR2 | 56-65 | 50-65 | 50-58 | 52-56 | 47-58 |
| HCDR3 | 105-117 | 95-102 | 95-102 | 95-102 | 93-101 |
| LCDR1 | 27-38 | 24-34 | 24-34 | 24-34 | 30-36 |
| LCDR2 | 56-65 | 50-56 | 50-56 | 50-56 | 46-55 |
| LCDR3 | 105-117 | 89-97 | 89-97 | 89-97 | 89-96 |

Unless otherwise stated, the "Kabat" numbering scheme is applied to the sequences of the variable regions and CDRs in examples of the present disclosure. Although one numbering scheme (e.g., Kabat) is employed to define amino acid residues in specific embodiments, corresponding technical solutions for other numbering schemes are to be considered as equivalent technical solutions.

The term "antibody fragment" refers to a molecule different from an intact antibody, which comprises a moiety of an intact antibody that retains the antigen-binding ability of the intact antibody. Examples of antibody fragments include, but are not limited to, Fv, Fab, Fab', Fab'-SH, F(ab')₂, single-domain antibodies, single-chain Fab (scFab), diabodies, linear antibodies, single-chain antibody molecules (e.g., scFv), and multispecific antibodies formed from antibody fragments.

The term "Fc region" or "fragment crystallizable region" is used to define the C-terminal region of the heavy chain of an antibody, including native Fc regions and modified Fc regions. In some embodiments, the Fc region comprises two subunits, which may be identical or different. In some embodiments, the Fc region of the human IgG heavy chain is defined as extending from the amino acid residue at position Cys226 or from Pro230 to its carboxyl terminus. Suitable native sequence Fc regions for the antibodies described herein include human IgG1. Unless otherwise indicated, the numbering scheme for the Fc region is the EU index.

The term "Titin chain" refers to, in a Titin protein, a peptide fragment that is 78-118 amino acids in length and comprises a Titin Ig-like 152 domain, or a functional variant thereof. The Titin chain is capable of binding to the Obscurin chain to form a dimerized complex.

The term "Obscurin chain" refers to, in the Obscurin protein, a peptide fragment that is 87-117 amino acids in length and comprises an Obscurin Ig-like 1 domain, or a functional variant thereof, or to, in the Obscurin-like 1 protein, a peptide fragment that is 78-118 amino acids in length and comprises an Obscurin-like Ig-like 1 domain, or a functional variant thereof. The Obscurin chain is capable of binding to the Titin chain to form a dimerized complex.

The Titin chain and the Obscurin chain of the present disclosure can be used to substitute the CH1 and CL in the Fab to form a substituted Fab (Fab-S), and the substitution does not affect the binding of the antigen-binding molecule to the antigen or an epitope thereof.

The term "chimeric" antibody refers to an antibody in which a portion of the heavy and/or light chain is derived from a particular source or species, while the remainder of the heavy and/or light chain is derived from a different source or species.

The term "humanized" antibody refers to an antibody that retains the reactivity of a non-human antibody while having low immunogenicity in humans. For example, the humanization can be achieved by retaining the non-human CDRs and substituting the remainder of the antibody with human counterparts (i.e., the constant regions and the framework region portion of the variable regions).

The term "affinity" refers to the strength of the non-covalent interaction between a single binding site of a molecule (e.g., an antibody) and its binding ligand (e.g., an antigen). Unless otherwise indicated, as used herein, "binding affinity" refers to an internal binding affinity that reflects a 1:1 interaction between members of a binding pair (e.g., an antibody and an antigen). The affinity of a molecule X for its ligand Y can be generally expressed by the equilibrium dissociation constant (KD). Affinity can be determined by conventional methods known in the art, including those described herein. The term "kassoc" or "ka" refers to the association rate of a particular antibody-antigen interaction, while the term "kdis" or "kd" as used herein refers to the dissociation rate of a particular antibody-antigen interaction. As used herein, the term "KD" refers to the equilibrium dissociation constant, which is obtained from the ratio of kd to ka (i.e., kd/ka) and expressed as molar concentration (M). The KD value of an antibody can be measured using methods known in the art, such as surface plasmon resonance, ELISA, or solution equilibrium titration (SET).

The term "monoclonal antibody" refers to a population of substantially homogeneous antibodies or a member thereof, that is, the amino acid sequences of the antibody molecules comprised in the population are identical, except for a small number of natural mutations that may exist. In contrast, a "polyclonal antibody" generally comprises a plurality of different antibodies having different amino acid sequences in their variable domains, which are generally specific for different epitopes. "Monoclonal" refers to the characteristics of an antibody obtained from a substantially homogeneous population of antibodies, and should not be construed as requiring the production of the antibody by any particular method. In some embodiments, the antibody provided by the present disclosure is a monoclonal antibody.

The term "antigen" refers to a molecule or a portion of a molecule that is capable of being selectively recognized or bound by an antigen-binding molecule (e.g., an antibody). An antigen may have one or more epitopes capable of interacting with different antigen-binding molecules (e.g., antibodies).

The term "epitope" refers to an area or region on an antigen that is capable of specifically binding to an antibody or an antigen-binding fragment thereof. Epitopes can be formed by contiguous amino acids (linear epitopes) or comprise non-contiguous amino acids (conformational epitopes), e.g., non-contiguous amino acids that are in spatial proximity to each other due to folding of an antigen (i.e., by tertiary folding of an antigen of a protein nature). The difference between the conformational epitope and the linear epitope is that in the presence of denaturing solvents, the binding of the antibody to the conformational epitope is lost. An epitope comprises at least 3, at least 4, at least 5, at least 6, at least 7, or 8-10 amino acids in a unique spatial conformation. Screening for antibodies that bind to particular epitopes (i.e., those that bind to identical epitopes) can be performed using routine methods in the art, such as but not limited to, alanine scanning, peptide blotting (see Meth. Mol. Biol. 248 (2004) 443-463), peptide cleavage analysis, epitope excision, epitope extraction, chemical modification of the antigen (see Prot. Sci. 9 (2000) 487-496), and cross-blocking (see "Antibodies", Harlow and Lane (Cold Spring Harbor Press, Cold Spring Harb., NY)).

The term "capable of specifically binding", "specifically binding", or "binding" means that an antibody is capable of binding to a certain antigen or epitope with higher affinity than to other antigens or epitopes. Generally, an antibody binds to an antigen or epitope with an equilibrium dissociation constant (KD) of about 1×10⁻⁷ M or less (e.g., about 1×10⁻⁸ M or less). In some embodiments, the KD for the binding of an antibody to an antigen is 10% or less (e.g., 1%) of the KD for the binding of the antibody to a non-specific antigen (e.g., BSA or casein). KD may be determined using known methods, for example, by a FACS or surface plasmon resonance assay. However, an antibody that specifically binds to an antigen or an epitope thereof may have cross-reactivity to other related antigens, e.g., to corresponding antigens from other species (homologous), such as humans or monkeys, e.g., *Macaca fascicularis* (cynomolgus, cyno), *Pan troglodytes* (chimpanzee, chimp), or *Callithrix jacchus* (commonmarmoset, marmoset).

The term "not bind" means that the antibody is not capable of binding to a certain antigen or an epitope thereof in the manner of the specific binding described above, for example, when the antibody binds to the antigen or the epitope thereof with an equilibrium dissociation constant (KD) of about 1 × 10⁻⁶ M or greater.

The term "antigen-binding moiety" refers to a polypeptide molecule that specifically binds to a target antigen or an epitope thereof. A specific antigen-binding moiety includes an antigen-binding domain of an antibody, e.g., comprises a heavy chain variable region and a light chain variable region. The term "antigen-binding moiety that specifically binds to PSMA" refers to a moiety that is capable of binding to PSMA or an epitope thereof with sufficient affinity, such that a molecule containing the moiety can be used as a diagnostic agent and/or therapeutic agent targeting PSMA. For example, the antigen-binding moiety that specifically binds to PSMA has the following equilibrium dissociation constant (KD): < about 1 × 10⁻⁸ M or less, as measured by a surface plasmon resonance assay. Antigen-binding moieties include antibody fragments as defined herein, e.g., a Fab, a substituted Fab, or an scFv.

The term "linker" refers to a linker unit that links two polypeptide fragments. Herein, the linkers present in the same structural formula may be identical or different. The linker may be a peptide linker comprising one or more amino acids, typically about 1-30, 2-24, or 3-15 amino acids. The linkers used herein may be identical or different. When "-" appears in a structural formula, it means that the units on both sides are directly linked by a covalent bond.

"Tm" is the temperature of dissolution and denaturation (endogenous fluorescence). When the protein is denatured (by heating or by a denaturant), the tertiary structure is opened and the aromatic amino acid microenvironment changes, resulting in a change in the emission fluorescence spectrum. In the present disclosure, Tm1 refers to the temperature at which the fluorescence value changes to half of the maximum value.

"Tonset" is the onset temperature of denaturation. It refers to the temperature at which the protein begins to denature, i.e., the temperature at which the fluorescence value begins to change.

"Tagg" is the onset temperature of aggregation. The temperature at which the sample begins to aggregate is monitored by detecting aggregates at two wavelengths of 266 nm and 473 nm by static light scattering. Tagg 266 refers to the onset temperature of aggregation monitored at 266 nm.

The term "nucleic acid" is used interchangeably herein with the term "polynucleotide" and refers to deoxyribonucleotide or ribonucleotide and a polymer thereof in either single-stranded or double-stranded form. The term encompasses nucleic acids comprising known nucleotide analogs or modified backbone residues or linkages, which are synthetic, naturally occurring, and non-naturally occurring, have similar binding properties to the reference nucleic acid, and are metabolized in a manner similar to the reference nucleotide. Examples of such analogs include, but are not limited to, phosphorothioate, phosphoramidate, methylphosphonate, chiral-methylphosphonate, 2-O-methyl ribonucleotide, and peptide-nucleic acid (PNA). "Isolated" nucleic acid refers to a nucleic acid molecule that has been separated from components of its natural environment. An isolated nucleic acid encoding the antigen-binding molecule refers to one or more nucleic acid molecules encoding antibody heavy and light chains (or fragments thereof), including such nucleic acid molecule(s) in a single vector or separate vectors, and such nucleic acid molecule(s) present at one or more locations in a host cell. Unless otherwise stated, a particular nucleic acid sequence also implicitly encompasses conservatively modified variants thereof (e.g., degenerate codon substitutions) and complementary sequences, as well as the sequence explicitly indicated. Specifically, as detailed below, degenerate codon substitutions may be obtained by generating sequences in which the third position of one or more selected (or all) codons is substituted with degenerate bases and/or deoxyinosine residues.

The terms "polypeptide" and "protein" are used interchangeably herein and refer to a polymer of amino acid residues. The terms apply to amino acid polymers in which one or more amino acid residues are corresponding artificial chemical mimics of naturally occurring amino acids, as well as to naturally occurring amino acid polymers and non-naturally occurring amino acid polymers. Unless otherwise stated, a particular polypeptide sequence also implicitly encompasses conservatively modified variants thereof.

The term "sequence identity" refers to the degree (percentage) to which the amino acids/nucleic acids of two sequences are identical at equivalent positions when the two sequences are optimally aligned. In the alignment process, gaps may be allowed to be introduced, if necessary, to achieve the maximum percent sequence identity, but any conservative substitution is not considered a part that constitutes sequence identity. To determine percent sequence identity, alignments can be accomplished by techniques known to those skilled in the art, for example, using publicly available computer software, such as BLAST, BLAST-2, ALIGN, ALIGN-2, or Megalign (DNASTAR) software. Those skilled in the art can determine parameters suitable for measuring alignment, including any algorithms required to achieve maximum alignment of the full length of the aligned sequences.

The term "fusion" or "linkage" means that components (e.g., antigen-binding moieties and Fc domains) are covalently linked directly or via a linker.

The term "vector" means a polynucleotide molecule capable of transporting another polynucleotide linked thereto. One type of vector is a "plasmid", which refers to a circular double-stranded DNA loop into which additional DNA segments can be ligated. Another type of vector is a viral vector, such as an adeno-associated viral vector (AAV or AAV2), wherein additional DNA segments can be ligated into the viral genome. Certain vectors are capable of autonomous replication in a host cell into which they are introduced (e.g., bacterial vectors having a bacterial origin of replication and episomal mammalian vectors). Other vectors (e.g., non-episomal mammalian vectors) can be integrated into the genome of a host cell upon introduction into the host cell, and thereby are replicated along with the host genome.

The term "expression vector" or "expression construct" refers to a vector that is applied to transform a host cell and comprises a nucleic acid sequence that directs and/or controls (along with the host cell) the expression of one or more heterologous coding regions operably linked thereto. Expression constructs may include, but are not limited to, sequences that affect or control transcription and translation and affect RNA splicing of a coding region operably linked thereto in the presence of an intron. The terms "host cell", "host cell line", and "host cell culture" are used interchangeably and refer to cells into which exogenous nucleic acids have been introduced, including progenies of such cells. Host cells include "transformants" and "transformed cells", which include primary transformed cells and progeny derived therefrom, regardless of the number of passages. Progeny may not be completely identical to parent cells in terms of nucleic acid content and may contain mutations. As used herein, the term includes mutant progenies that have the same function or biological activity as the cells screened or selected from the primary transformed cells. Host cells include prokaryotic and eukaryotic host cells, wherein eukaryotic host cells include, but are not limited to, mammalian cells, insect cell lines, plant cells, and fungal cells. Mammalian host cells include human, mouse, rat, canine, monkey, porcine, goat, bovine, equine, and hamster cells, including but not limited to, Chinese hamster ovary (CHO) cells, NSO, SP2 cells, HeLa cells, baby hamster kidney (BHK) cells, monkey kidney cells (COS), human hepatocellular carcinoma cells (e.g., Hep G2), A549 cells, 3T3 cells, and HEK-293 cells. Fungal cells include yeast and filamentous fungal cells, including, for example, *Pichiapastoris, Pichia finlandica, Pichia trehalophila, Pichia koclamae, Pichia membranaefaciens, Pichia minuta (Ogataea minuta, Pichia lindneri), Pichiaopuntiae*, *Pichia thermotolerans, Pichia salictaria*, *Pichia guercuum*, *Pichia pijperi*, *Pichia stiptis*, *Pichia methanolica*, Pichia, *Saccharomycescerevisiae*, Saccharomyces, *Hansenula polymorpha*, Kluyveromyces, *Kluyveromyces lactis, Candida albicans, Aspergillus nidulans, Aspergillus niger, Aspergillus oryzae*, *Trichoderma reesei*, *Chrysosporium lucknowense*, *Fusarium* sp., *Fusarium gramineum*, *Fusarium venenatum*, *Physcomitrella patens*, and *Neurospora crassa.* Pichia, any Saccharomyces, *Hansenula polymorpha*, any Kluyveromyces, *Candida albicans*, any Aspergillus, *Trichoderma reesei*, *Chrysosporium lucknowense*, any Fusarium, *Yarrowia lipolytica*, and *Neurospora crassa.* The host cell of the present patent does not include objects not authorized by the Patent Laws.

"Optional" or "optionally" means that the event or circumstance subsequently described may, but does not necessarily, occur, and this description includes instances where the event or circumstance occurs or does not occur.

The term "pharmaceutical composition" refers to a mixture comprising one or more of the antigen-binding molecules or antibodies described herein and other chemical components; the other components are, for example, physiological/pharmaceutically acceptable carriers and excipients.

The term "pharmaceutically acceptable carrier, diluent, buffer, or excipient" refers to an ingredient in a pharmaceutical formulation that is different from the active ingredient and is not toxic to the subject. Pharmaceutically acceptable carriers, diluents, buffers, or excipients include, but are not limited to, buffers, excipients, stabilizers, or preservatives.

The term "subject" or "individual" includes humans and non-human animals. Non-human animals include all vertebrates (e.g., mammals and non-mammals) such as non-human primates (e.g., cynomolgus monkeys), sheep, dogs, cows, chickens, amphibians, and reptiles. Unless clearly indicated, the terms "patient" and "subject" are used interchangeably herein. As used herein, the term "cynomolgus monkey (cyno)" or "cynomolgus" refers to Macaca fascicularis. In certain embodiments, the individual or subject is a human.

"Administrating" or "giving", when applied to animals, humans, experimental subjects, cells, tissue, organs, or biological fluids, refers to contact of an exogenous drug, a therapeutic agent, a diagnostic agent, or a composition with the animals, humans, subjects, cells, tissue, organs, or biological fluids.

The term "sample" refers to a collection isolated from a subject (such as fluids, cells, or tissues), as well as fluids, cells, or tissues present in a subject. Exemplary samples are biological fluids (such as blood; serum; serosal fluids; plasma; lymph; urine; saliva; cystic fluids; tears; excretions; sputum; mucosal secretions of secretory tissue or organs; vaginal secretions; ascites; fluids in the pleura; pericardium; peritoneum; fluids from abdominal cavity and other body cavities; fluids collected from bronchial lavage; synovial fluids; liquid solutions in contact with a subject or biological source, e.g., culture media (including conditioned media); lavage fluids; and the like), tissue biopsy samples, fine needle punctures, surgically excised tissues, organ cultures, or cell cultures.

"Treatment" or "treat" (and grammatical variations thereof) refers to clinical intervention intended to be applied to the treated individual, which may be performed either for prophylactic purposes or during the course of clinical pathology. Desirable effects of the treatment include, but are not limited to, preventing the occurrence or recurrence of a disease, alleviating symptoms, alleviating/reducing any direct or indirect pathological consequences of the disease, preventing metastasis, decreasing the rate of disease progression, ameliorating or alleviating the disease state, and regressing or improving prognosis. In some embodiments, the molecule of the present disclosure is used to delay the development of or slow the progression of disease.

"Effective amount" is generally an amount sufficient to reduce the severity and/or frequency of symptoms, eliminate symptoms and/or underlying causes, prevent the appearance of symptoms and/or their underlying causes, and/or ameliorate or improve damage caused by or associated with a disease state. In some embodiments, the effective amount is a therapeutically effective amount or a prophylactically effective amount.

"Therapeutically effective amount" is an amount sufficient to treat a disease state or symptom, particularly a state or symptom associated with the disease state, or to otherwise prevent, hinder, delay, or reverse the progression of the disease state or any other undesirable symptoms associated with the disease in any way.

"Prophylactically effective amount" is an amount that, when administered to a subject, will have a predetermined prophylactic effect, e.g., preventing or delaying the onset (or recurrence) of the disease state, or reducing the likelihood of the onset (or recurrence) of the disease state or associated symptoms. A complete therapeutic or prophylactic effect does not necessarily occur after administration of one dose and may occur after administration of a series of doses. Thus, a therapeutically or prophylactically effective amount may be administered in one or more doses. "Therapeutically effective amount" and "prophylactically effective amount" may vary depending on a variety of factors such as the disease state, age, sex, and weight of the individual, and the ability of a therapeutic agent or combination of therapeutic agents to elicit a desired response in the individual. Exemplary indicators of an effective therapeutic agent or combination of therapeutic agents include, for example, improved health condition of a patient.

### Target Molecule

"PSMA" should be understood in the broadest sense and is intended to encompass various forms of molecules of PSMA in various stages in mammals, such as, but not limited to, molecules produced by the PSMA gene during amplification, replication, transcription, splicing, processing, translation, and modification, e.g., precursor PSMA, mature PSMA, PSMA expressed on the membrane, PSMA splice variants, modified PSMA, or fragments thereof; the term also encompasses PSMA artificially prepared or expressed *in vitro.*

"CD3" should be understood in the broadest sense and is intended to encompass various forms of molecules of CD3 in various stages in mammals, such as, but not limited to, molecules produced by the BCMA gene during amplification, replication, transcription, splicing, processing, translation, and modification, e.g., precursor CD3, mature CD3, CD3 expressed on the membrane, CD3 splice variants, modified CD3, or fragments thereof; the term also encompasses CD3 artificially prepared or expressed *in vitro.*

### Antigen-Binding Molecules of the Present Disclosure

The present disclosure provides an antigen-binding molecule having a number of advantageous properties, such as high affinity for PSMA and PSMA-expressing cells, *in vitro* killing activity, therapeutic activity, safety, pharmacokinetic properties, and druggability (e.g., yield, purity, and stability).

### Exemplary Antigen-Binding Molecules

The antigen-binding molecule of the present disclosure includes bispecific antigen-binding molecules (e.g., bispecific antibodies) that specifically bind to PSMA and CD3 and anti-PSMA antibodies. Particularly, the antigen-binding molecule of the present disclosure has one or more of the following:
a. High affinity of antibodies for PSMA. In some embodiments, the antibody binds to PSMA with an EC₅₀ of less than 10 µg/mL, 1.5 µg/mL, 1.3 µg/mL, 1.1 µg/mL, 1 µg/mL, 0.7 µg/mL, 0.6 µg/mL, 0.5 µg/mL, 0.4 µg/mL, 0.3 µg/mL, 0.2 µg/mL, 0.1 µg/mL, 0.09 µg/mL, 0.08 µg/mL, 0.07 µg/mL, 0.06 µg/mL, 0.05 µg/mL, 0.04 µg/mL, or 0.03 µg/mL, as measured by ELISA.
b. High affinity of antigen-binding molecules (e.g., bispecific antibodies) for PSMA. In some embodiments, the antigen-binding molecule binds to human PSMA at 25 °C with a KD of less than 1 × 10⁻⁷ M, 1 × 10⁻⁸ M, 9 × 10⁻⁹ M, 8 × 10⁻⁹ M, 7 × 10⁻⁹ M, 6 × 10⁻⁹ M, 5 × 10⁻⁹ M, 4 × 10⁻⁹ M, 3 × 10⁻⁹ M, 2.9 × 10⁻⁹ M, 2.8 × 10⁻⁹ M, 2.7 × 10⁻⁹ M, 2.6 × 10⁻⁹ M, 2.5 × 10⁻⁹ M, 2.4 × 10⁻⁹ M, 2.3 × 10⁻⁹ M, 2.2 × 10⁻⁹ M, 2.1 × 10⁻⁹ M, 2.0 × 10⁻⁹ M, 1.9 × 10⁻⁹ M, 1.8 × 10⁻⁹ M, 1.7 × 10⁻⁹ M, 1.6 × 10⁻⁹ M, 1.5 × 10⁻⁹ M, or 1.4 × 10⁻⁹ M, as measured by surface plasmon resonance.
c. High affinity for cell surface PSMA. The antibody binds to cell surface PSMA with an EC₅₀ of less than 10 µg/mL, 2 µg/mL, 1.5 µg/mL, 1.3 µg/mL, 1.2 µg/mL, 1.1 µg/mL, 1 µg/mL, 0.1 µg/mL, 0.09 µg/mL, 0.08 µg/mL, 0.07 µg/mL, 0.06 µg/mL, 0.05 µg/mL, or 0.04 µg/mL, as measured by ELISA. The cell is CHO overexpressing human PSMA.
d. Specific *in vitro* killing activity against PSMA-expressing cells.
e. Induction of low-level release of cytokines (IL6 and IFNγ).
f. Stronger *in vivo* therapeutic activity.

The present disclosure provides an antigen-binding molecule comprising at least one antigen-binding moiety that specifically binds to PSMA and at least one antigen-binding moiety that specifically binds to CD3, wherein the antigen-binding moiety that specifically binds to PSMA comprises a PSMA-VH and a PSMA-VL, and the antigen-binding moiety that specifically binds to CD3 comprises a CD3-VH and a CD3-VL.

The present disclosure also provides an isolated antibody capable of specifically binding to PSMA, wherein the antibody comprises a PSMA-VH and a PSMA-VL. Specifically, the examples herein disclose a series of antibodies 2, 13, 15, 19, 27, 31, 41, 43, 46, 50, 71, and 73. Antibody 15 is described below as an example of the antibodies herein.

The PSMA-VH of the antigen-binding molecule that specifically binds to PSMA and CD3 or the anti-PSMA antibody has: a PSMA-HCDR1 the amino acid sequence of which is set forth in SEQ ID NO: 13, a PSMA-HCDR2 the amino acid sequence of which is set forth in SEQ ID NO: 14, and a PSMA-HCDR3 the amino acid sequence of which is set forth in SEQ ID NO: 15, and the PSMA-VL has: a PSMA-LCDR1 the amino acid sequence of which is set forth in SEQ ID NO: 16, a PSMA-LCDR2 the amino acid sequence of which is set forth in SEQ ID NO: 17, and a PSMA-LCDR3 the amino acid sequence of which is set forth in SEQ ID NO: 18.

In some embodiments, provided is the antigen-binding molecule or the antibody according to the above, wherein the PSMA-VH and/or the PSMA-VL are/is murine or humanized. In some embodiments, the PSMA-VH and/or the PSMA-VL are/is humanized. In some embodiments, FR1, FR2, and FR3 of the humanized PSMA-VH have at least 60%, 70%, or 80% sequence identity to FR1, FR2, and FR3 of SEQ ID NO: 77, and FR4 of the humanized PSMA-VH has at least 80% or 90% sequence identity to FR4 of SEQ ID NO: 77; FR1, FR2, and FR3 of the humanized PSMA-VL have at least 60%, 70%, or 80% sequence identity to FR1, FR2, and FR3 of SEQ ID NO: 78, and/or FR4 of the humanized PSMA-VL has at least 80% or 90% sequence identity to FR4 of SEQ ID NO: 78. In some embodiments, the PSMA-VH has an FR1, an FR2, and an FR3 derived from IGHV1-3*01 and an FR4 derived from IGHJ1*01, and is unsubstituted or has one or more amino acid substitutions selected from the group consisting of 1E, 2F, 28S, 44C, 48I, 67A, 69L, 71V, 73Q, and 76T; and/or the PSMA-VL has an FR1, an FR2, and an FR3 derived from IGKV1-27*01 and an FR4 derived from IGKJ2*01, and is unsubstituted or has one or more amino acid substitutions selected from the group consisting of 43S, 60D, and 100C. In some embodiments, 44C and 100C are present simultaneously. In some embodiments, the variable regions and CDRs are defined according to the Kabat numbering scheme.

In some embodiments, provided is the antigen-binding molecule or the antibody according to any one of the above, wherein the amino acid sequence of the PSMA-VH has at least 90%, 95%, 96%, 97%, 98%, or 99% sequence identity to SEQ ID NO: 77, 107, 108, 109, 110, 111, or 112, and the amino acid sequence of the PSMA-VL has at least 90%, 95%, 96%, 97%, 98%, or 99% sequence identity to SEQ ID NO: 78, 113, 114, or 115. In some embodiments, the amino acid sequence of the PSMA-VH is set forth in SEQ ID NO: 77, 107, 108, 109, 110, 111, or 112, and the amino acid sequence of the PSMA-VL is set forth in SEQ ID NO: 78, 113, 114, or 115. In some embodiments, the amino acid sequence of the PSMA-VH is set forth in SEQ ID NO: 107, 108, 109, 110, 111, or 112, and the amino acid sequence of the PSMA-VL is set forth in SEQ ID NO: 113, 114, or 115.

In some embodiments, provided is the antigen-binding molecule or the antibody according to any one of the above, wherein
the amino acid sequence of the PSMA-VH is set forth in SEQ ID NO: 112, and the amino acid sequence of the PSMA-VL is set forth in SEQ ID NO: 115, or
the amino acid sequence of the PSMA-VH is set forth in SEQ ID NO: 110, and the amino acid sequence of the PSMA-VL is set forth in SEQ ID NO: 114, or
the amino acid sequence of the PSMA-VH is set forth in SEQ ID NO: 77, and the amino acid sequence of the PSMA-VL is set forth in SEQ ID NO: 78, or
the amino acid sequence of the PSMA-VH is set forth in SEQ ID NO: 107, and the amino acid sequence of the PSMA-VL is set forth in SEQ ID NO: 113, or
the amino acid sequence of the PSMA-VH is set forth in SEQ ID NO: 108, and the amino acid sequence of the PSMA-VL is set forth in SEQ ID NO: 113, or
the amino acid sequence of the PSMA-VH is set forth in SEQ ID NO: 109, and the amino acid sequence of the PSMA-VL is set forth in SEQ ID NO: 113, or
the amino acid sequence of the PSMA-VH is set forth in SEQ ID NO: 110, and the amino acid sequence of the PSMA-VL is set forth in SEQ ID NO: 113, or
the amino acid sequence of the PSMA-VH is set forth in SEQ ID NO: 111, and the amino acid sequence of the PSMA-VL is set forth in SEQ ID NO: 113, or
the amino acid sequence of the PSMA-VH is set forth in SEQ ID NO: 107, and the amino acid sequence of the PSMA-VL is set forth in SEQ ID NO: 114, or
the amino acid sequence of the PSMA-VH is set forth in SEQ ID NO: 108, and the amino acid sequence of the PSMA-VL is set forth in SEQ ID NO: 114, or
the amino acid sequence of the PSMA-VH is set forth in SEQ ID NO: 109, and the amino acid sequence of the PSMA-VL is set forth in SEQ ID NO: 114, or
the amino acid sequence of the PSMA-VH is set forth in SEQ ID NO: 111, and the amino acid sequence of the PSMA-VL is the amino acid sequence set forth in SEQ ID NO: 114.

In some embodiments, provided is the antigen-binding molecule or the antibody according to any one of the above, wherein the amino acid sequence of the PSMA-VH is set forth in SEQ ID NO: 112, and the amino acid sequence of the PSMA-VL is set forth in SEQ ID NO: 115, or
the amino acid sequence of the PSMA-VH is set forth in SEQ ID NO: 110, and the amino acid sequence of the PSMA-VL is set forth in SEQ ID NO: 114.

In some embodiments, provided is the antigen-binding molecule according to the above, wherein the CD3-VH has: a CD3-HCDR1 the amino acid sequence of which is set forth in SEQ ID NO: 160, a CD3-HCDR2 the amino acid sequence of which is set forth in SEQ ID NO: 161, and a CD3-HCDR3 the amino acid sequence of which is set forth in SEQ ID NO: 162; and the CD3-VL has: a CD3-LCDR1 the amino acid sequence of which is set forth in SEQ ID NO: 163, a CD3-LCDR2 the amino acid sequence of which is set forth in SEQ ID NO: 164, and a CD3-LCDR3 the amino acid sequence of which is set forth in SEQ ID NO: 165.

In some embodiments, provided is the antigen-binding molecule according to any one of the above, wherein the CD3-VH and/or the CD3-VL are/is murine or humanized. In some embodiments, the CD3-VH and/or the CD3-VL are/is humanized. In some embodiments, the amino acid sequence of the CD3-VH is set forth in SEQ ID NO: 166, and the amino acid sequence of the CD3-VL is set forth in SEQ ID NO: 167. In some embodiments, the variable regions and CDRs are defined according to the Kabat numbering scheme.

According to the technical solutions of the series of antibodies 2, 13, 19, 27, 31, 41, 43, 46, 50, 71, and 73 disclosed in the examples herein, these antibodies have a technical solution range similar to that of the antibody 15 described above.

### Structure of Antigen-Binding Molecule

The bispecific antigen-binding molecule of the present disclosure is not limited to a particular molecular structure, as long as it has the desired antigen-binding function. For example, the bispecific antigen-binding molecule herein may be bivalent (1 + 1), trivalent (2 + 1), or tetravalent (2 + 2). The antigen-binding moieties in the antigen-binding molecule may be any antibody fragments having antigen-binding activity that are fused via a peptide linker. The peptide linkers of the present disclosure (e.g., linkers 1 through 11) can be any suitable peptide chains, as long as the antigen-binding molecules are capable of exhibiting the desired antigen-binding activity. For example, the peptide linkers may be flexible peptides of 1-50 or 3-20 amino acid residues. In some embodiments, the peptide linkers each independently have a structure of L₁-(GGGGS)n-L₂, wherein Li is a bond, A, GS, GGS, GGGS (SEQ ID NO: 276), SGGGGS (SEQ ID NO: 178), or GGGTKLTVLGGG (SEQ ID NO: 177), n is 0, 1, 2, 3, 4, 5, 6, 7, 8, 9, or 10, L2 is a bond, G, GG, GGG, or GGGG (SEQ ID NO: 179), and the peptide linkers are not bonds. In some embodiments, the peptide linker is 3-15 amino acid residues in length. In some embodiments, the peptide linkers each independently have a structure of (GGGGS)n, wherein n is 1, 2, or 3. In some embodiments, the peptide linkers are GGGGS (SEQ ID NO: 173), GGGGSGGGGSGGGGS (SEQ ID NO: 176), GGGTKLTVLGGG (SEQ ID NO: 177), SGGGGS (SEQ ID NO: 178), or GGG. In some embodiments, the amino acid sequences of the linker 1, linker 3, and linker 10 are set forth in SEQ ID NO: 176; the amino acid sequence of the linker 2 is set forth in SEQ ID NO: 178; the amino acid sequences of the linker 4 and linker 11 are GGG; the amino acid sequences of the linker 5, linker 6, linker 7, linker 8, and linker 9 are set forth in SEQ ID NO: 173.

Illustratively, the antigen-binding molecule of the present disclosure comprises one first chain having a structure represented by formula (a-i) and one second chain having a structure represented by formula (b):
formula (a-i)
   [PSMA-VH]-[GGGGSGGGGSGGGGS]-[PSMA-VL]-[SGGGGS]-[CD3-VH]-[GGG GSGGGGSGGGGS]-[CD3-VL]-[GGG]-[Fc2], and
formula (b) [Fc1];
   the structures represented by formulas (a-i) and (b) are arranged from N-terminus to C-terminus.

An exemplary bivalent antigen-binding molecule has:
the antigen-binding molecule has: one first chain the amino acid sequence of which is set forth in SEQ ID NO: 192 and one second chain the amino acid sequence of which is set forth in SEQ ID NO: 169; or
the antigen-binding molecule has: one first chain the amino acid sequence of which is set forth in SEQ ID NO: 191 and one second chain the amino acid sequence of which is set forth in SEQ ID NO: 169.

### Variants of Antigen-Binding Molecules

In certain embodiments, amino acid sequence variants of the antigen-binding molecules provided herein are encompassed. For example, it may be desirable to improve the binding affinity and/or other biological properties of the antibody. Amino acid sequence variants of an antibody can be prepared by introducing appropriate modifications into the nucleotide sequence encoding the antibody, or by peptide synthesis. Such modifications include, for example, deletions from, and/or insertions into and/or substitutions of residues within the amino acid sequence of the antigen-binding molecule. Any combination of deletion, insertion, and substitution can be made to obtain the final construct, provided that the final construct possesses the desired characteristics, e.g., antigen binding.

### Variants with Substitutions, Insertions, and Deletions

In certain embodiments, antigen-binding molecule variants having one or more amino acid substitutions are provided. Substitutions may be made in the CDRs and FRs. Conservative substitutions are shown in Table 2 under the heading of "Preferred substitution". More substantial changes are provided in Table 2 under the heading of "exemplary substitution", and as further described below with reference to amino acid side chain classes. Amino acid substitutions can be introduced into an antibody of interest and the products are screened for a desired activity, e.g., retained/improved antigen binding, reduced immunogenicity, or improved ADCC or CDC.

**Table 2. Amino acid substitutions**

| Original residue | Exemplary substitution | Preferred substitution |
|---|---|---|
| Ala (A) | Val; Leu; Ile | Val |
| Arg (R) | Lys; Gln; Asn | Lys |
| Asn (N) | Gln; His; Asp,Lys; Arg | Gln |
| Asp (D) | Glu; Asn | Glu |
| Cys (C) | Ser; Ala | Ser |
| Gln (Q) | Asn; Glu | Asn |
| Glu (E) | Asp; Gln | Asp |
| Gly (G) | Ala | Ala |
| His (H) | Asn; Gln; Lys; Arg | Arg |
| Ile (I) | Leu; Val; Met; Ala; Phe; norleucine | Leu |
| Leu (L) | Norleucine; Ile; Val; Met; Ala; Phe | Ile |
| Lys (K) | Arg; Gln; Asn | Arg |
| Met (M) | Leu; Phe; Ile | Leu |
| Phe (F) | Trp; Leu; Val; Ile; Ala; Tyr | Tyr |
| Pro (P) | Ala | Ala |
| Ser (S) | Thr | Thr |
| Thr (T) | Ser | Ser |
| Trp (W) | Tyr; Phe | Tyr |
| Tyr (Y) | Trp; Phe; Thr; Ser | Phe |
| Val (V) | Ile; Leu; Met; Phe; Ala; norleucine | Leu |

According to common side-chain properties, amino acids can be grouped as follows:
(1) hydrophobic: norleucine, Met, Ala, Val, Leu, Ile;
(2) neutral, hydrophilic: Cys, Ser, Thr, Asn, Gln;
(3) acidic: Asp, Glu;
(4) basic: His, Lys, Arg;
(5) residues affecting chain orientation: Gly, Pro;
(6) aromatic: Trp, Tyr, Phe.

A non-conservative substitution refers to the substitution of a member of one class for a member of another class.

One type of substitutional variant involves substituting one or more CDR residues of a parent antibody (e.g., a humanized or human antibody). Generally, the resulting variant selected for further study will have changes (e.g., improvements) in certain biological properties (e.g., increased affinity or reduced immunogenicity) relative to the parent antibody, and/or will have substantially retained certain biological properties of the parent antibody. An exemplary substitutional variant is an affinity-matured antibody, which can be conveniently produced, for example, using phage display-based affinity maturation techniques such as those described herein. Briefly, one or more CDR residues are mutated and the variant antibodies are displayed on phages and screened for a particular biological activity (e.g. binding affinity). Changes (e.g., substitutions) can be made in CDRs, for example, to improve antibody affinity. Such changes can be made in CDR "hotspots", i.e., residues encoded by codons that undergo mutation at high frequency during the somatic maturation process, and/or antigen-contacting residues, and meanwhile, the resulting variant VH or VL is tested for binding affinity. In some embodiments of affinity maturation, diversity is introduced into the variable genes selected for maturation by any one of a variety of methods (e.g., error-prone PCR, chain shuffling, or oligonucleotide-directed mutagenesis). A secondary library is then created. The library is then screened to identify any antibody variants with the desired affinity. Another method for introducing diversity involves CDR-directed methods in which several CDR residues (e.g., 4-6 residues at a time) are randomized. CDR residues involved in antigen binding can be specifically identified, for example, using alanine scanning mutagenesis or modeling.

In certain embodiments, substitutions, insertions or deletions may occur within one or more CDRs so long as such changes do not substantially reduce the ability of the antibody to bind to the antigen. For example, conservative changes (e.g., conservative substitutions as provided herein) that do not substantially reduce binding affinity may be made in CDRs. In certain embodiments of the variant VH and VL sequences provided above, each CDR is unchanged or contains no more than 1, 2, or 3 amino acid substitutions.

A useful method for identifying residues or regions of an antibody that can be used as mutagenesis targets is called "alanine scanning mutagenesis". In this method, a residue or group of residues (e.g., charged residues such as Arg, Asp, His, Lys and Glu) are identified and replaced with a neutral or negatively charged amino acid (e.g., Ala or polyalanine) to determine whether the interaction of the antibody with the antigen is affected. Further substitutions may be introduced at amino acid locations that show functional sensitivity to the initial substitutions. In addition, a crystal structure of an antigen-antibody complex may be studied to identify contact points between the antibody and antigen. These contact residues and neighboring residues may be targeted or eliminated as substitution candidates. Variants may be screened to determine whether they contain the desired properties.

Amino acid sequence insertions include: amino- and/or carboxy-terminal fusions to 1 residue or polypeptides of 100 or more residues in length, and intrasequence insertions of single or multiple amino acid residues. Examples of terminal insertions include an antibody with an N-terminal methionyl residue. Other insertional variants of the antibody molecule include the fusion of the N- or C-terminus of the antibody fused to an enzyme (or a polypeptide that increases the serum half-life of the antibody).

### Engineering of Fab

In one aspect, in the antigen-binding molecule of the present disclosure, one of the antigen-binding moiety that specifically binds to PSMA and the antigen-binding moiety that specifically binds to CD3 is a substituted Fab comprising a heavy chain variable region, a light chain variable region, a Titin chain, and an Obscurin chain. In the substituted Fab, the original CH1 and CL of the Fab are substituted with the Titin chain and the Obscurin chain. Illustratively, the sequences of the Titin chain and the Obscurin chain are shown in Tables 3-1 and 3-2.

**Table 3-1. The amino acid sequences of Titin chains**

| No. | SEQ ID NO | Amino acid sequence |
|---|---|---|
| T.0 | 210 | GIPPKIEALPSDISIDEGKVLTVACAFTGEPTPEVTWSCG GRKIHSQEQGRFHIENTDDLTTLIIMDVQKQDGGLYTLS LGNEFGSDSATVNIHIRSI |
| T.1 | 211 | GIPPKIECLPSDISIDEGKVLTVASAFTGEPTPEVTWSTG GRKIHSQEQGRFHIENTDDLTTLIIMDVQKQDGGLYTLS LGNEFGSDSATVNIHIRSI |
| T.2 | 212 | GIPPKIEALPSDISIDEGKCLTVASAFTGEPTPEVTWSTG GRKIHSQEQGRFHIENTDDLTTLIIMDVQKQDGGLYTLS LGNEFGSDSATVNIHIRSI |
| T.3 | 213 | GIPPKIEALPSDISIDEGKVLTVASCFTGEPTPEVTWSTG GRKIHSQEQGRFHIENTDDLTTLIIMDVQKQDGGLYTLS LGNEFGSDSATVNIHIRSI |
| T.4 | 214 | GIPPKIEALPSDISIDEGKVLCVASAFTGEPTPEVTWSTG GRKIHSQEQGRFHIENTDDLTTLIIMDVQKQDGGLYTLS LGNEFGSDSATVNIHIRSI |
| T.5 | 215 | KAGIRGIPPKIECLPSDISIDEGKVLTVASAFTGEPTPEVT WSTGGRKIHSQEQGRFHIENTDDLTTLIIMDVQKQDGG LYTLSLGNEFGSDSATVNIHIRSI |
| T.6 | 216 | KAGIRGIPPKIEALPSDISIDEGKVLTVACAFTGEPTPEVT WSCGGRKIHSQEQGRFHIENTDDLTTLIIMDVQKQDGG LYTLSLGNEFGSDSATVNIHIRSI |
| T.7 | 217 | GIPPKIECLPSDISIDEGKVLTVASAFTGEPTPEVTWSTG GRKIHSQEQGRFHIENTDDLTTLIISDVQKQDGGLYSLS LGNEFGSDSATVNIHIRSI |
| T.8 | 218 | GIPPKIECLPSDISIDEGKVLTVASAFTGEPTPEVTWSTG GRKIHSQEQGRFHIENTDDLTTLIIKDVQRQDGGLYTLT LRNEFGSDSATVNIHIRSI |
| T.9 | 219 | GIWPKIECLPIDLSIDEGKVLMVASAFTGEPTPEVTWST GGRKIHSQEQGRFHIENTDDLTTLIIMDVQKQDGGLYT LSLGMEFGSDSATVNIHIRSI |
| T.10 | 220 | GIPPKIECLPIDISIDEGKVLTVASAFTGEPTPEVTWSTGG RKIHSQEQGRFHIENTDDLTTLIIKDVQKQDGGLYTLTL RNEFGSDSATVNIHIRSI |
| T.11 | 221 | GIPPKIECLPSDISIDEGKVLTVASAFTGEPTPEVTWSTS GKKISSEEGGRFHIESTEDLTTLIIMDVQKQDGGVYTLS LGNDLGSDSATVNIHIRSI |
| T.12 | 222 | GIPPKIECLPSDISIDEGKVLTVASAFTGEPTPEVTWSTG GRKIHSEEGGRFHIESTEDLTTLIIMDVQKQDGGVYTLS LGNEFGSDSATVNIHIRSI |
| T.13 | 223 | GIPPKIECLPSDISIDEGKVLTVASAFTGEPTPEVTWSTG GRKIHSQEQGRFHIESTEDLTTLIIMDVQKQDGGVYTLS LGNEFGSDSATVNIHIRSI |
| T.14 | 224 | GIPPKIECLPSDISIDEGKVLTVASAFTGEPTPEVTWSTG GRKIHSQEQGRFHIESTEDLTTLIISDVQKQDGGLYSLSL GNEFGSDSATVNIHIRSI |
| T.15 | 225 | KAGIRGIPPKIECLPSDISIDEGKVLTVASAFTGEPTPEVT WSTGGRKIHSQEQGRFHIESTEDLTTLIISDVQKQDGGL YSLSLGNEFGSDSATVNIHIRSI |
| T.16 | 226 | GIPPKIECLPIDISIDEGKVLTVASAFTGEPTPEVTWSTGG RKIHSQEQGRFHIENTDDSTTLTIKDVQKQDGGLYTLTL RNEFGSDSATVNIHIRSI |
| T.17 | 227 | GIPPKIECLPIDISIDEGKCLTVASAFTGEPTPEVTWSTGG RKIHSQEQGRFHIENTDDSTTLTIKDVQKQDGGLYTLTL RNEFGSDSATVNIHIRSI |
| T.18 | 228 | GIPPKIECLPIDISIDEGKVLTVASCFTGEPTPEVTWSTGG RKIHSQEQGRFHIENTDDSTTLTIKDVQKQDGGLYTLTL RNEFGSDSATVNIHIRSI |

**Table 3-2. The amino acid sequences of Obscurin chains**

| No. | SEQ ID NO | Amino acid sequence |
|---|---|---|
| O.0 | 229 | SGAPRFLTRPKAFVVSVGKDATLSCQIVGNPTPQVSWE KDQQPVAAGARFRLAQDGDLYRLTILDLALGDSGQYV CRARNAIGEAFAAVGLQVDAEA |
| OL.0 | 230 | QGSPPCFLRFPRPVRVVSGAEAELKCVVLGEPPPVVVW EKGGQQLAASERLSFPADGAEHGLLLTAALPTDAGVY VCRARNAAGEAYAAAAVTVLEPP |
| O.1 | 231 | SGAPRFLTRPKAFVVSVGKDATLSCQIVGNPTPQVSWE KDQQPVAAGARFRLAQDGDLYRLTILDLALGDSGQYV CRARNAIGEAFACVGLQVDAEA |
| O.2 | 232 | SGCPRFLTRPKAFVVSVGKDATLSCQIVGNPTPQVSWE KDQQPVAAGARFRLAQDGDLYRLTILDLALGDSGQYV CRARNAIGEAFAAVGLQVDAEA |
| O.3 | 233 | SGAPRFLTCPKAFVVSVGKDATLSCQIVGNPTPQVSWE KDQQPVAAGARFRLAQDGDLYRLTILDLALGDSGQYV CRARNAIGEAFAAVGLQVDAEA |
| O.4 | 234 | SGAPRFLTRPKAFVVSVGKDATLSSQIVGNPTPQVSWE KDQQPVAAGARFRLAQDGDLYRLTILDLALGDSGQYV SRARNAIGEAFACVGLQVDAEA |
| O.5 | 235 | SGCPRFLTRPKAFVVSVGKDATLSSQIVGNPTPQVSWE KDQQPVAAGARFRLAQDGDLYRLTILDLALGDSGQYV SRARNAIGEAFAAVGLQVDAEA |
| O.6 | 236 | SGAPRFLTCPKAFVVSVGKDATLSSQIVGNPTPQVSWE KDQQPVAAGARFRLAQDGDLYRLTILDLALGDSGQYV SRARNAIGEAFAAVGLQVDAEA |
| O.7 | 237 | SGAPRFKTRPKAFVVSVGKDATLSSQIVGNPTPQVSWE KDQQPVAAGARFRLAQDGDLYRLKILDLALGDSGQYV SRARNAIGEAFACVGLQVDAEA |
| O.8 | 238 | SGAPRFKTRPKAFVVSVGKDATLSSQIVGNPTPQVSWE KDQQPVAAGARFRLAQDGDLYRLHILDLALGDSGQYV SRARNAIGEAFACVGLQVDAEA |
| O.9 | 239 | SGAPRFLTRPLAFVVSVGKDATLSSQIVGNPTPQVSWE KDQQPVAAGARFRLAQDGDLYRLKILDLALGDSGQYV SRARNAIGEAFACVGLQVDAEA |
| O.10 | 240 | SGAPRFLTRPLAFVVSVGKDATLSSQIVGNPTPQVSWE KDQQPVAAGARFRLAQDGDLYRLHILDLALGDSGQYV SRARNAIGEAFACVGLQVDAEA |
| O.11 | 241 | DQPQFSGAPRFLTRPKAFVVSVGKDATLSSQIVGNPTPQ VSWEKDQQPVAAGARFRLAQDGDLYRLTILDLALGDS GQYVSRARNAIGEAFACVGLQVDAEA |
| O.12 | 242 | DQPQFSGAPRFKTRPKAFVVSVGKDATLSSQIVGNPTP QVSWEKDQQPVAAGARFRLAQDGDLYRLKILDLALGD SGQYVSRARNAIGEAFACVGLQVDAEA |
| O.13 | 243 | DQPQFSGAPRFKTRPKAFVVSVGKDATLSSQIVGNPTP QVSWEKDQQPVAAGARFRLAQDGDLYRLHILDLALGD SGQYVSRARNAIGEAFACVGLQVDAEA |
| O.14 | 244 | DQPQFSGAPRFRTRPKAFVVSVGKDATLSSQIVGNPTP QVSWEKDQQPVAAGARFRLAQDGDLYRLKILDLALGD SGQYVSRARNAIGEAFACVGLQVDAEA |
| O.15 | 245 | DQPQFSGAPRFRTRPKAFVVSVGKDATLSSQIVGNPTP QVSWEKDQQPVAAGARFRLAQDGDLYRLHILDLALGD SGQYVSRARNAIGEAFACVGLQVDAEA |
| O.16 | 246 | DQPQFSGAPRFLTRPKAFVVSVGKDATLSCQIVGNPTP QVSWEKDQQPVAAGARFRLAQDGDLYRLTILDLALGD SGQYVCRARNAIGEAFAAVGLQVDAEA |
| OL.1 | 247 | QGSPPEFLRFPRPVRVVSGAEAELKCVVLGEPPPVVVW EKGGQQLAASERLSFPADGAEHGLLLTAALPTDAGCY VCRARNAAGEAYAAAAVTVLEPP |
| OL.2 | 248 | QGSPPEFLRFPRPVRVVSGAEAELKCVVLGEPPPVVVW EKGGQQLAASERLSFPADGAEHGLLLTAALPTDAGVY VCRARNAACEAYAAAAVTVLEPP |
| OL.3 | 249 | QGSPPEFLRFPRPVRVVSGAEAELKCVVLGEPPPVVVW EKGGQQLAASERLSFPADGAEHGLLLTAALPTDAGVY VCRARNAAGECYAAAAVTVLEPP |
| OL.4 | 250 | QGSPPEFLRFPRPVRVVSGAEAELKSVVLGEPPPVVVW EKGGQQLAASERLSFPADGAEHGLLLTAALPTDAGCY VSRARNAAGEAYAAAAVTVLEPP |
| OL.5 | 251 | QGSPPEFLRFPRPVRVVSGAEAELKSVVLGEPPPVVVW EKGGQQLAASERLSFPADGAEHGLLLTAALPTDAGVY VSRARNAACEAYAAAAVTVLEPP |
| OL.6 | 252 | QGSPPEFLRFPRPVRVVSGAEAELKSVVLGEPPPVVVW EKGGQQLAASERLSFPADGAEHGLLLTAALPTDAGVY VSRARNAAGECYAAAAVTVLEPP |
| O.17 | 253 | SGAPRFLTRPKSYTVSVGKDASLSSQIVGNPTPQVSWE KDQQPVAAGARFRLAQDGDLYRLKILDLALGDSGQYV SRARNAIGEAFACVGLQVDAEA |
| O.18 | 254 | SEAPRFLTRPLAFVVSEGKDATLSSQIVGDPPPEVTWEK DQQPITAGARFRLAQDGDVYRLEILDLQLSDSGQYVSR ARNAIGEAFACVGLQVDAEG |
| O.19 | 255 | SGAPRFLTRPLAFVVSVGKLAMLSSQIVGNPTPQVSWE KDQQPVAAGARFRLLQDGDLYRLKILDLALGDSGQYV SRARNAIGEAFACVGLQVDAEA |
| O.20 | 256 | SGAPRFLTRPLAFVVSVGKDATLSSQIVGNPTPQVSWE KDKQPVTAGARFRLAQDGDLYRLKILDLQLSDSGQYV SRARNAIGEAFACLGLQVDAEA |
| O.21 | 257 | SGAPRFLTRPLAFVVSVGKDATLSSQIVGNPTPQVSWE KDQLPVTAGARFRLAQDGDLYRLKILDLTLSDSGQYVS RARNAIGEAFACVGLEVGAEA |
| O.22 | 258 | SGAPRFLTRPLSYVVSVGKDASLSSQIVGNPTPQVSWE KDQLPVTAGARFRLAQDGDLYRLKILDLQLSDSGQYV SRARNAIGEAFACVGLEVGAEA |
| O.23 | 259 | DQPQFSGAPRFLTRPLSYVVSVGKDASLSSQIVGNPTPQ VSWEKDQLPVTAGARFRLAQDGDLYRLKILDLQLSDS GQYVSRARNAIGEAFACVGLEVGAEA |
| O.24 | 260 | SGAPRFLTRPKAFVVSVGKDATLSSQIVGNPFPQVSWE KDKQPVTAGVRFRLAQDGDLYRLKILDLQLSDSGQYV SRARNAIGEAFACLGLQVDAEA |
| O.25 | 261 | SGAPRFLTRPKASVVSVGKDATLSSQIVGNPFPQVSWE KDKQPVTAGVRFRLAQDGDLYRLKILDLQLSDSGQYV SRARNAHGEAFACLGLQVDAEA |
| O.26 | 262 | SGCPRFLTRPKASVVSVGKDATLSSQIVGNPFPQVSWE KDKQPVTAGVRFRLAQDGDLYRLKILDLQLSDSGQYV SRARNAHGEAFACLGLQVDAEA |
| O.27 | 263 | SGAPRFLTCPKASVVSVGKDATLSSQIVGNPFPQVSWE KDKQPVTAGVRFRLAQDGDLYRLKILDLQLSDSGQYV SRARNAHGEAFACLGLQVDAEA |
| O.28 | 264 | SGAPRFLTRPKASVVSVGKDATLSCQIVGNPFPQVSWE KDKQPVTAGVRFRLAQDGDLYRLKILDLQLSDSGQYV CRARNAHGEAFACLGLQVDAEA |
| O.29 | 265 | SGCPRFLTRPKASVVSVGKDATLSCQIVGNPFPQVSWE KDKQPVTAGVRFRLAQDGDLYRLKILDLQLSDSGQYV CRARNAHGEAFACLGLQVDAEA |
| O.30 | 266 | SGAPRFLTCPKASVVSVGKDATLSCQIVGNPFPQVSWE KDKQPVTAGVRFRLAQDGDLYRLKILDLQLSDSGQYV CRARNAHGEAFACLGLQVDAEA |
| O.31 | 267 | SGAPRFLTRPKASVVSVGKDATLSSQIVGNPFPQVSWE KDKQPVTAGVRFRLAQDGDLYRLKILDCQLSDSGQYV SRARNAHGEAFACLGLQCDAEA |
| O.32 | 268 | SGCPRFLTRPKASVVSVGKDATLSSQIVGNPFPQVSWE KDKQPVTAGVRFRLAQDGDLYRLKILDCQLSDSGQYV SRARNAHGEAFACLGLQCDAEA |
| O.33 | 269 | SGAPRFLTCPKASVVSVGKDATLSSQIVGNPFPQVSWE KDKQPVTAGVRFRLAQDGDLYRLKILDCQLSDSGQYV SRARNAHGEAFACLGLQCDAEA |

### Engineering of Fc region

In one aspect, the Fc region of the antigen-binding molecule of the present disclosure comprises one or more amino acid substitutions that reduce its binding to Fc receptors, e.g., its binding to Fcy receptors, and reduce or eliminate effector functions. A natural IgG Fc region, specifically an IgG₁ Fc region or an IgG₄ Fc region, may cause the antigen-binding molecule of the present disclosure to target cells expressing an Fc receptor, rather than cells expressing an antigen. The engineered Fc region of the present disclosure exhibits reduced binding affinity for an Fc receptor and/or reduced effector functions. In some embodiments, the engineered Fc region shows no less than 50%, 80%, 90%, or 95% reduction in binding affinity for an Fc receptor as compared to the native Fc region. In some embodiments, the Fc receptor is an Fcy receptor. In some embodiments, the Fc receptor is a human Fcγ receptor, e.g., FcyRI, FcyRIIa, FcyRIIB, or FcyRIIIa. In some embodiments, the engineered Fc region has reduced binding affinity for a complement (e.g., C1q) as compared to the native Fc region. In some embodiments, the engineered Fc region has no reduced binding affinity for a neonatal Fc receptor (FcRn) as compared to the native Fc region. In some embodiments, the engineered Fc region has reduced effector functions, which may include, but are not limited to, one or more of the following: reduced complement-dependent cytotoxicity (CDC), reduced antibody-dependent cell-mediated cytotoxicity (ADCC), reduced antibody-dependent cellular phagocytosis (ADCP), reduced cytokine secretion, reduced immune complex-mediated antigen uptake by antigen presenting cells, reduced binding to NK cells, reduced binding to macrophages, reduced binding to monocytes, reduced binding to polymorphonuclear cells, reduced direct signaling-induced apoptosis, reduced dendritic cell maturation, and reduced T cell priming.

For the IgG₁ Fc region, amino acid residue substitutions at positions such as positions 238, 265, 269, 270, 297, 327, and 329 can reduce effector functions. In some embodiments, the Fc region is a human IgG₁ Fc region, and the amino acid residues at positions 234 and 235 are A, as numbered according to the EU index. For the IgG₄ Fc region, amino acid residue substitutions at positions such as position 228 can reduce effector functions.

The antigen-binding molecule may also comprise disulfide bond modifications, such as 354C for the first subunit and 349C for the second subunit. To increase the serum half-life of the antigen-binding molecule, 252Y, 254T, and 256E mutations may be introduced.

When the antigen-binding molecule comprises different binding moieties fused to the two subunits of the Fc region, it may result in undesired homodimerization. To improve yield and purity, it is thus advantageous to introduce modifications that promote heterodimerization in the Fc region of the antigen-binding molecule of the present disclosure. In some embodiments, the Fc region of the present disclosure comprises engineering according to the knob-into-hole (KIH) technique, which involves introducing a knob structure at the interface of the first subunit and a hole structure at the interface of the second subunit. As such, the knob structure can be positioned in the hole structure, thereby promoting the formation of heterodimers and inhibiting the production of homodimers. The knob structure is constructed by substituting a small amino acid side chain at the interface of the first subunit with a larger side chain (e.g., tyrosine or tryptophan). The hole structure is created at the interface of the second subunit by substituting a large amino acid side chain with a smaller amino acid side chain (e.g., alanine or threonine). The knob and hole structures are prepared by altering the nucleic acid encoding the polypeptide, with optional amino acid substitutions shown in the table below:

**Table 4. Combinations of KIH mutations**

| First subunit | T366Y | T366W | T394W | F405W | T366W | T366Y | T366W | F405W |
|---|---|---|---|---|---|---|---|---|
| | | | | | | F405A | F405W | Y407A |
| Second subunit | Y407T | Y407A | F405A | T394S | T366S | T394W | T394W | T366W |
| | | | | | L368A | | | |
| | | | | | Y407V | Y407T | Y407A | T394S |

In addition to the knob-into-hole technique, other techniques for modifying the CH3 domain of the heavy chain to achieve heterodimerization are also known in the art, e.g., WO96/27011, WO98/050431, EP1870459, WO2007/110205, WO 007/147901, WO2009/089004, WO2010/129304, WO2011/90754, WO2011/143545, WO2012/058768, WO2013/157954, and WO 013/096291.

The C-terminus of the Fc region may be an intact C-terminus ending with amino acid residues PGK or a truncated C-terminus, e.g., a truncated C-terminus in which one or two C-terminal amino acid residues are removed. In a preferred aspect, the C-terminus of the heavy chain is a shortened C-terminus ending with PG. Thus, in some embodiments, a composition of intact antibodies may comprise antibody populations with all K447 residues and/or G446 + K447 residues removed. In some embodiments, a composition of intact antibodies may comprise antibody populations without K447 residue and/or G446 + K447 residue removed. In some embodiments, a composition of intact antibodies comprises antibody populations having a mixture of antibodies with and without K447 residues and/or G446 + K447 residues.

### Recombination Method

The antigen-binding molecule may be produced using recombination methods. For these methods, one or more isolated nucleic acids encoding the antigen-binding molecule are provided.

In the case of a native antibody, a native antibody fragment or a bispecific antibody with homodimeric heavy chains, two nucleic acids are required, one for the light chain or a fragment thereof and the other for the heavy chain or a fragment thereof. Such nucleic acids encode an amino acid sequence comprising the VL of the antibody and/or an amino acid sequence comprising the VH of the antibody (e.g., the light and/or heavy chain(s) of the antibody). These nucleic acids can be on the same expression vector or different expression vectors.

In the case of a bispecific antibody with heterodimeric heavy chains, four nucleic acids, for example, are required, one for the first light chain, one for the first heavy chain comprising the first heteromonomeric Fc-region polypeptide, one for the second light chain, and one for the second heavy chain comprising the second heteromonomeric Fc-region polypeptide. The four nucleic acids can be contained in one or more nucleic acid molecules or expression vectors. Generally, these nucleic acids are located on two or three expression vectors, that is, one vector can comprise more than one of these nucleic acids.

In one embodiment, the present disclosure provides an isolated nucleic acid encoding the aforementioned antibody. Such nucleic acids may independently encode any one of the polypeptide chains described above. In another aspect, the present disclosure provides one or more vectors (e.g., expression vectors) comprising such nucleic acids. In another aspect, the present disclosure provides a host cell comprising such nucleic acids. In one embodiment, provided is a method for preparing an antigen-binding molecule, wherein the method comprises culturing a host cell comprising a nucleic acid encoding the antibody, as provided above, under conditions suitable for antibody expression, and optionally collecting the antibody from the host cell (or host cell medium).

For recombinant production of the antigen-binding molecule, the nucleic acid encoding the protein is isolated and inserted into one or more vectors for further cloning and/or expression in a host cell. Such nucleic acids can be readily isolated and sequenced using conventional procedures (e.g., by using oligonucleotide probes that are capable of binding specifically to genes encoding the heavy and light chains of an antibody), or produced by recombinant methods or obtained by chemical synthesis. Suitable host cells for cloning or expressing a vector encoding the antibody include prokaryotic or eukaryotic cells described herein. For example, the antibody can be produced in bacteria, particularly when the antibody does not require glycosylation and Fc effector functions. After expression, the antibody can be isolated from the bacterial cell paste in a soluble fraction and can be further purified.

In addition to prokaryotes, eukaryotic microorganisms such as filamentous fungi or yeast are suitable cloning or expression hosts for antibody-encoding vectors, including fungal and yeast strains, whose glycosylation pathways have been "humanized", such that an antibody with a partially or fully human glycosylation pattern is produced. Suitable host cells for expression of (glycosylated) antibodies may also be derived from multicellular organisms (invertebrates and vertebrates); examples of invertebrate cells include plant and insect cells. A number of baculovirus strains have been identified, which can be used in combination with insect cells, particularly for transfection of *Spodoptera frugiperda* cells; plant cell cultures may also be used as hosts, see, e.g., US5959177, US 6040498, US6420548, US 7125978 and US6417429; vertebrate cells can also be used as hosts, e.g., mammalian cell lines adapted for growth in a suspension. Other examples of suitable mammalian host cell lines include a SV40-transformed monkey kidney CVl line (COS-7); a human embryonic kidney line (293 or 293T cells); baby hamster kidney cells (BHK); mouse sertoli cells (TM4 cells); monkey kidney cells (CV1); African green monkey kidney cells (VERO-76); human cervical cancer cells (HELA); canine kidney cells (MDCK); buffalo rat liver cells (BRL3A); human lung cells (W138); human liver cells (Hep G2); mouse mammary tumor cells (MMT 060562); TRI cells; MRC5 cells; and FS4 cells. Other suitable mammalian host cell lines include Chinese hamster ovary (CHO) cells, including DHFR-CHO cells; and myeloma cell lines such as Y0, NS0, and Sp2/0. For reviews of certain mammalian host cell lines suitable for the production of the antibody, see, e.g., Yazaki, P. and Wu, A.M., Methods in Molecular Biology, Vol. 248, Lo, B.K.C. (eds.), Humana Press, Totowa, NJ (2004), pp. 255-268.

### Diagnostic and Therapeutic Compositions

In certain embodiments, the antigen-binding molecule provided in the present disclosure can be used to detect the presence of PSMA and/or CD3 in a biological sample. As used herein, the term "detection" encompasses quantitative or qualitative detection. In certain embodiments, the biological sample includes a cell or tissue, such as tumor tissue.

In one embodiment, an antigen-binding molecule for use in a diagnostic or detection method is provided. In yet another aspect, a method for detecting the presence of PSMA and/or CD3 in a biological sample is provided. In certain embodiments, the method comprises contacting the biological sample with an antigen-binding molecule under suitable conditions and detecting whether a complex is formed between the detection agent and the antigen. Such methods may be *in vitro* or *in vivo* methods. In one embodiment, an antigen-binding molecule is used to select a subject suitable for treatment; for example, PSMA and/or CD3 are biomarkers for selecting a patient. Exemplary disorders that can be diagnosed using the antigen-binding molecule of the present disclosure are, for example, tumors or cancers.

In certain embodiments, a labeled antigen-binding molecule is provided. Labels include, but are not limited to, labels or moieties that are detected directly (such as fluorescent, chromophoric, electron-dense, chemiluminescent, and radioactive labels), and moieties that are detected indirectly (e.g., moieties that are detected indirectly through an enzymatic reaction or molecular interaction, such as enzymes or ligands). In an additional aspect, a pharmaceutical composition comprising the antigen-binding molecule is provided, for example, for use in any of the following treatment methods. In one aspect, the pharmaceutical composition comprises any of the antigen-binding molecules provided herein and a pharmaceutically acceptable carrier. In another aspect, the pharmaceutical composition comprises any of the antigen-binding molecules provided herein and at least one additional therapeutic agent.

The pharmaceutical composition of the antigen-binding molecule described in the present disclosure is prepared by: mixing such an antigen-binding molecule having desired purity with one or more optional pharmaceutically acceptable carriers. The pharmaceutical composition is in the form of a lyophilized composition or an aqueous solution. Formulations for *in vivo* administration are generally sterile. Sterility can be readily achieved, for example, by filtration through a sterile filter membrane.

### Treatment Method and Route of Administration

Any of the antigen-binding molecules provided herein can be used in the treatment method.

In yet another aspect, the present disclosure provides use of the antigen-binding molecule in the manufacture or preparation of a medicament. In one embodiment, the medicament is for treating a tumor or cancer. And the medicament is present in an amount effective for the diseases described above. In some embodiments, the effective amount is a unit daily dose or a unit weekly dose. In one such embodiment, the use further comprises administering to a subject an effective amount of at least one additional therapeutic agent (e.g., one, two, three, four, five, or six additional therapeutic agents). The "subject" according to any of the above embodiments may be a human.

In yet another aspect, provided is a pharmaceutical composition comprising the antigen-binding molecule, e.g., for any of the above pharmaceutical uses or treatment methods. In another embodiment, the pharmaceutical composition also comprises at least one additional therapeutic agent.

The antigen-binding molecule of the present disclosure may be used alone or in combination with other agents for the treatment. For example, the antigen-binding molecule of the present disclosure may be co-administered with at least one additional therapeutic agent.

The antigen-binding molecule of the present disclosure (and any additional therapeutic agents) may be administered by any suitable means, including parenteral, intrapulmonary, and intranasal administration, and if required for local treatment, intralesional administration. Parenteral infusion includes intramuscular, intravenous, intra-arterial, intraperitoneal, or subcutaneous administration. Administration may be performed by any suitable route, e.g., by injection, such as intravenous or subcutaneous injection, depending in part on whether the administration is short-term or long-term. Various administration schedules are contemplated herein, including but not limited to, single administration or multiple administrations at multiple time points, bolus administration, and pulse infusion.

The antigen-binding molecule of the present disclosure will be formulated, administered, and applied in a manner consistent with GOOD MEDICAL PRACTICE. Factors considered in this context include the specific disorder being treated, the specific mammal being treated, the clinical state of the individual patient, the cause of the disorder, the site of delivery of the agent, the method of administration, the timing of administration, and other factors known to medical practitioners. The antigen-binding molecule is optionally formulated along with one or more agents currently used for preventing or treating the disorder. The effective amount of such additional agents depends on the amount of the antigen-binding molecule present in the pharmaceutical composition, the type of the disorder or treatment, and other factors discussed above. These are generally used in the same dosages and with routes of administration as described herein, or in about 1 to 99% of the dosages described herein, or in any dosage and by any route that is empirically/clinically determined to be appropriate.

For the prevention or treatment of a disease, the appropriate dosage of the antigen-binding molecule of the present disclosure (when used alone or in combination with one or more additional therapeutic agents) will depend on the type of disease to be treated, the type of therapeutic molecule, the severity and course of the disease, the purpose of the administration (prophylactic or therapeutic), previous treatment, clinical history and response to the therapeutic molecule of the patient, and the discretion of the attending physician. The therapeutic molecule is suitably administered to a patient in one or a series of treatments. Depending on the type and severity of the disease, about 1 µg/kg to 15 mg/kg of the antigen-binding molecule can be an initial candidate dosage for administration to the patient, whether, for example, by one or more separate administrations, or by continuous infusion. One typical daily dosage may range from about 1 µg/kg to 100 mg/kg or more, depending on the factors mentioned above. Accordingly, taking 50 kg body weight as an example, an exemplary unit daily dosage is 50 µg-5 g.

### Product

In another aspect of the present disclosure, provided is a product, which comprises materials useful for the treatment, prevention, and/or diagnosis of the disorders described above. The product comprises a container and a label or package insert on or associated with the container. Suitable containers include, for example, bottles, vials, syringes, IV solution bags, and the like. The container may be formed from a variety of materials such as glass or plastic. The container contains a composition effective in the treatment, prevention, and/or diagnosis of a disease, either alone or in combination with another composition, and may have a sterile access port (e.g., the container may be an intravenous solution bag or vial with a stopper pierceable by a hypodermic injection needle). At least one active agent in the composition is the antigen-binding molecule of the present disclosure. The label or package insert indicates that the composition is used to treat the selected condition. Further, the product may comprise: (a) a first container containing a composition, wherein the composition comprises the antigen-binding molecule of the present disclosure; and (b) a second container containing a composition, wherein the composition comprises other cytotoxic agents or additional therapeutic agents. The product in this embodiment of the present disclosure may further comprise a package insert indicating that the composition may be used to treat a particular condition. Alternatively or additionally, the product may further comprise a second (or third) container containing a pharmaceutically acceptable buffer. From a commercial and user standpoint, it may further contain other materials as required, including other buffers, diluents, filters, needles, and syringes.

As an example, the product is prepared in the form of a kit.

### Examples and Test Examples

The present disclosure is further described below with reference to the following examples and test examples, which, however, do not limit the present disclosure. The experimental methods in the examples and test examples of the present disclosure in which specific conditions are not specified are generally performed under conventional conditions such as Antibodies: A Laboratory Manual and Molecular Cloning: A Laboratory Manual by Cold Spring Harbor Laboratory, or under conditions recommended by the manufacturers of the starting materials or commercial products. Reagents without specific origins indicated are commercially available conventional agents.

### Example 1. Antigen-Binding Molecules Comprising Titin Chain/Obscurin Chain

The Titin chain/Obscurin chain of the present disclosure may be derived from any suitable polypeptide, including polypeptides derived from WO2021139758A1 (incorporated herein by reference in its entirety) and CN202110527339.7 and the patents which refer to it as a priority document (incorporated herein by reference in their entirety). Bispecific antibodies were constructed in which the CL was the kappa light chain constant region in WO2021139758A1. The amino acid sequences of the Titin chain and the Obscurin chain are shown in Tables 3-1 and 3-2; linker sequences include GGGGS (SEQ ID NO: 173), ASTKG (SEQ ID NO: 270), or RTVAS (SEQ ID NO: 271), and the amino acid sequences of the Fc1, Fc2, and CH1 in this example are shown below.
> Fc1 (knob, SEQ ID NO: 272) 
DKTHTCPPCPAPEAAGGPSVFLFPPKPKDTLMISRTPEVTCVVVDVSHEDPEV
KFNWYVDGVEVHNAKTKPREEQYNSTYRVVSVLTVLHQDWLNGKEYKCKV
SNKALPAPIEKTISKAKGQPREPQVYTLPPCREEMTKNQVSLWCLVKGFYPSDI
AVEWESNGQPENNYKTTPPVLDSDGSFFLYSKLTVDKSRWQQGNVFSCSVMH
EALHNHYTQKSLSLSPGK;
> Fc2 (hole, SEQ ID NO: 273) 
DKTHTCPPCPAPEAAGGPSVFLFPPKPKDTLMISRTPEVTCVVVDVSHEDPEV
KFNWYVDGVEVHNAKTKPREEQYNSTYRVVSVLTVLHQDWLNGKEYKCKV
SNKALPAPIEKTISKAKGQPREPQVCTLPPSREEMTKNQVSLSCAVKGFYPSDI
AVEWESNGQPENNYKTTPPVLDSDGSFFLVSKLTVDKSRWQQGNVFSCSVMH
EALHNHYTQKSLSLSPGK;
> CH1 (SEQ ID NO: 168).

### 1.1 DI bispecific antibodies

Construction of DI bispecific antibodies against hNGF and hRANKL: DI-2 to DI-20 comprising a first heavy chain, a second heavy chain, a first light chain, and a second light chain as described below with reference to Example 5 in WO2021139758A1:
the first heavy chain is, from N-terminus to C-terminus, [VH1-I]-[linker 1]-[Obscurin chain]-[Fc2],
the first light chain is, from N-terminus to C-terminus, [VL1-I]-[linker 2]-[Titin chain],
the second heavy chain is, from N-terminus to C-terminus, [VH2-D]-[CH1]-[Fc1], and
the second light chain is, from N-terminus to C-terminus, [VL2-D]-[CL];
wherein the VH1-I and VL1-I were the heavy chain variable region and the light chain variable region of I0 in WO2021139758A1, respectively, and the VH2-D and VL2-D were the heavy chain variable region and the light chain variable region of D0 in WO2021139758A1, respectively. The structures of the Obscurin chain, the Titin chain, the linker 1, and the linker 2 in the DI bispecific antibodies of this example are shown in the table below.

**Table 5. Obscurin chain/Titin chain and linkers in DI bispecific antibodies**

| No. | First heavy chain | | First light chain | |
|---|---|---|---|---|
| | Obscurin chain | Linker 1 | Titin chain | Linker 2 |
| DI-2 | O.20 | GGGGS | T.10 | GGGGS |
| DI-3 | O.25 | GGGGS | T.16 | GGGGS |
| DI-4 | O.26 | GGGGS | T.17 | GGGGS |
| DI-5 | O.27 | GGGGS | T.18 | GGGGS |
| DI-6 | O.28 | GGGGS | T.16 | GGGGS |
| DI-7 | O.29 | GGGGS | T.17 | GGGGS |
| DI-8 | O.30 | GGGGS | T.18 | GGGGS |
| DI-9 | O.31 | GGGGS | T.16 | GGGGS |
| DI-10 | O.32 | GGGGS | T.17 | GGGGS |
| DI-11 | O.33 | GGGGS | T.18 | GGGGS |
| DI-12 | O.25 | ASTKG | T.16 | RTVAS |
| DI-13 | O.26 | ASTKG | T.17 | RTVAS |
| DI-14 | O.27 | ASTKG | T.18 | RTVAS |
| DI-15 | O.28 | ASTKG | T.16 | RTVAS |
| DI-16 | O.29 | ASTKG | T.17 | RTVAS |
| DI-17 | O.30 | ASTKG | T.18 | RTVAS |
| DI-18 | O.31 | ASTKG | T.16 | RTVAS |
| DI-19 | O.32 | ASTKG | T.17 | RTVAS |
| DI-20 | O.33 | ASTKG | T.18 | RTVAS |

| | | | | |
|---|---|---|---|---|
| Note: The numbers of the Titin chain and the Obscurin chain in the tables are shown in Tables 3-1 and 3-2. | | | | |

The binding activity of the bispecific antibodies DI-2 to DI-20 against their antigens was assayed by the method of Test Example 4 of WO2021139758A1. The antibodies were subjected to a thermal stability study. The study method was as follows: the antibodies were each diluted to a concentration of 5 mg/mL with PBS and tested for thermal stability using a high throughput differential scanning fluorimeter (UNCHAINED, Specification: Unit). The experimental results showed that the binding activity of the modified bispecific antibodies against the antigen was not significantly changed; meanwhile, DI-4 to DI-8, DI-10 to DI-16, and DI-20 had significantly improved Tm1 (°C) and Tonset (°C) as compared to DI-2, and therefore, the bispecific antibodies had better thermal stability.

**Table 6. Binding activity assay on DI bispecific antibodies**

| No. | RANKL | NGF | No. | RANKL | NGF |
|---|---|---|---|---|---|
| | EC₅₀ (nM) | | | EC₅₀ (nM) | |
| DI-2 | 0.3832 | 6.633 | DI-12 | 0.4125 | 6.5156 |
| DI-3 | 0.3613 | 5.7730 | DI-13 | 0.4440 | 5.5420 |
| DI-4 | 0.3959 | 6.2930 | DI-14 | 0.4182 | 3.2610 |
| DI-5 | 0.3290 | 6.1890 | DI-15 | 0.2206 | 5.2800 |
| DI-6 | 0.2509 | 5.6720 | DI-16 | 0.1474 | 5.5140 |
| DI-7 | 0.2557 | 6.6430 | DI-17 | 0.2329 | 6.7270 |
| DI-8 | 0.3643 | 7.6250 | DI-18 | 0.2662 | 5.9080 |
| DI-9 | 0.2944 | 8.4950 | DI-19 | 0.1843 | 5.9280 |
| DI-10 | 0.3460 | 7.1660 | DI-20 | 0.3184 | 6.4250 |
| DI-11 | 0.3721 | 10.9600 | | | |

**Table 7. Thermal stability test results for DI bispecific antibodies**

| No. | Tm1 (°C) | Tonset (°C) | No. | Tm1 (°C) | Tonset (°C) |
|---|---|---|---|---|---|
| DI-2 | 55.6 | 48.3 | DI-11 | 57.35 | - |
| DI-4 | 60.1 | 52.493 | DI-12 | 59.9 | 51.726 |
| DI-5 | 61 | 51.967 | DI-13 | 61 | 50.988 |
| DI-6 | 60.8 | 53.012 | DI-14 | 61.2 | 52.191 |
| DI-7 | 60.34 | 52.003 | DI-15 | 60.41 | 50.558 |
| DI-8 | 60.61 | 50.425 | DI-16 | 61.5 | 50.691 |
| DI-10 | 60.2 | 52.766 | DI-20 | 60.7 | 51.859 |

Solutions of the DI bispecific antibodies were formulated with a buffer containing 10 mM acetic acid at pH 5.5 and 9% sucrose, and the solutions were incubated in an incubator at 40 °C for four weeks. After the end of the incubation, the antibodies were concentrated to the concentration at the start of the incubation, and the precipitation of the solutions was observed. The experimental results showed that there was precipitation in the solution of DI-2 bispecific antibody group, and therefore, DI-3 to DI-7 had better stability than DI-2.

**Table 8. Precipitation of solutions of DI bispecific antibodies**

| No. | Initial concentration | Concentration to be achieved at week 4 | Precipitation in solution |
|---|---|---|---|
| DI-2 | 20 mg/mL | 20 mg/mL | Precipitation occurred |
| DI-3 | 20 mg/mL | 20 mg/mL | No precipitation |
| DI-4 | 60 mg/mL | 60 mg/mL | No precipitation |
| DI-5 | 25 mg/mL | 25 mg/mL | No precipitation |
| DI-6 | 60 mg/mL | 60 mg/mL | No precipitation |
| DI-7 | 16 mg/mL | 16 mg/mL | No precipitation |

### 1.2 PL bispecific antibodies

Construction of PL bispecific antibodies against hPDL1 and hCTLA4: PL-1 to PL-19 comprising a first heavy chain, a second heavy chain, a first light chain, and a second light chain as described below:
the first heavy chain is, from N-terminus to C-terminus, [VH1-P]-[linker 1]-[Obscurin chain]-[Fc1],
the first light chain is, from N-terminus to C-terminus, [VL1-P]-[linker 2]-[Titin chain],
the second heavy chain is, from N-terminus to C-terminus, [VH2-L]-[CHl]-[Fc2], and the second light chain is, from N-terminus to C-terminus, [VL2-L]-[CL];
wherein the VH1-P and VL1-P were the heavy chain variable region and the light chain variable region of the h1831K antibody in WO2020177733A1, respectively.

The amino acid sequences of the VH2-L and VL2-L are shown below.
> VH2-L (SEQ ID NO: 274) 
QVQLVESGGGVVQPGRSLRLSCAASGFTFSSYTMHWVRQAPGKGLEWVTFIS
YDGNNKYYADSVKGRFTISRDNSKNTLYLQMNSLRAEDTAIYYCARTGWLGP
FDYWGQGTLVTVSS
> VL2-L (SEQ ID NO: 275)

EIVLTQSPGTLSLSPGERATLSCRASQSVGSSYLAWYQQKPGQAPRLLIYGAFS
RATGIPDRFSGSGSGTDFTLTISRLEPEDFAVYYCQQYGSSPWTFGQGTKVEIK

The structures of the Obscurin chain, the Titin chain, the linker 1, and the linker 2 in the PL bispecific antibodies of this example are shown in the table below.

**Table 9. Obscurin chain/Titin chain and linkers in PL bispecific antibodies**

| No. | First heavy chain | | First light chain | |
|---|---|---|---|---|
| | Obscurin chain | Linker 1 | Titin chain | Linker 2 |
| PL-1 | O.20 | GGGGS | T.10 | GGGGS |
| PL-2 | O.25 | GGGGS | T.16 | GGGGS |
| PL-3 | O.26 | GGGGS | T.17 | GGGGS |
| PL-4 | O.27 | GGGGS | T.18 | GGGGS |
| PL-5 | O.28 | GGGGS | T.16 | GGGGS |
| PL-6 | O.29 | GGGGS | T.17 | GGGGS |
| PL-7 | O.30 | GGGGS | T.18 | GGGGS |
| PL-8 | O.31 | GGGGS | T.16 | GGGGS |
| PL-9 | O.32 | GGGGS | T.17 | GGGGS |
| PL-10 | O.33 | GGGGS | T.18 | GGGGS |
| PL-11 | O.25 | ASTKG | T.16 | RTVAS |
| PL-12 | O.26 | ASTKG | T.17 | RTVAS |
| PL-13 | O.27 | ASTKG | T.18 | RTVAS |
| PL-14 | O.28 | ASTKG | T.16 | RTVAS |
| PL-15 | O.29 | ASTKG | T.17 | RTVAS |
| PL-16 | O.30 | ASTKG | T.18 | RTVAS |
| PL-17 | 0.31 | ASTKG | T.16 | RTVAS |
| PL-18 | O.32 | ASTKG | T.17 | RTVAS |
| PL-19 | O.33 | ASTKG | T.18 | RTVAS |

| | | | | |
|---|---|---|---|---|
| Note: The numbers of the Titin chain and the Obscurin chain in the tables are shown in Tables 3-1 and 3-2. | | | | |

The binding activity of PL bispecific antibodies was tested by the ELISA assay with reference to Test Example 4 of WO2021139758A1, wherein hPDL1 and hCTLA4 antigens were purchased from Sino biology. The antibodies were subjected to a thermal stability study. The method was as follows: the antibodies were each diluted to a concentration of 1.4-3 mg/mL with PBS and tested for thermal stability using a high throughput differential scanning fluorimeter (UNCHAINED, Specification: Unit). The experimental results showed that the PL bispecific antibodies still had good binding activity to the antigen; meanwhile, PL-2 to PL-19 had significantly improved Tm1 (°C), Tagg 266 (°C), and Tonset (°C) as compared to PL-1, and therefore, the bispecific antibodies had better thermal stability.

**Table 10. Binding activity assay on PL bispecific antibodies**

| No. | hPDL1 | hCTLA4 | No. | hPDL1 | hCTLA4 |
|---|---|---|---|---|---|
| | EC₅₀ (nM) | | | EC₅₀ (nM) | |
| PL-1 | 1.6 | 16.5 | PL-11 | 0.6 | 5.6 |
| PL-2 | 0.5 | 8.0 | PL-12 | 0.5 | 8.0 |
| PL-3 | 0.6 | 7.9 | PL-13 | 0.5 | 4.4 |
| PL-4 | 0.7 | 6.5 | PL-14 | 1.3 | 6.0 |
| PL-5 | 0.7 | 6.7 | PL-15 | 1.4 | 3.2 |
| PL-6 | 0.8 | 5.3 | PL-16 | 1.7 | 34.9 |
| PL-7 | 0.5 | 7.3 | PL-17 | 1.4 | 6.2 |
| PL-8 | 1.4 | 14.5 | PL-18 | 1.6 | 6.7 |
| PL-9 | 0.6 | 5.5 | PL-19 | 1.6 | 6.5 |
| PL-10 | 0.6 | 6.9 | | | |

**Table 11. Thermal stability test results for PL bispecific antibodies**

| No. | Tm1 (°C) | Tagg 266 (°C) | Tonset (°C) | No. | Tm1 (°C) | Tagg 266 (°C) | Tonset (°C) |
|---|---|---|---|---|---|---|---|
| PL-1 | 61.64 | 64.82 | 52.566 | PL-11 | 65.88 | - | 57.976 |
| PL-2 | 66.20 | 66.55 | 58.317 | PL-12 | 65.54 | 67.94 | - |
| PL-3 | 64.07 | 68.28 | 57.661 | PL-13 | 71.85 | 68.17 | 58.581 |
| PL-4 | 70.71 | 67.29 | 56.246 | PL-14 | 74.18 | 69.42 | 58.589 |
| PL-5 | 74.56 | 68.11 | 58.407 | PL-15 | 70.96 | 69.91 | 58.622 |
| PL-6 | 70.43 | 70.12 | 61.069 | PL-16 | 63.48 | 68.98 | 58.702 |
| PL-7 | 68.46 | 67.41 | 63.031 | PL-17 | 70.15 | 69.86 | 56.193 |
| PL-8 | 65.00 | - | - | PL-18 | - | 69.43 | - |
| PL-9 | 69.24 | 67.83 | 58.597 | PL-19 | - | 69.52 | 57.766 |
| PL-10 | 69.63 | 68.12 | 56.788 | | | | |

### 1.3 HJ bispecific antibodies

Construction of HJ bispecific antibodies against hIL5 and hTSLP: HJ-3 to HJ11 comprising a first heavy chain, a second heavy chain, a first light chain, and a second light chain as described below:
the first heavy chain is, from N-terminus to C-terminus, [VH1-H]-[linker 1]-[Titin chain]-[Fc1],
the first light chain is, from N-terminus to C-terminus, [VL1-H]-[linker 2]-[Obscurin chain],
the second heavy chain is, from N-terminus to C-terminus, [VH2-J]-[CHl]-[Fc2], and the second light chain is, from N-terminus to C-terminus, [VL2-J]-[CL];
wherein the VH1-H and VL1-H were the heavy chain variable region and the light chain variable region of H0 in WO2021139758A1, respectively, and the VH2-J and VL2-J were the heavy chain variable region and the light chain variable region of J1 in WO2021139758A1, respectively. The structures of the Obscurin chain, the Titin chain, the linker 1, and the linker 2 in the HJ bispecific antibodies of this example are shown in the table below.

**Table 12. Obscurin chain/Titin chain and linkers in HJ bispecific antibodies**

| No. | First heavy chain | | First light chain | |
|---|---|---|---|---|
| | Titin chain | Linker 1 | Obscurin chain | Linker 2 |
| HJ-3 | T.10 | GGGGS | O.20 | GGGGS |
| HJ-5 | T.16 | GGGGS | O.25 | GGGGS |
| HJ-6 | T.16 | GGGGS | O.27 | GGGGS |
| HJ-7 | T.16 | GGGGS | O.28 | GGGGS |
| HJ-8 | T.16 | ASTKG | O.25 | RTVAS |
| HJ-9 | T.16 | ASTKG | O.27 | RTVAS |
| HJ-10 | T.16 | ASTKG | O.28 | RTVAS |
| HJ-11 | T.16 | ASTKG | O.29 | RTVAS |

The antigen-binding activity of HJ bispecific antibodies was tested by the method with reference to Test Example 4 of WO2021139758A1. The thermal stability test was performed on the antibodies by the following process: the dilutions of the HJ bispecific antibodies were formulated with a buffer containing 10 mM acetic acid at pH 5.5 and 9% sucrose; the bispecific antibodies were concentrated by ultrafiltration and concentration to obtain different concentrations of solutions (the HJ bispecific antibody concentrations are shown in Table 13-2) of the HJ bispecific antibodies; the concentrated solutions were incubated in an incubator at 40 °C, and the samples were tested for SEC purity on day 0 (i.e., before the start of incubation at 40 °C, D0), day 7 (the 7th day of incubation at 40 °C, D7), day 14 (the 14th day of incubation at 40 °C, D14), day 21 (the 21st day of incubation at 40 °C, D21), and day 28 (the 28th day of incubation at 40 °C, D28); after 28 days of incubation at 40 °C, the samples were immediately taken for the determination of CE-SDS purity. The experimental results showed that the binding activity of the HJ bispecific antibodies constructed in the present disclosure against the antigen was not significantly changed; meanwhile, the HJ-5 to HJ-11 bispecific antibodies had better thermal stability than HJ-3.

**Table 13-1. Binding activity assay on HJ bispecific antibodies**

| No. | hIL5 | hTSLP | No. | hIL5 | hTSLP |
|---|---|---|---|---|---|
| | EC₅₀ (nM) | | | EC₅₀ (nM) | |
| HJ-3 | 17.38 | 3.234 | HJ-8 | 18.74 | 3.332 |
| HJ-5 | 15.96 | 2.639 | HJ-9 | 10.86 | 3.184 |
| HJ-6 | 37.05 | 2.884 | HJ-10 | 20.81 | 3.173 |
| HJ-7 | 17.69 | 2.182 | HJ-11 | 9.464 | 3.005 |

**Table 13-2. Accelerated stability test results for HJ bispecific antibodies**

| No. | D0 concentration (mg/mL) | D0 SEC purity (%) | D28 SEC purity (%) | D28Δ SEC purity (%) | D28 non-reduced CE-SDS purity (%) |
|---|---|---|---|---|---|
| HJ-3 | 50 | 98 | 84 | 14 | 67 |
| HJ-5 | 60.8 | 98.09 | 93.86 | 4.23 | 82.93 |
| HJ-6 | 54 | 98.14 | 89.68 | 8.46 | 80.76 |
| HJ-7 | 56.8 | 97.7 | 92.72 | 4.98 | 85.47 |
| HJ-8 | 62.6 | 93.39 | 84.28 | 9.11 | 73.03 |
| HJ-9 | 53.5 | 90.01 | 85.93 | 4.08 | 80.32 |
| HJ-10 | 69.8 | 90.9 | 88.31 | 2.59 | 80.4 |
| HJ-11 | 50.4 | 92.55 | 90.89 | 1.66 | 82.96 |

### Example 2. Screening and Identification of Anti-Human PSMA Hybridoma Antibodies

Monoclonal antibodies against human PSMA were prepared by hybridoma technology in the present disclosure. The obtained antibodies bind specifically to human PSMA with high affinity and can cross-react with cynomolgus monkey PSMA. The obtained antibodies have relatively good binding activity to human PSMA and cynomolgus monkey PSMA on the cell surface.

Human PSMA-ECD-his, LnCap cells, or ChoK1-human PSMA cells were used as an immunization agent, or human PSMA-ECD-his and LnCap cells were used as cross-immunization agents, and TiterMax^{®} Gold Adjuvant (Sigma Cat No. T2684) and Thermo Imject^{®} Alum (Thermo Cat No. 77161) were used as adjuvants to cross-immunize mice. After the first immunization and 7 boost immunizations, mice 1-1, 1-2, 2-1, 2-5, 3-3, 3-4, 3-5, and 4-2#, with high antibody titers in serum (titer > 307.2K), were selected for spleen cell fusion. After fusion, the hybridoma culture supernatant was assayed based on the growth density of hybridoma cells, and antibodies specifically binding to cell surface PSMA were selected by screening. Monoclonal hybridoma cell strains with good activity were obtained by screening. Hybridoma cells at logarithmic growth phase were harvested, and the RNA was extracted using NucleoZol (MN) (following the procedures in the kit instructions) and reverse-transcribed (PrimeScript^{™} Reverse Transcriptase, Takara, cat # 2680A). The cDNA obtained by the reverse transcription was amplified by PCR using mouse Ig-Primer Set (Novagen, TB326 Rev.B 0503) and then sent for sequencing. The amino acid sequences of the CDRs and variable regions of the monoclonal hybridoma cell strains obtained by screening are as follows:

**Table 14. The CDRs of PSMA antibodies**

| No. | Heavy chain | | Light chain | |
|---|---|---|---|---|
| 2 | HCDR1 | DYYMA (SEQ ID NO: 1) | LCDR1 | SASSSVSHMY (SEQ ID NO: 4) |
| | HCDR2 | NINFDGSSTYYLDSLKS (SEQ ID NO: 2) | LCDR2 | RTSNLAS (SEQ ID NO: 5) |
| | HCDR3 | DLDGYYGYFDV (SEQ ID NO: 3) | LCDR3 | QQYHSYPLM (SEQ ID NO: 6) |
| 13 | HCDR1 | NYAIS (SEQ ID NO: 7) | LCDR1 | RASQDISNYLN (SEQ ID NO: 10) |
| | HCDR2 | VIWTGGGTHYNSALKS (SEQ ID NO: 8) | LCDR2 | YTSRLQS (SEQ ID NO: 11) |
| | HCDR3 | KGNYYGSSLYYYAMNY (SEQ ID NO: 9) | LCDR3 | QQGDTLPYT (SEQ ID NO: 12) |
| 15 | HCDR1 | DHNMN (SEQ ID NO: 13) | LCDR1 | KASQGVDTAVA (SEQ ID NO: 16) |
| | HCDR2 | LINPNYGTADYNQKFKG (SEQ ID NO: 14) | LCDR2 | WASTRHT (SEQ ID NO: 17) |
| | HCDR3 | GSSLFAY (SEQ ID NO: 15) | LCDR3 | QQYSRYPLT (SEQ ID NO: 18) |
| 19 | HCDR1 | DYYMA (SEQ ID NO: 19) | LCDR1 | RASQDISKYLN (SEQ ID NO: 22) |
| | HCDR2 | NINYDGSSTYYLDSLKS (SEQ ID NO: 20) | LCDR2 | YTSSLHS (SEQ ID NO: 23) |
| | HCDR3 | GGDYWDAMDY (SEQ ID NO: 21) | LCDR3 | QQGNRLPRT (SEQ ID NO: 24) |
| 27 | HCDR1 | DYYMA (SEQ ID NO: 25) | LCDR1 | SASSSVSYMY (SEQ ID NO: 28) |
| | HCDR2 | NINYDGSSTYYLDSLKS (SEQ ID NO: 26) | LCDR2 | RTSNLAS (SEQ ID NO: 29) |
| | HCDR3 | DLDGYYGYFDV (SEQ ID NO: 27) | LCDR3 | QQYHSYPLT (SEQ ID NO: 30) |
| 31 | HCDR1 | DYYMA (SEQ ID NO: 31) | LCDR1 | RASGNIHNYLA (SEQ ID NO: 34) |
| | HCDR2 | SINYDGSSTYYLDSLKS (SEQ ID NO: 32) | LCDR2 | NAETLAD (SEQ ID NO: 35) |
| | HCDR3 | SDGYWAWFAY (SEQ ID NO: 33) | LCDR3 | QHFWSTPPT (SEQ ID NO: 36) |
| 41 | HCDR1 | SYGIS (SEQ ID NO: 37) | LCDR1 | RASQDIGSSLN (SEQ ID NO: 40) |
| | HCDR2 | EIFPRRGNTYYNEKFRG (SEQ ID NO: 38) | LCDR2 | ATSSLDS (SEQ ID NO: 41) |
| | HCDR3 | VFDGYYNYAMDY (SEQ ID NO: 39) | LCDR3 | LQYASSPYT (SEQ ID NO: 42) |
| 43 | HCDR1 | NYWMN (SEQ ID NO: 43) | LCDR1 | KASQNVHTSVA (SEQ ID NO: 46) |
| | HCDR2 | QVRLKSDNYATHYAESVK G (SEQ ID NO: 44) | LCDR2 | SASNRYS (SEQ ID NO: 47) |
| | HCDR3 | NYYGPAGPFDF (SEQ ID NO: 45) | LCDR3 | QQYSSYPLT (SEQ ID NO: 48) |
| 46 | HCDR1 | NYGMS (SEQ ID NO: 49) | LCDR1 | KASQSVGTAVA (SEQ ID NO: 52) |
| | HCDR2 | TIGSGGGYTYYSDSVKG (SEQ ID NO: 50) | LCDR2 | SASSRYT (SEQ ID NO: 53) |
| | HCDR3 | HPQIGY (SEQ ID NO: 51) | LCDR3 | HQYSSYPLT (SEQ ID NO: 54) |
| 50 | HCDR1 | NYGIS (SEQ ID NO: 55) | LCDR1 | RSSQSILHSNGNTYL E (SEQ ID NO: 58) |
| | HCDR2 | EIYPRSGNTYYNTNFKG (SEQ ID NO: 56) | LCDR2 | RVSNRFS (SEQ ID NO: 59) |
| | HCDR3 | NPFTTVVEGFAF (SEQ ID NO: 57) | LCDR3 | FQGLHVPPT (SEQ ID NO: 60) |
| 71 | HCDR1 | NYRMH (SEQ ID NO: 61) | LCDR1 | RSSQSLVHSYGNTYL H (SEQ ID NO: 64) |
| | HCDR2 | VITVKSDNYGSSYAESVKG (SEQ ID NO: 62) | LCDR2 | KVSNRFS (SEQ ID NO: 65) |
| | HCDR3 | ELGQVLVF (SEQ ID NO: 63) | LCDR3 | SQTTHVPWT (SEQ ID NO: 66) |
| 73 | HCDR1 | TSWMN (SEQ ID NO: 67) | LCDR1 | STSQDIYNNLS (SEQ ID NO: 70) |
| | HCDR2 | RIYPGDGDTKYNGKFQG (SEQ ID NO: 68) | LCDR2 | YTSSLHS (SEQ ID NO: 71) |
| | HCDR3 | SIYYLYYFDY (SEQ ID NO: 69) | LCDR3 | QQYNNLPFT (SEQ ID NO: 72) |

> 2 murine heavy chain variable region (SEQ ID NO: 73) 
EVKLVESEGGLVQPGSSMKLSCTASGFTFSDYYMAWVRQVPEKGLEWVANIN
FDGSSTYYLDSLKSRFIISRDNAKNILYLQMSSLKSEDTATYYCARDLDGYYG
YFDVWGTGTTVTVSS
> 2 murine light chain variable region (SEQ ID NO: 74) 
QIVLTQSPAIMSASPGEKVTISCSASSSVSHMYWYQQKPGSSPKPWIYRTSNLA
SGVPARFSGSGSGTSYSLTISSMEAEDAATYYCQQYHSYPLMFGAGTKLELK
> 13 murine heavy chain variable region (SEQ ID NO: 75) 
QVQLKESGPGLVAPSQSLSITCTVSGFSLTNYAISWVRQPPGKGLEWLGVIWTG
GGTHYNSALKSRLSISKDNSKSQVFLKMNSLQTDDTARYYCARKGNYYGSSL
YYYAMNYWGQGTSVTVSSTVSS
> 13 murine light chain variable region (SEQ ID NO: 76) 
DIQMTQTTSSLSASLGDRVTISCRASQDISNYLNWYQQKPDGTVKLLIYYTSR
LQSGVPSRFSGSGSGTDYSLSISNLEQEDIATYFCQQGDTLPYTFGGGTKLEI
> 15 murine heavy chain variable region (SEQ ID NO: 77) 
EFQLQQSGPELVKPGASVKISCKASGYSFTDHNMNWVKQSNGKSLEWIGLIN
PNYGTADYNQKFKGKATLTVDQSSTTAFMQFTSLTSEDSAVYYCARGSSLFAY
WGQGTLVTVSA
> 15 murine light chain variable region (SEQ ID NO: 78) 
DIVMTQSHKFMSTSVGDRVNITCKASQGVDTAVAWYQQKPGQSPKLLIYWAS
TRHTGVPDRFTGSGSGTDFTLTISNVQSEDLADYFCQQYSRYPLTFGAGTKLE
LK
> 19 murine heavy chain variable region (SEQ ID NO: 79) 
EVKLVESEGGLVQPGSSMKLSCTASGFTFSDYYMAWVRQVPERGLEWVANIN
YDGSSTYYLDSLKSRFIISRDNAKNILYLQMSSLKSEDTATYYCARGGDYWDA
 MDYWGQGTSVTVSS
> 19 murine light chain variable region (SEQ ID NO: 80) 
DIQMTQTTSSLSASLGDRVTISCRASQDISKYLNWYQQKPDGTVKLLIYYTSSL
HSGVPSRFSGSGSGTDYSLTISNLEQEDIATYFCQQGNRLPRTFGGGTKLEIK
> 27 murine heavy chain variable region (SEQ ID NO: 81) 
EVKLVESEGGLVQPGSSMKLSCTASGFTFSDYYMAWVRQVPEKGLEWVANIN
YDGSSTYYLDSLKSRFIISRDNAKNILYLQMSSLKSEDTATYYCARDLDGYYG
YFDVWGTGTTVTVSS
> 27 murine light chain variable region (SEQ ID NO: 82) 
QIVLTQSPAIMSASPGEKVTISCSASSSVSYMYWYQQKPGSSPKPWIYRTSNLA
SGVPARFSGSGSGTSYSLTISSMEAEDAATYYCQQYHSYPLTFGAGTKLELK
> 31 murine heavy chain variable region (SEQ ID NO: 83) 
EVKLVESEGGLVQPGSSKKLSCTASGFTFSDYYMAWVRQVPEKGLEWVASIN
YDGSSTYYLDSLKSRFIISRDNAKNNLYLQMNSLKSEDTATYYCAGSDGYWA
WFAYWGQGTLVTVSA
> 31 murine light chain variable region (SEQ ID NO: 84) 
DIQMTQSPASLSASVGEAVTITCRASGNIHNYLAWYQQKQGKSPQLLVYNAET
LADGVPSRFSGSGSGTQFSLKINSLRPEDFGSYYCQHFWSTPPTFGGGTKLEIK
> 41 murine heavy chain variable region (SEQ ID NO: 85) 
QVQLQQSGAELARPGASVKLSCKASGYTFTSYGISWVKQRTGQGLEWIGEIFP
RRGNTYYNEKFRGKATLTADKSSSAAYMELRSLTSEDSAVYFCARVFDGYYN
YAMDYWGQGTSVTVSS
> 41 murine light chain variable region (SEQ ID NO: 86) 
DIQMTQSPSSLSASLGERVSLTCRASQDIGSSLNWLQQQPDGTIKRLIYATSSLD
SGVPTRFSGSRSGSDYSLTISSLESEDFVDYYCLQYASSPYTFGGGTKLEIK
> 43 murine heavy chain variable region (SEQ ID NO: 87) 
EVKFEESGGGLVQPGGSMKLSCVASGFTFSNYWMNWLRQSPEKGLEWVAQV
RLKSDNYATHYAESVKGRFTISRDDSKSSAYLQMNNLRAEDTGIYYCSENYYG
PAGPFDFWGQGTTLTVSS
> 43 murine light chain variable region (SEQ ID NO: 88) 
DIVMTQSQKFMSTTVGDRVSITCKASQNVHTSVAWYQQKPGQSPKLLIYSAS
NRYSGVPDRFTGSGSGTDFTLTINNMQSEDLADYFCQQYSSYPLTFGVGTKLE
LK
> 46 murine heavy chain variable region (SEQ ID NO: 89) 
EVQLVESGGDLVKPGGSLKLSCAASGFTFSNYGMSWVRQTPDKRIKWVATIG
SGGGYTYYSDSVKGRFTISRDNAKNTLYLQMSSLKSEDTAIYYCVRHPQIGY
WGQGTLVTVSA
> 46 murine light chain variable region (SEQ ID NO: 90) 
DIVMTQSQKFMSTTVGDRVTITCKASQSVGTAVAWYQQKPGQSPKLLIYSASS
RYTGVPDRFTGSGSGTDFTLTITNMQSEDLADYFCHQYSSYPLTFGGGTKLEIK
> 50 murine heavy chain variable region (SEQ ID NO: 91) 
QVQLQQSGAELARPGASVKLSCKASGFTFTNYGISWVKQRTGQGLEWIGEIY
PRSGNTYYNTNFKGEATLTADKSSSTAFMELRSLTSEDSAVYFCARNPFTTVVE
GFAFWGQGTLVTVSA
> 50 murine light chain variable region (SEQ ID NO: 92) 
DVFMTQSPLSLPVSLGDQASISCRSSQSILHSNGNTYLEWYLQKPGQSPKLLIY
RVSNRFSGVPDRFSGSGSGTDFTLKISRVEAEDLGVYYCFQGLHVPPTFGAGT
KLELK
> 71 murine heavy chain variable region (SEQ ID NO: 93) 
QVQLVETGGGLVRPGNSLKLSCVTSGFTFSNYRMHWLRQPPGKRLEWIAVIT
VKSDNYGSSYAESVKGRFAISRDDSKSSVYLEMNRLREEDTATYFCSRELGQV
LVFWGQGTLVTVSA
> 71 murine light chain variable region (SEQ ID NO: 94) 
DVVVTQTPLSLPVSLGDQASISCRSSQSLVHSYGNTYLHWYLQKPGQSPKLLI
YKVSNRFSGVPDRFSGSGSGTDFTLKISRVEAEDLGVYFCSQTTHVPWTFGGG
TKLEIK
> 73 murine heavy chain variable region (SEQ ID NO: 95) 
QVQLQQSGPELVKPGASVKISCKASGYAFSTSWMNWVKERPGKGLEWIGRIY
PGDGDTKYNGKFQGKATLTADKSSSTAYMKLSSLTSEDSAVYFCVRSIYYLYY
FDYWGQGTTLTVSS
> 73 murine light chain variable region (SEQ ID NO: 96) 
DIQMTQPTSSLSASLGDRVTISCSTSQDIYNNLSWFQQKPDGTVRLLIYYTSSL
HSGVPSRFSGSGSGTDYSLTISNLEPEDIATYYCQQYNNLPFTFGGGTNLEIR;
Note: The CDRs obtained based on the Kabat numbering scheme are underlined.

The variable region sequences of the murine anti-PSMA antibodies were combined with the constant regions set forth in SEQ ID NO: 97 and SEQ ID NO: 98 to obtain chimeric antibodies. Illustratively, Chi13 represents a chimeric antibody comprising the 13 murine heavy chain variable region, the 13 murine light chain variable region, and the constant regions.
> hIgG1: CH1-Fc (SEQ ID NO: 97)
> CL (SEQ ID NO: 98)



.

### Example 3. Humanization Design for Anti-Human PSMA Monoclonal Antibodies

Humanization of the murine monoclonal antibodies was performed according to the method described in many publications in the art. In brief, based on the typical structures of the VH/VL CDRs of the murine antibodies obtained, a human germline database was searched for homologous sequences of the light chain variable regions (VL) and the heavy chain variable regions (VH); the CDRs of the murine antibodies were grafted onto a human template, and some residues of the VL and VH were mutated; the constant regions of the murine antibodies were replaced with a human constant region to obtain the final humanized molecules.

### 3-1. Humanization of antibody 2

For the humanized antibody of murine antibody 2, the FR1, FR2, and FR3 of IGHV3-7*01 and the FR4 of IGHJ6*01 were selected as a heavy chain framework region template, and the FR1, FR2, and FR3 of IGKV6-21*02 and the FR4 of IGKJ2*01 were selected as a light chain framework region template. Optionally, the amino acid residue at position 49 of the light chain variable region of the humanized antibody was substituted; in particular, the mutation was K49Y (according to the Kabat numbering scheme, K at position 49 was mutated to Y). The sequences of the antibody variable regions are as follows:
> hu2H1 (SEQ ID NO: 99) 
EVQLVESGGGLVQPGGSLRLSCAASGFTFSDYYMAWVRQAPGKGLEWVANI
NFDGSSTYYLDSLKSRFTISRDNAKNSLYLQMNSLRAEDTAVYYCARDLDGY
YGYFDVWGQGTTVTVSS
> hu2L1 (SEQ ID NO: 100)

EIVLTQSPDFQSVTPKEKVTITCSASSSVSHMYWYQQKPDQSPKLLIYRTSNLA
SGVPSRFSGSGSGTDFTLTINSLEAEDAATYYCQQYHSYPLMFGQGTKLEIK.

### 3-2. Humanization of antibody 13

For the humanized antibody of murine antibody 13, the FR1, FR2, and FR3 of IGHV2-26*01 and the FR4 of IGHJ6*01 were selected as a heavy chain framework region template, and the FR1, FR2, and FR3 of IGKV1-39*01 and the FR4 of IGKJ4*01 were selected as a light chain framework region template. Optionally, the amino acid residue(s) at position(s) 1, 30, 44, 49, 73, and/or 89 in the heavy chain variable region of the humanized antibody was/were substituted; and/or the amino acid residue(s) at position(s) 41, 42, 43, 44, and/or 71 in the light chain variable region of the humanized antibody was/were substituted.

**Table 15-1. Mutations for humanized antibody 13**

| 13.VL | | 13.VH | |
|---|---|---|---|
| L1 | | H1 | Q1E |
| L2 | G41D, K42G, F71Y | H2 | Q1E, A49G, T89R |
| L3 | G41D, K42G, A43T, P44V, F71Y | H3 | Q1E, S30T, A44G, A49G, T73N, T89R |

The sequences of the antibody variable regions are as follows:
> hu13H1 (SEQ ID NO: 101) 
EVTLKESGPVLVKPTETLTLTCTVSGFSLSNYAISWIRQPPGKALEWLAVIWTG
GGTHYNSALKSRLTISKDTSKSQVVLTMTNMDPVDTATYYCARKGNYYGSSL
YYYAMNYWGQGTTVTVSS
> hu13H2 (SEQ ID NO: 102) 
EVTLKESGPVLVKPTETLTLTCTVSGFSLSNYAISWIRQPPGKALEWLGVIWTG
GGTHYNSALKSRLTISKDTSKSQVVLTMTNMDPVDTARYYCARKGNYYGSSL
YYYAMNYWGQGTTVTVSS
> hu13H3 (SEQ ID NO: 103) 
EVTLKESGPVLVKPTETLTLTCTVSGFSLTNYAISWIRQPPGKGLEWLGVIWTG
GGTHYNSALKSRLTISKDNSKSQVVLTMTNMDPVDTARYYCARKGNYYGSS
LYYYAMNYWGQGTTVTVSS
> hu13L1 (SEQ ID NO: 104) 
DIQMTQSPSSLSASVGDRVTITCRASQDISNYLNWYQQKPGKAPKLLIYYTSR
LQSGVPSRFSGSGSGTDFTLTISSLQPEDFATYYCQQGDTLPYTFGGGTKVEIK
> hu13L2 (SEQ ID NO: 105) 
DIQMTQSPSSLSASVGDRVTITCRASQDISNYLNWYQQKPDGAPKLLIYYTSR
LQSGVPSRFSGSGSGTDYTLTISSLQPEDFATYYCQQGDTLPYTFGGGTKVEIK
> hu13L3 (SEQ ID NO: 106)

DIQMTQSPSSLSASVGDRVTITCRASQDISNYLNWYQQKPDGTVKLLIYYTSR
LQSGVPSRFSGSGSGTDYTLTISSLQPEDFATYYCQQGDTLPYTFGGGTKVEIK.

### 3-3. Humanization of antibody 15

For the humanized antibody of murine antibody 15, the FR1, FR2, and FR3 of IGHV1-3*01 and the FR4 of IGHJ1*01 were selected as a heavy chain framework region template, and the FR1, FR2, and FR3 of IGKV1-27*01 and the FR4 of IGKJ2*01 were selected as a light chain framework region template. Optionally, the amino acid residue(s) at position(s) 1, 2, 28, 44, 48, 67, 69, 71, 73, and/or 76 in the heavy chain variable region of the humanized antibody was/were substituted; and/or the amino acid residue(s) at position(s) 43, 60, and/or 100 in the light chain variable region of the humanized antibody was/were substituted.

**Table 15-2. Mutations for humanized antibody 15**

| 15.VL | | 15.VH | |
|---|---|---|---|
| L1 | | H1 | Q1E,V2F |
| L2 | V43S,S60D | H2 | Q1E,V2F,T28S |
| L3 | V43S,S60D,Q100C | H3 | Q1E,V2F,T28S,R71V,T73Q |
| | | H4 | Q1E,V2F,T28S,I69L,R71V,T73Q,S76T |
| | | H5 | Q1E,V2F,T28S,M48I,V67A,I69L,R71V,T7 3Q,S76T |
| | | H6 | Q1E,V2F,T28S,R44C, I69L,R71V,T73Q,S76T |

The sequences of the antibody variable regions are as follows:
> hu15H1 (SEQ ID NO: 107) 
EFQLVQSGAEVKKPGASVKVSCKASGYTFTDHNMNWVRQAPGQRLEWMGL
INPNYGTADYNQKFKGRVTITRDTSASTAYMELSSLRSEDTAVYYCARGSSLFA
YWGQGTLVTVSS
> hu15H2 (SEQ ID NO: 108) 
EFQLVQSGAEVKKPGASVKVSCKASGYSFTDHNMNWVRQAPGQRLEWMGL
INPNYGTADYNQKFKGRVTITRDTSASTAYMELSSLRSEDTAVYYCARGSSLFA
YWGQGTLVTVSS
> hu15H3 (SEQ ID NO: 109) 
EFQLVQSGAEVKKPGASVKVSCKASGYSFTDHNMNWVRQAPGQRLEWMGL
INPNYGTADYNQKFKGRVTITVDQSASTAYMELSSLRSEDTAVYYCARGSSLF
AYWGQGTLVTVSS
> hu15H4 (SEQ ID NO: 110) 
EFQLVQSGAEVKKPGASVKVSCKASGYSFTDHNMNWVRQAPGQRLEWMGL
INPNYGTADYNQKFKGRVTLTVDQSATTAYMELSSLRSEDTAVYYCARGSSLF
AYWGQGTLVTVSS
> hu15H5 (SEQ ID NO: 111) 
EFQLVQSGAEVKKPGASVKVSCKASGYSFTDHNMNWVRQAPGQRLEWIGLI
NPNYGTADYNQKFKGRATLTVDQSATTAYMELSSLRSEDTAVYYCARGSSLF
AYWGQGTLVTVSS
> hu15H6 (SEQ ID NO: 112) 
EFQLVQSGAEVKKPGASVKVSCKASGYSFTDHNMNWVRQAPGQCLEWMGL
INPNYGTADYNQKFKGRVTLTVDQSATTAYMELSSLRSEDTAVYYCARGSSLF
AYWGQGTLVTVSS
> hu15L1 (SEQ ID NO: 113) 
DIQMTQSPSSLSASVGDRVTITCKASQGVDTAVAWYQQKPGKVPKLLIYWAST
RHTGVPSRFSGSGSGTDFTLTISSLQPEDVATYYCQQYSRYPLTFGQGTKLEIK
> hu15L2 (SEQ ID NO: 114) 
DIQMTQSPSSLSASVGDRVTITCKASQGVDTAVAWYQQKPGKSPKLLIYWAST
RHTGVPDRFSGSGSGTDFTLTISSLQPEDVATYYCQQYSRYPLTFGQGTKLEIK
> hu15L3 (SEQ ID NO: 115)

DIQMTQSPSSLSASVGDRVTITCKASQGVDTAVAWYQQKPGKSPKLLIYWAST
RHTGVPDRFSGSGSGTDFTLTISSLQPEDVATYYCQQYSRYPLTFGCGTKLEIK.

### 3-4. Humanization of antibody 19

For the humanized antibody of murine antibody 19, the FR1, FR2, and FR3 of IGHV3-7*01 and the FR4 of IGHJ6*01 were selected as a heavy chain framework region template, and the FR1, FR2, and FR3 of IGKV1-39*01 and the FR4 of IGKJ4*01 were selected as a light chain framework region template. Optionally, the amino acid residue at position 3 in the heavy chain variable region of the humanized antibody was substituted; and/or the amino acid residue at position 71 in the light chain variable region of the humanized antibody was substituted.

**Table 15-3. Mutations for humanized antibody 19**

| 19.VL | | 19.VH | |
|---|---|---|---|
| L1 | | H1 | |
| L2 | F71Y | H2 | Q3K |

The sequences of the antibody variable regions are as follows:
> hu19H1 (SEQ ID NO: 116) 
EVQLVESGGGLVQPGGSLRLSCAASGFTFSDYYMAWVRQAPGKGLEWVANI
NYDGSSTYYLDSLKSRFTISRDNAKNSLYLQMNSLRAEDTAVYYCARGGDY
WDAMDYWGQGTTVTVSS
> hu19H2 (SEQ ID NO: 117) 
EVKLVESGGGLVQPGGSLRLSCAASGFTFSDYYMAWVRQAPGKGLEWVANI
NYDGSSTYYLDSLKSRFTISRDNAKNSLYLQMNSLRAEDTAVYYCARGGDY
WDAMDYWGQGTTVTVSS
> hu19L1 (SEQ ID NO: 118) 
DIQMTQSPSSLSASVGDRVTITCRASQDISKYLNWYQQKPGKAPKLLIYYTSSL
HSGVPSRFSGSGSGTDFTLTISSLQPEDFATYYCQQGNRLPRTFGGGTKVEIK
> hu19L2 (SEQ ID NO: 119)

DIQMTQSPSSLSASVGDRVTITCRASQDISKYLNWYQQKPGKAPKLLIYYTSSL
HSGVPSRFSGSGSGTDYTLTISSLQPEDFATYYCQQGNRLPRTFGGGTKVEIK.

### 3-5. Humanization of antibody 27

For the humanized antibody of murine antibody 27, the FR1, FR2, and FR3 of IGHV3-7*01 and the FR4 of IGHJ6*01 were selected as a heavy chain framework region template, and the FR1, FR2, and FR3 of IGKV6-21*02 and the FR4 of IGKJ2*01 were selected as a light chain framework region template. Optionally, the amino acid residue(s) at position(s) 1, 22, 46, 47, 49, and/or 71 in the light chain variable region of the humanized antibody was/were substituted.

**Table 15-4. Mutations for humanized antibody 27**

| 27.VL | | 27.VH | |
|---|---|---|---|
| L1 | L46P,L47W,K49Y | H1 | |
| L2 | L46P,L47W,K49Y,F71Y | | |
| L3 | E1Q,L46P,L47W,K49Y,F71Y | | |
| L4 | L46P,L47W,K49Y+T22Q | | |
| L5 | L46P,L47W,K49Y,F71Y+T22Q | | |
| L6 | E1Q,L46P,L47W,K49Y,F71Y+T22Q | | |

The sequences of the antibody variable regions are as follows:
> hu27H1 (SEQ ID NO: 120) 
EVQLVESGGGLVQPGGSLRLSCAASGFTFSDYYMAWVRQAPGKGLEWVANI
NYDGSSTYYLDSLKSRFTISRDNAKNSLYLQMNSLRAEDTAVYYCARDLDGY
YGYFDVWGQGTTVTVSS
> hu27L1 (SEQ ID NO: 121) 
EIVLTQSPDFQSVTPKEKVTITCSASSSVSYMYWYQQKPDQSPKPWIYRTSNL
ASGVPSRFSGSGSGTDFTLTINSLEAEDAATYYCQQYHSYPLTFGQGTKLEIK
> hu27L2 (SEQ ID NO: 122) 
EIVLTQSPDFQSVTPKEKVTITCSASSSVSYMYWYQQKPDQSPKPWIYRTSNL
ASGVPSRFSGSGSGTDYTLTINSLEAEDAATYYCQQYHSYPLTFGQGTKLEIK
> hu27L3 (SEQ ID NO: 123) 
QIVLTQSPDFQSVTPKEKVTITCSASSSVSYMYWYQQKPDQSPKPWIYRTSNL
ASGVPSRFSGSGSGTDYTLTINSLEAEDAATYYCQQYHSYPLTFGQGTKLEIK
> hu27L4 (SEQ ID NO: 124) 
EIVLTQSPDFQSVTPKEKVTIQCSASSSVSYMYWYQQKPDQSPKPWIYRTSNL
ASGVPSRFSGSGSGTDFTLTINSLEAEDAATYYCQQYHSYPLTFGQGTKLEIK
> hu27L5 (SEQ ID NO: 125) 
EIVLTQSPDFQSVTPKEKVTIQCSASSSVSYMYWYQQKPDQSPKPWIYRTSNL
ASGVPSRFSGSGSGTDYTLTINSLEAEDAATYYCQQYHSYPLTFGQGTKLEIK
> hu27L6 (SEQ ID NO: 126)

QIVLTQSPDFQSVTPKEKVTIQCSASSSVSYMYWYQQKPDQSPKPWIYRTSNL
ASGVPSRFSGSGSGTDYTLTINSLEAEDAATYYCQQYHSYPLTFGQGTKLEIK.

### 3-6. Humanization of antibody 31

For the humanized antibody of murine antibody 31, the FR1, FR2, and FR3 of IGHV3-7*01 and the FR4 of IGHJ1*01 were selected as a heavy chain framework region template, and the FR1, FR2, and FR3 of IGKV1-39*01 and the FR4 of IGKJ4*01 were selected as a light chain framework region template. Optionally, the amino acid residue at position 94 in the heavy chain variable region of the humanized antibody was substituted; and/or the amino acid residue(s) at position(s) 43, 45, 48, and/or 70 in the light chain variable region of the humanized antibody was/were substituted.

**Table 15-5. Mutations for humanized antibody 31**

| 31.VL | | 31.VH | |
|---|---|---|---|
| L1 | | H1 | R94G |
| L2 | K45Q,I48V | | |
| L3 | A43S,K45Q,I48V,D70Q | | |

The sequences of the antibody variable regions are as follows:
> hu31H1 (SEQ ID NO: 127) 
EVQLVESGGGLVQPGGSLRLSCAASGFTFSDYYMAWVRQAPGKGLEWVASI
NYDGSSTYYLDSLKSRFTISRDNAKNSLYLQMNSLRAEDTAVYYCAGSDGYW
AWFAYWGQGTLVTVSS
> hu31L1 (SEQ ID NO: 128) 
DIQMTQSPSSLSASVGDRVTITCRASGNIHNYLAWYQQKPGKAPKLLIYNAET
LADGVPSRFSGSGSGTDFTLTISSLQPEDFATYYCQHFWSTPPTFGGGTKVEIK
> hu31L2 (SEQ ID NO: 129 
DIQMTQSPSSLSASVGDRVTITCRASGNIHNYLAWYQQKPGKAPQLLVYNAE
TLADGVPSRFSGSGSGTDFTLTISSLQPEDFATYYCQHFWSTPPTFGGGTKVEI
K
> hu31L3 (SEQ ID NO: 130)

DIQMTQSPSSLSASVGDRVTITCRASGNIHNYLAWYQQKPGKSPQLLVYNAET
LADGVPSRFSGSGSGTQFTLTISSLQPEDFATYYCQHFWSTPPTFGGGTKVEIK.

### 3-7. Humanization of antibody 41

For the humanized antibody of murine antibody 41, the FR1, FR2, and FR3 of IGHV1-18*01 and the FR4 of IGHJ6*01 were selected as a heavy chain framework region template, and the FR1, FR2, and FR3 of IGKV1-39*01 and the FR4 of IGKJ4*01 were selected as a light chain framework region template. Optionally, the amino acid residue(s) at position(s) 1, 69, 71, and/or 73 in the heavy chain variable region of the humanized antibody was/were substituted; and/or the amino acid residue(s) at position(s) 36, 42, 44, 46, 66, 69, and/or 71 in the light chain variable region of the humanized antibody was/were substituted.

**Table 15-6. Mutations for humanized antibody 41**

| 41.VL | | 41.VH | |
|---|---|---|---|
| L1 | Y36L,L46R,G66R | H1 | Q1E,T73K |
| L2 | Y36L,P44I,L46R,G66R,F71Y | H2 | Q1E,M69L,T71A,T73K |
| L3 | Y36L,K42G,P44 I,L46R,G66R,T69S,F71Y | | |

The sequences of the antibody variable regions are as follows:
> hu41H1 (SEQ ID NO: 131) 
EVQLVQSGAEVKKPGASVKVSCKASGYTFTSYGISWVRQAPGQGLEWMGEIF
PRRGNTYYNEKFRGRVTMTTDKSTSTAYMELRSLRSDDTAVYYCARVFDGYY
NYAMDYWGQGTTVTVSS
> hu41H2 (SEQ ID NO: 132) 
EVQLVQSGAEVKKPGASVKVSCKASGYTFTSYGISWVRQAPGQGLEWMGEIF
PRRGNTYYNEKFRGRVTLTADKSTSTAYMELRSLRSDDTAVYYCARVFDGYY
NYAMDYWGQGTTVTVSS
> hu41L1 (SEQ ID NO: 133) 
DIQMTQSPSSLSASVGDRVTITCRASQDIGSSLNWLQQKPGKAPKRLIYATSSL
DSGVPSRFSGSRSGTDFTLTISSLQPEDFATYYCLQYASSPYTFGGGTKVEIK
> hu41L2 (SEQ ID NO: 134) 
DIQMTQSPSSLSASVGDRVTITCRASQDIGSSLNWLQQKPGKAIKRLIYATSSL
DSGVPSRFSGSRSGTDYTLTISSLQPEDFATYYCLQYASSPYTFGGGTKVEIK
> hu41L3 (SEQ ID NO: 135)

DIQMTQSPSSLSASVGDRVTITCRASQDIGSSLNWLQQKPGGAIKRLIYATSSL
DSGVPSRFSGSRSGSDYTLTISSLQPEDFATYYCLQYASSPYTFGGGTKVEIK.

### 3-8. Humanization of antibody 43

For the humanized antibody of murine antibody 43, the FR1, FR2, and FR3 of IGHV3-7*01 and the FR4 of IGHJ6*01 were selected as a heavy chain framework region template, and the FR1, FR2, and FR3 of IGKV1-12*01 or IGKV1-27*01 and the FR4 of IGKJ2*01 were selected as a light chain framework region template. Optionally, the amino acid residue(s) at position(s) 4, 37, 72, 73, 78, 93, and/or 94 in the heavy chain variable region of the humanized antibody was/were substituted; and/or the amino acid residue at position 43 in the light chain variable region of the humanized antibody was substituted.

**Table 15-7. Mutations for humanized antibody 43**

| 43.VL | | 43.VH | |
|---|---|---|---|
| L1 | (IGKV1-12*01) A43S | H1 | L4F,L78A,A93S,R94E |
| L2 | (IGKV1-27*01) V43S | H2 | L4F,N73D,L78A,A93S,R94E + D72E |
| | | H3 | L4F,V37L,N73D,L78A,A93S,R94E+D72E |

The sequences of the antibody variable regions are as follows:
> hu43H1 (SEQ ID NO: 136) EVQFVESGGGLVQPGGSLRLSCAASGFTFSNYWMNWVRQAPGKGLEWVAQ 
VRLKSDNYATHYAESVKGRFTISRDNAKNSAYLQMNSLRAEDTAVYYCSENY
YGPAGPFDFWGQGTTVTVSS
> hu43H2 (SEQ ID NO: 137) 
EVQFVESGGGLVQPGGSLRLSCAASGFTFSNYWMNWVRQAPGKGLEWVAQ
VRLKSDNYATHYAESVKGRFTISREDAKNSAYLQMNSLRAEDTAVYYCSENY
YGPAGPFDFWGQGTTVTVSS
> hu43H3 (SEQ ID NO: 138) 
EVQFVESGGGLVQPGGSLRLSCAASGFTFSNYWMNWLRQAPGKGLEWVAQV
RLKSDNYATHYAESVKGRFTISREDAKNSAYLQMNSLRAEDTAVYYCSENYY
GPAGPFDFWGQGTTVTVSS
> hu43L1 (SEQ ID NO: 139) 
DIQMTQSPSSVSASVGDRVTITCKASQNVHTSVAWYQQKPGKSPKLLIYSASN
RYSGVPSRFSGSGSGTDFTLTISSLQPEDFATYYCQQYSSYPLTFGQGTKLEIK
> hu43L2 (SEQ ID NO: 140)

DIQMTQSPSSLSASVGDRVTITCKASQNVHTSVAWYQQKPGKSPKLLIYSASN
RYSGVPSRFSGSGSGTDFTLTISSLQPEDVATYYCQQYSSYPLTFGQGTKLEIK.

### 3-9. Humanization of antibody 46

For the humanized antibody of murine antibody 46, the FR1, FR2, and FR3 of IGHV3-21*01 and the FR4 of IGHJ1*01 were selected as a heavy chain framework region template, and the FR1, FR2, and FR3 of IGKV1-39*01 and the FR4 of IGKJ4*01 were selected as a light chain framework region template. Optionally, the amino acid residue(s) at position(s) 45, 46, 49, and/or 93 in the heavy chain variable region of the humanized antibody was/were substituted; and/or the amino acid residue(s) at position(s) 60, 85, and/or 87 in the light chain variable region of the humanized antibody was/were substituted.

**Table 15-8. Mutations for humanized antibody 46**

| 46.VL | | 46.VH | |
|---|---|---|---|
| L1 | | H1 | |
| L2 | T85D,Y87F | H2 | A93V |
| L3 | S60D,T85D,Y87F | H3 | L45I,E46K,A93V |
| | | H4 | L45I,E46K,S49A,A93V |

> hu46H1 (SEQ ID NO: 141) 
EVQLVESGGGLVKPGGSLRLSCAASGFTFSNYGMSWVRQAPGKGLEWVSTIG
SGGGYTYYSDSVKGRFTISRDNAKNSLYLQMNSLRAEDTAVYYCARHPQIGY
WGQGTLVTVSS
> hu46H2 (SEQ ID NO: 142) 
EVQLVESGGGLVKPGGSLRLSCAASGFTFSNYGMSWVRQAPGKGLEWVSTIG
SGGGYTYYSDSVKGRFTISRDNAKNSLYLQMNSLRAEDTAVYYCVRHPQIGY
 WGQGTLVTVSS
> hu46H3 (SEQ ID NO: 143) 
EVQLVESGGGLVKPGGSLRLSCAASGFTFSNYGMSWVRQAPGKGIKWVSTIG
SGGGYTYYSDSVKGRFTISRDNAKNSLYLQMNSLRAEDTAVYYCVRHPQIGY
WGQGTLVTVSS
> hu46H4 (SEQ ID NO: 144) 
EVQLVESGGGLVKPGGSLRLSCAASGFTFSNYGMSWVRQAPGKGIKWVATIG
SGGGYTYYSDSVKGRFTISRDNAKNSLYLQMNSLRAEDTAVYYCVRHPQIGY
WGQGTLVTVSS
> hu46L1 (SEQ ID NO: 145) 
DIQMTQSPSSLSASVGDRVTITCKASQSVGTAVAWYQQKPGKAPKLLIYSASS
RYTGVPSRFSGSGSGTDFTLTISSLQPEDFATYYCHQYSSYPLTFGGGTKVEIK
> hu46L2 (SEQ ID NO: 146) 
DIQMTQSPSSLSASVGDRVTITCKASQSVGTAVAWYQQKPGKAPKLLIYSASS
RYTGVPSRFSGSGSGTDFTLTISSLQPEDFADYFCHQYSSYPLTFGGGTKVEIK
> hu46L3 (SEQ ID NO: 147)

DIQMTQSPSSLSASVGDRVTITCKASQSVGTAVAWYQQKPGKAPKLLIYSASS
RYTGVPDRFSGSGSGTDFTLTISSLQPEDFADYFCHQYSSYPLTFGGGTKVEIK.

### 3-10. Humanization of antibody 50

For the humanized antibody of murine antibody 50, the FR1, FR2, and FR3 of IGHV1-18*01 and the FR4 of IGHJ1*01 were selected as a heavy chain framework region template, and the FR1, FR2, and FR3 of IGKV2-28*01 and the FR4 of IGKJ2*01 were selected as a light chain framework region template. Optionally, the amino acid residue(s) at position(s) 1, 27, 69, 71, 73, and/or 85 in the heavy chain variable region of the humanized antibody was/were substituted; and/or the amino acid residue(s) at position(s) 2, 3, and/or 28 in the light chain variable region of the humanized antibody was/were substituted.

**Table 15-9. Mutations for humanized antibody 50**

| 50.VL | | 50.VH | |
|---|---|---|---|
| L1 | Graft (IGKV2-28*01) | H1 | Graft (IGHV1-18*01) + Q1E,Y27F,T73K,D85E |
| L2 | Graft (IGKV2-28*01) + I2V,V3F | H2 | Graft (IGHV1-18*01) + Q1E,Y27F,M69L,T71A,T73K,D85E |
| L3 | Graft (IGKV2-28*01) + N28Y | | |

> hu50H1 (SEQ ID NO: 148) 
EVQLVQSGAEVKKPGASVKVSCKASGFTFTNYGISWVRQAPGQGLEWMGEI
YPRSGNTYYNTNFKGRVTMTTDKSTSTAYMELRSLRSEDTAVYYCARNPFTT
 VVEGFAFWGQGTLVTVSS
> hu50H2 (SEQ ID NO: 149) 
EVQLVQSGAEVKKPGASVKVSCKASGFTFTNYGISWVRQAPGQGLEWMGEI
YPRSGNTYYNTNFKGRVTLTADKSTSTAYMELRSLRSEDTAVYYCARNPFTTV
VEGFAFWGQGTLVTVSS
> hu50L1 (SEQ ID NO: 150) 
DIVMTQSPLSLPVTPGEPASISCRSSQSILHSNGNTYLEWYLQKPGQSPQLLIYR
VSNRFSGVPDRFSGSGSGTDFTLKISRVEAEDVGVYYCFQGLHVPPTFGQGTK
LEIK
> hu50L2 (SEQ ID NO: 151) 
DVFMTQSPLSLPVTPGEPASISCRSSQSILHSNGNTYLEWYLQKPGQSPQLLIYR
VSNRFSGVPDRFSGSGSGTDFTLKISRVEAEDVGVYYCFQGLHVPPTFGQGTK
LEIK
> hu50L3 (SEQ ID NO: 152) 
DIVMTQSPLSLPVTPGEPASISCRSSQSILHSYGNTYLEWYLQKPGQSPQLLIYR
VSNRFSGVPDRFSGSGSGTDFTLKISRVEAEDVGVYYCFQGLHVPPTFGQGTK
LEIK
> hu50L3 LCDR1 RSSQSILHSYGNTYLE (SEQ ID NO: 159).

### 3-11. Humanization of antibody 71

For the humanized antibody of murine antibody 71, the FR1, FR2, and FR3 of IGHV3-30*09 and the FR4 of IGHJ1*01 were selected as a heavy chain framework region template, and the FR1, FR2, and FR3 of IGKV2-40*01 and the FR4 of IGKJ4*01 were selected as a light chain framework region template. Optionally, the amino acid residue(s) at position(s) 1, 24, 37, 44, 72, 73, 76, and/or 93 in the heavy chain variable region of the humanized antibody was/were substituted; and/or the amino acid residue(s) at position(s) 2 and/or 4 in the light chain variable region of the humanized antibody was/were substituted.

**Table 15-10. Mutations for humanized antibody 71**

| 71.VL | | 71.VH | |
|---|---|---|---|
| L1 | | H1 | Q1E,A93S |
| L2 | I2V,M4V | H2 | Q1E,A24T,V37L,A93S |
| | | H3 | Q1E,A24T,N73D,N76S,A93S + D72E |
| | | H4 | Q1E,A24T,V37L,G44R,N73D,N76S,A93S + D72E |

> hu71H1 (SEQ ID NO: 153) 
EVQLVESGGGVVQPGRSLRLSCAASGFTFSNYRMHWVRQAPGKGLEWVAVI
TVKSDNYGSSYAESVKGRFAISRDNSKNTLYLQMNSLRAEDTAVYYCSRELG
QVLVFWGQGTLVTVSS
> hu71H2 (SEQ ID NO: 154) 
EVQLVESGGGVVQPGRSLRLSCATSGFTFSNYRMHWLRQAPGKGLEWVAVIT
VKSDNYGSSYAESVKGRFAISRDNSKNTLYLQMNSLRAEDTAVYYCSRELGQ
VLVFWGQGTLVTVSS
> hu71H3 (SEQ ID NO: 155) 
EVQLVESGGGVVQPGRSLRLSCATSGFTFSNYRMHWVRQAPGKGLEWVAVIT
VKSDNYGSSYAESVKGRFAISREDSKSTLYLQMNSLRAEDTAVYYCSRELGQV
LVFWGQGTLVTVSS
> hu71H4 (SEQ ID NO: 156) 
EVQLVESGGGVVQPGRSLRLSCATSGFTFSNYRMHWLRQAPGKRLEWVAVIT
VKSDNYGSSYAESVKGRFAISREDSKSTLYLQMNSLRAEDTAVYYCSRELGQV
LVFWGQGTLVTVSS
> hu71L1 (SEQ ID NO: 157) 
DIVMTQTPLSLPVTPGEPASISCRSSQSLVHSYGNTYLHWYLQKPGQSPQLLIY
KVSNRFSGVPDRFSGSGSGTDFTLKISRVEAEDVGVYYCSQTTHVPWTFGGG
TKVEIK
> hu71L2 (SEQ ID NO: 158)

DVVVTQTPLSLPVTPGEPASISCRSSQSLVHSYGNTYLHWYLQKPGQSPQLLIY
KVSNRFSGVPDRFSGSGSGTDFTLKISRVEAEDVGVYYCSQTTHVPWTFGGG
TKVEIK.

The heavy chain variable region and the light chain variable region of each of the groups described above were combined with the constant regions set forth in SEQ ID NO: 97 and SEQ ID NO: 98 to obtain complete humanized antibodies. hu2H1L1 represents a humanized antibody constructed by combining the humanized heavy chain variable region of antibody 2, H1 (hu2H1), with the constant region SEQ ID NO: 97 and combining the humanized light chain variable region L1 (hu2L1) with the constant region SEQ ID NO: 98. This applies to other humanized antibodies.

### 3-12. Humanization of antibody 73

For the humanized antibody of murine antibody 73, the FR1, FR2, and FR3 of IGHV1-69*02 and the FR4 of IGHJ6*01 were selected as a heavy chain framework region template, and the FR1, FR2, and FR3 of IGKV1-27*01 and the FR4 of IGKJ4*01 were selected as a light chain framework region template. Optionally, the amino acid residue(s) at position(s) 27, 28, 39, 43, 69, and/or 93 in the heavy chain variable region of the humanized antibody was/were substituted; and/or the amino acid residue(s) at position(s) 36, 41, 42, 43, 44, 45, and/or 71 in the light chain variable region of the humanized antibody was/were substituted.

**Table 15-11. Mutations for humanized antibody 73**

| 73.VL | | 73.VH | |
|---|---|---|---|
| L1 | Y36F, F71Y | H1 | G27Y, T28A, A93V |
| L2 | Y36F, P44V, K45R, F71Y | H2 | G27Y, T28A, I69L, A93V |
| L3 | Y36F, G41D, K42G, P44V, K45R, F71Y | H3 | G27Y, T28A, Q39E, Q43K, I69L, A93V |
| L4 | Y36F, G41D, K42G, V43T, P44V, K45R, F71Y | | |

> hu73H1 (SEQ ID NO: 277) 
EVQLVQSGAEVKKPGSSVKVSCKASGYAFSTSWMNWVRQAPGQGLEWMGR
IYPGDGDTKYNGKFQGRVTITADKSTSTAYMELSSLRSEDTAVYYCVRSIYYLY
YFDYWGQGTTVTVSS
> hu73H2 (SEQ ID NO: 278) 
EVQLVQSGAEVKKPGSSVKVSCKASGYAFSTSWMNWVRQAPGQGLEWMGR
IYPGDGDTKYNGKFQGRVTLTADKSTSTAYMELSSLRSEDTAVYYCVRSIYYL
YYFDYWGQGTTVTVSS
> hu73H3 (SEQ ID NO: 279) 
EVQLVQSGAEVKKPGSSVKVSCKASGYAFSTSWMNWVREAPGKGLEWMGRI
YPGDGDTKYNGKFQGRVTLTADKSTSTAYMELSSLRSEDTAVYYCVRSIYYLY
YFDYWGQGTTVTVSS
> hu73L1 (SEQ ID NO: 280) 
DIQMTQSPSSLSASVGDRVTITCSTSQDIYNNLSWFQQKPGKVPKLLIYYTSSL
HSGVPSRFSGSGSGTDYTLTISSLQPEDVATYYCQQYNNLPFTFGGGTKVEIK
> hu73L2 (SEQ ID NO: 281) 
DIQMTQSPSSLSASVGDRVTITCSTSQDIYNNLSWFQQKPGKVVRLLIYYTSSL
HSGVPSRFSGSGSGTDYTLTISSLQPEDVATYYCQQYNNLPFTFGGGTKVEIK
> hu73L3 (SEQ ID NO: 282) 
DIQMTQSPSSLSASVGDRVTITCSTSQDIYNNLSWFQQKPDGVVRLLIYYTSSL
HSGVPSRFSGSGSGTDYTLTISSLQPEDVATYYCQQYNNLPFTFGGGTKVEIK
> hu73L4 (SEQ ID NO: 283)

DIQMTQSPSSLSASVGDRVTITCSTSQDIYNNLSWFQQKPDGTVRLLIYYTSSL
HSGVPSRFSGSGSGTDYTLTISSLQPEDVATYYCQQYNNLPFTFGGGTKVEIK.

### Example 4. Preparation of Anti-PSMA-CD3 Bispecific Antibodies

The CD3-binding molecule of the present disclosure can be derived from any suitable antibody. Specifically, S107E was used in the examples of the present disclosure.

**Table 16. The CDRs of S107E**

| No. | Heavy chain | | Light chain | |
|---|---|---|---|---|
| S107E | HCDR1 | KYAMN (SEQ ID NO: 160) | LCDR1 | GSSTGAVTSGNYPN (SEQ ID NO: 163) |
| | HCDR2 | RIRSKYNNYATYYADSVKD (SEQ ID NO: 161) | LCDR2 | GTKFLAP (SEQ ID NO: 164) |
| | HCDR3 | HGNFGNEYISYWAY (SEQ ID NO: 162) | LCDR3 | VLWYSNRWV (SEQ ID NO: 165) |

The variable region sequences of S107E are as follows:
> S107E-VH (SEQ ID NO: 166) 
EVQLVESGGGLVQPGGSLKLSCAASGFTFNKYAMNWVRQAPGKGLEWVARI
RSKYNNYATYYADSVKDRFTISRDDSKNTAYLQMNNLKTEDTAVYYCVRHGN
FGNEYISYWAYWGQGTLVTVSS
> S 107E-VL (SEQ ID NO: 167)

QTVVTQEPSLTVSPGGTVTLTCGSSTGAVTSGNYPNWVQQKPGQAPRGLIGGT
KFLAPGTPARFSGSLLGGKAALTLSGVQPEDEAEYYCVLWYSNRWVFGGGTK
LTVL.

The present disclosure comprises the following molecular structures.

Format 1 is an asymmetrically structured molecule, comprising
chain 1: VH (anti-PSMA)-IgG1 (CH1)-IgG₁Fc (Knob, L234A/L235A/S354C/T366W);
chain 2: VL (anti-PSMA)-CL;
chain 3 (Ob-hole): VH (S107E)-linker a-Obscurin-IgG₁Fc (Hole, L234A/L235A/Y349C/T366S/L368A/Y407V); and
chain 4 (VL-Titin): VL (S107E)-linker a-Titin.

It is shown schematically in FIG. 1A (Ob represents Obscurin; this applies hereinafter).
> IgG₁ (CH1) (SEQ ID NO: 168) 
ASTKGPSVFPLAPSSKSTSGGTAALGCLVKDYFPEPVTVSWNSGALTSGVHTFPAVL
QSSGLYSLSSVVTVPSSSLGTQTYICNVNHKPSNTKVDKKVEPKSC
> CL (SEQ ID NO: 98)
> IgG₁Fc (Knob, L234A/L235A/S354C/T366W) (SEQ ID NO: 169) 
DKTHTCPPCPAPEAAGGPSVFLFPPKPKDTLMISRTPEVTCVVVDVSHEDPEVKF
NWYVDGVEVHNAKTKPREEQYNSTYRVVSVLTVLHQDWLNGKEYKCKVSNKALPA
PIEKTISKAKGQPREPQVYTLPPCRDELTKNQVSLWCLVKGFYPSDIAVEWESNGQ
PENNYKTTPPVLDSDGSFFLYSKLTVDKSRWQQGNVFSCSVMHEALHNHYTQKSL
SLSPGK
> IgG₁Fc (Hole, L234A/L235A/Y349C/T366S/L368A/Y407V) (SEQ ID NO: 170) 
DKTHTCPPCPAPEAAGGPSVFLFPPKPKDTLMISRTPEVTCVVVDVSHEDPEVKF
NWYVDGVEVHNAKTKPREEQYNSTYRVVSVLTVLHQDWLNGKEYKCKVSNKALPA
PIEKTISKAKGQPREPQVCTLPPSRDELTKNQVSLSCAVKGFYPSDIAVEWESNGQP
ENNYKTTPPVLDSDGSFFLVSKLTVDKSRWQQGNVFSCSVMHEALHNHYTQKSLS
LSPGK
> Ob-hole (SEQ ID NO: 171) 
EVQLVESGGGLVQPGGSLKLSCAASGFTFNKYAMNWVRQAPGKGLEWVARI
RSKYNNYATYYADSVKDRFTISRDDSKNTAYLQMNNLKTEDTAVYYCVRHGN
FGNEYISYWAYWGQGTLVTVSSGGGGSSGAPRFLTRPKASVVSVGKDATLSC
QIVGNPFPQVSWEKDKQPVTAGVRFRLAQDGDLYRLKILDLQLSDSGQYVCR
ARNAHGEAFACLGLQVDAEADKTHTCPPCPAPEAAGGPSVFLFPPKPKDTLMIS
RTPEVTCVVVDVSHEDPEVKFNWYVDGVEVHNAKTKPREEQYNSTYRVVSVLTVL
HQDWLNGKEYKCKVSNKALPAPIEKTISKAKGQPREPQVCTLPPSRDELTKNQVSL
SCAVKGFYPSDIAVEWESNGQPENNYKTTPPVLDSDGSFFLVSKLTVDKSRWQQG
NVFSCSVMHEALHNHYTQKSLSLSPGK
> VL-Titin: VL (S107E)-linker a-Titin (SEQ ID NO: 172) 
QTVVTQEPSLTVSPGGTVTLTCGSSTGAVTSGNYPNWVQQKPGQAPRGLIGGT
KFLAPGTPARFSGSLLGGKAALTLSGVQPEDEAEYYCVLWYSNRWVFGGGTK
LTVLGGGGSGIPPKIECLPIDISIDEGKVLTVASAFTGEPTPEVTWSTGGRKIHSQ
EQGRFHIENTDDSTTLTIKDVQKQDGGLYTLTLRNEFGSDSATVNIHIRSI
> linker a (SEQ ID NO: 173) GGGGS

Note: The CDRs of the CD3-binding domain obtained based on the Kabat numbering scheme are single-underlined, the Titin or Obscurin sequence is double-underlined, and the constant regions are in italics. This applies hereinafter.

Format 2 is an asymmetrically structured molecule, comprising
chain 1: VH (anti-PSMA)-IgG1 (CH1)-IgG₁Fc (Knob, L234A/L235A/S354C/T366W);
chain 2 (two chains): VL (anti-PSMA)-CL;
chain 3: VH (anti-PSMA)-IgG1 (CH1)-VH (S107E)-linker a-Titin-IgG₁Fc (Hole, L234A/L235A/Y349C/T366S/L368A/Y407V); and
chain 4 (VL-Ob): VL (S107E)-linker a-Ob. It is shown schematically in FIG. 1B.
> VL-Ob: VL (S107E)-linker a-Ob (SEQ ID NO: 174)

QTVVTQEPSLTVSPGGTVTLTCGSSTGAVTSGNYPNWVQQKPGQAPRGLIGGT
KFLAPGTPARFSGSLLGGKAALTLSGVQPEDEAEYYCVLWYSNRWVFGGGTK
LTVLGGGGSSGAPRFLTRPKASVVSVGKDATLSCQIVGNPFPQVSWEKDKQPV
TAGVRFRLAQDGDLYRLKILDLQLSDSGQYVCRARNAHGEAFACLGLQVDA
EA.

Format 3 is a symmetrically structured molecule, comprising
chain 1 (two chains): VH (anti-PSMA)-IgG1 (CH1)-VH (S107E)-linker a-Titin-IgG₁ (AA) Fc;
chain 2 (two chains): VL (anti-PSMA)-CL; and
chain 4 (two chains) being VL-Ob: VL (S107E)-linker a-Ob. It is shown schematically in FIG. 1C.
> IgG₁ (AA) Fc (SEQ ID NO: 175)

DKTHTCPPCPAPEAAGGPSVFLFPPKPKDTLMISRTPEVTCVVVDVSHEDPEVKF
NWYVDGVEVHNAKTKPREEQYNSTYRVVSVLTVLHQDWLNGKEYKCKVSNKALPA

PIEKTISKAKGQPREPQVYTLPPSRDELTKNQVSLTCLVKGFYPSDIAVEWESNGQP
ENNYKTTPPVLDSDGSFFLYSKLTVDKSRWQQGNVFSCSVMHEALHNHYTQKSLS
LSPGK.

Format 4 is a symmetrically structured molecule, comprising two identical heavy chains (chain 1) and two identical light chains (chain 2). The heavy chain is: VH (anti-PSMA)-IgG₁(CH₁)-VH (S107E)-linker b-VL (S107E)-linker c-IgG₁ (AA) Fc; the light chain is VL (anti-PSMA)-CL. It is shown schematically in FIG. 1D.
> linker b (SEQ ID NO: 176)
   GGGGSGGGGSGGGGS
   > linker c
   GGG.
   Format 5 is an asymmetrically structured molecule, comprising
      chain 1: VH (anti-PSMA)-linker b-VL (anti-PSMA)-linker d-VH (S107E)-linker b-VL (S107E)-linker c-IgG₁Fc (Hole, L234A/L235A/Y349C/T366S/L368A/Y407V); and
      chain 3 (Fc (Knob)): IgG₁Fc (Knob, L234A/L235A/S354C/T366W). It is shown schematically in FIG. 1E.
> IgG₁Fc (Hole, Y349C/T366S/L368A/Y407V) (SEQ ID NO: 170)
> IgG₁Fc (Knob, L234A/L235A/S354C/T366W) (SEQ ID NO: 169)
> linker d (SEQ ID NO: 178)
   SGGGGS
> linker c: GGG.

Based on 2, 13, 15, 19, 27, 31, 43, 46, 73, and the humanized antibody amino acid sequences, the following bispecific antibodies were constructed.

**Table 17. The bispecific antibodies of the present disclosure**

| No. | Chain 1 | Chain 2 | Chain 3 | Chain 4 |
|---|---|---|---|---|
| 19-F1 | hu19H2-F1-1 (SEQ ID NO: 193) | hu 19L2-CL (SEQ ID NO: 194) | Ob-hole (SEQ ID NO: 171) | VL-Titin (SEQ ID NO: 172) |
| 31-F1 | hu31H1-F1-1 (SEQ ID NO: 201) | hu31L1-CL (SEQ ID NO: 202) | Ob-hole (SEQ ID NO: 171) | VL-Titin (SEQ ID NO: 172) |
| 46-F1 | hu46H2-F1-1 (SEQ ID NO: 206) | hu46L2-CL (SEQ ID NO: 207) | Ob-hole (SEQ ID NO: 171) | VL-Titin (SEQ ID NO: 172) |
| 73-F1 | 73VH-F1-1 (SEQ ID NO: 284) | 73VL-CL (SEQ ID NO: 285) | Ob-hole (SEQ ID NO: 171) | VL-Titin (SEQ ID NO: 172) |
| 2-F2 | hu2H1-F2-1 (SEQ ID NO: 180) | hu2L1-CL (SEQ ID NO: 181) | hu2H 1-F2-3 (SEQ ID NO: 182) | VL-Ob (SEQ ID NO: 174) |
| 15-F2 | hu15H4-F2-1 (SEQ ID NO: 187) | hu 15L2-CL (SEQ ID NO: 188) | hu 15H4-F2-3 (SEQ ID NO: 189) | VL-Ob (SEQ ID NO: 174) |
| 19-F2 | hu 19H2-F2-1 (SEQ ID NO: 193) | hu 19L2-CL (SEQ ID NO: 194) | hu 19H2-F2-3 (SEQ ID NO: 195) | VL-Ob (SEQ ID NO: 174) |
| 46-F2 | hu46H2-F2-1 | hu46L2-CL (SEQ ID NO: 207) | hu46H2-F2-3 (SEQ ID NO: 208) | VL-Ob (SEQ ID NO: 174) |
| 2-F3 | hu2H1-F3-1 (SEQ ID NO: 183) | hu2L1-CL (SEQ ID NO: 181) | | VL-Ob (SEQ ID NO: 174) |
| 13-F3 | hu13H1-F3-1 (SEQ ID NO: 185) | hu13L1-CL (SEQ ID NO: 186) | | VL-Ob (SEQ ID NO: 174) |
| 19-F3 | hu19H2-F3-1 (SEQ ID NO: 196) | hu 19L2-CL (SEQ ID NO: 194) | | VL-Ob (SEQ ID NO: 174) |
| 43-F3 | hu43H3-F3-1 (SEQ ID NO: 203) | hu43L2-CL (SEQ ID NO: 204) | | VL-Ob (SEQ ID NO: 174) |
| 73-F3 | 73VH-F3-1 (SEQ ID NO: 286) | 73VL-CL (SEQ ID NO: 285) | | VL-Ob (SEQ ID NO: 174) |
| 2-F4 | hu2H1-F4-1 (SEQ ID NO: 184) | hu2L1-CL (SEQ ID NO: 181) | | |
| 15-F4 | hu15H4-F4-1 (SEQ ID NO: 190) | hu 15L2-CL (SEQ ID NO: 188) | | |
| 19-F4 | hu 19H2-F4-1 (SEQ ID NO: 197) | hu 19L2-CL (SEQ ID NO: 194) | | |
| 27-F4 | hu27H1-F4-1 (SEQ ID NO: 199) | hu27L6-CL (SEQ ID NO: 200) | | |
| 43-F4 | hu43H3-F4-1 (SEQ ID NO: 205) | hu43L2-CL (SEQ ID NO: 204) | | |
| 46-F4 | hu46H2-F4-1 (SEQ ID NO: 209) | hu46L2-CL (SEQ ID NO: 207) | | |
| 15-F5-1 | hu15H4L2-F5-1 (SEQ ID NO: 191) | | Fc (Knob) (SEQ ID NO: 169) | |
| 15-F5-2 | hu15H6L3-F5-1 (SEQ ID NO: 192) | | Fc (Knob) (SEQ ID NO: 169) | |
| 19-F5 | hu19H2L2-F5-1 (SEQ ID NO: 198) | | Fc (Knob) (SEQ ID NO: 169) | |

2-F2 means that the molecule uses the variable regions of antibody 2 and its humanized antibody as PSMA-binding domains and uses Format 2 as the molecular structure. hu2H1-F2-1 means that the chain uses hu2H1 as the heavy chain variable region and uses Format 2 as the molecular structure. This applies to other chains. The specific amino acid sequences are as follows:
> hu2H1-F2-1 (SEQ ID NO: 180) 
EVQLVESGGGLVQPGGSLRLSCAASGFTFSDYYMAWVRQAPGKGLEWVANI
NFDGSSTYYLDSLKSRFTISRDNAKNSLYLQMNSLRAEDTAVYYCARDLDGY
YGYFDVWGQGTTVTVSSASTKGPSVFPLAPSSKSTSGGTAALGCLVKDYFPEP
 
VTVSWNSGALTSGVHTFPAVLQSSGLYSLSSVVTVPSSSLGTQTYICNVNHKPS
NTKVDKKVEPKSCDKTHTCPPCPAPEAAGGPSVFLFPPKPKDTLMISRTPEVTC
VVVDVSHEDPEVKFNWYVDGVEVHNAKTKPREEQYNSTYRVVSVLTVLHQ
DWLNGKEYKCKVSNKALPAPIEKTISKAKGQPREPQVYTLPPCRDELTKNQVS
LWCLVKGFYPSDIAVEWESNGQPENNYKTTPPVLDSDGSFFLYSKLTVDKSRW
QQGNVFSCSVMHEALHNHYTQKSLSLSPGK
> hu2L1-CL (SEQ ID NO: 181) 
EIVLTQSPDFQSVTPKEKVTITCSASSSVSHMYWYQQKPDQSPKLLIYRTSNLA
SGVPSRFSGSGSGTDFTLTINSLEAEDAATYYCQQYHSYPLMFGQGTKLEIKRT
VAAPSVFIFPPSDEQLKSGTASVVCLLNNFYPREAKVQWKVDNALQSGNSQES
VTEQDSKDSTYSLSSTLTLSKADYEKHKVYACEVTHQGLSSPVTKSFNRGEC
> hu2H1-F2-3 (SEQ ID NO: 182) 
EVQLVESGGGLVQPGGSLRLSCAASGFTFSDYYMAWVRQAPGKGLEWVANI
NFDGSSTYYLDSLKSRFTISRDNAKNSLYLQMNSLRAEDTAVYYCARDLDGY
YGYFDVWGQGTTVTVSSASTKGPSVFPLAPSSKSTSGGTAALGCLVKDYFPEP
VTVSWNSGALTSGVHTFPAVLQSSGLYSLSSVVTVPSSSLGTQTYICNVNHKPS
NTKVDKKVEPKSCEVQLVESGGGLVQPGGSLKLSCAASGFTFNKYAMNWVR
QAPGKGLEWVARIRSKYNNYATYYADSVKDRFTISRDDSKNTAYLQMNNLKT
EDTAVYYCVRHGNFGNEYISYWAYWGQGTLVTVSSGGGGSGIPPKIECLPIDIS
IDEGKVLTVASAFTGEPTPEVTWSTGGRKIHSQEQGRFHIENTDDSTTLTIKDV
QKQDGGLYTLTLRNEFGSDSATVNIHIRSIDKTHTCPPCPAPEAAGGPSVFLFPP
KPKDTLMISRTPEVTCVVVDVSHEDPEVKFNWYVDGVEVHNAKTKPREEQY
NSTYRVVSVLTVLHQDWLNGKEYKCKVSNKALPAPIEKTISKAKGQPREPQV
CTLPPSRDELTKNQVSLSCAVKGFYPSDIAVEWESNGQPENNYKTTPPVLDSD
GSFFLVSKLTVDKSRWQQGNVFSCSVMHEALHNHYTQKSLSLSPGK
> hu2H1-F3-1 (SEQ ID NO: 183) 
EVQLVESGGGLVQPGGSLRLSCAASGFTFSDYYMAWVRQAPGKGLEWVANI
NFDGSSTYYLDSLKSRFTISRDNAKNSLYLQMNSLRAEDTAVYYCARDLDGY
YGYFDVWGQGTTVTVSSASTKGPSVFPLAPSSKSTSGGTAALGCLVKDYFPEP
VTVSWNSGALTSGVHTFPAVLQSSGLYSLSSVVTVPSSSLGTQTYICNVNHKPS
NTKVDKKVEPKSCEVQLVESGGGLVQPGGSLKLSCAASGFTFNKYAMNWVR
QAPGKGLEWVARIRSKYNNYATYYADSVKDRFTISRDDSKNTAYLQMNNLKT
EDTAVYYCVRHGNFGNEYISYWAYWGQGTLVTVSSGGGGSGIPPKIECLPIDIS
IDEGKVLTVASAFTGEPTPEVTWSTGGRKIHSQEQGRFHIENTDDSTTLTIKDV
QKQDGGLYTLTLRNEFGSDSATVNIHIRSIDKTHTCPPCPAPEAAGGPSVFLFPP
KPKDTLMISRTPEVTCVVVDVSHEDPEVKFNWYVDGVEVHNAKTKPREEQY
NSTYRVVSVLTVLHQDWLNGKEYKCKVSNKALPAPIEKTISKAKGQPREPQV
YTLPPSRDELTKNQVSLTCLVKGFYPSDIAVEWESNGQPENNYKTTPPVLDSD
GSFFLYSKLTVDKSRWQQGNVFSCSVMHEALHNHYTQKSLSLSPGK
> hu2H1-F4-1 (SEQ ID NO: 184) 
EVQLVESGGGLVQPGGSLRLSCAASGFTFSDYYMAWVRQAPGKGLEWVANI
NFDGSSTYYLDSLKSRFTISRDNAKNSLYLQMNSLRAEDTAVYYCARDLDGY
YGYFDVWGQGTTVTVSSASTKGPSVFPLAPSSKSTSGGTAALGCLVKDYFPEP
VTVSWNSGALTSGVHTFPAVLQSSGLYSLSSVVTVPSSSLGTQTYICNVNHKPS
NTKVDKKVEPKSCEVQLVESGGGLVQPGGSLKLSCAASGFTFNKYAMNWVR
QAPGKGLEWVARIRSKYNNYATYYADSVKDRFTISRDDSKNTAYLQMNNLKT
EDTAVYYCVRHGNFGNEYISYWAYWGQGTLVTVSSGGGGSGGGGSGGGGSQ
TVVTQEPSLTVSPGGTVTLTCGSSTGAVTSGNYPNWVQQKPGQAPRGLIGGTK
FLAPGTPARFSGSLLGGKAALTLSGVQPEDEAEYYCVLWYSNRWVFGGGTKL
TVLGGGDKTHTCPPCPAPEAAGGPSVFLFPPKPKDTLMISRTPEVTCVVVDVS
HEDPEVKFNWYVDGVEVHNAKTKPREEQYNSTYRVVSVLTVLHQDWLNGK
EYKCKVSNKALPAPIEKTISKAKGQPREPQVYTLPPSRDELTKNQVSLTCLVKG
FYPSDIAVEWESNGQPENNYKTTPPVLDSDGSFFLYSKLTVDKSRWQQGNVFS
CSVMHEALHNHYTQKSLSLSPGK
> hu13H1-F3-1 (SEQ ID NO: 185) 
EVTLKESGPVLVKPTETLTLTCTVSGFSLSNYAISWIRQPPGKALEWLAVIWTG
GGTHYNSALKSRLTISKDTSKSQVVLTMTNMDPVDTATYYCARKGNYYGSSL
YYYAMNYWGQGTTVTVSSASTKGPSVFPLAPSSKSTSGGTAALGCLVKDYFP
EPVTVSWNSGALTSGVHTFPAVLQSSGLYSLSSVVTVPSSSLGTQTYICNVNHK
PSNTKVDKKVEPKSCEVQLVESGGGLVQPGGSLKLSCAASGFTFNKYAMNW
VRQAPGKGLEWVARIRSKYNNYATYYADSVKDRFTISRDDSKNTAYLQMNNL
KTEDTAVYYCVRHGNFGNEYISYWAYWGQGTLVTVSSGGGGSGIPPKIECLPI
DISIDEGKVLTVASAFTGEPTPEVTWSTGGRKIHSQEQGRFHIENTDDSTTLTIK
DVQKQDGGLYTLTLRNEFGSDSATVNIHIRSIDKTHTCPPCPAPEAAGGPSVFL
FPPKPKDTLMISRTPEVTCVVVDVSHEDPEVKFNWYVDGVEVHNAKTKPREE
QYNSTYRVVSVLTVLHQDWLNGKEYKCKVSNKALPAPIEKTISKAKGQPREP
QVYTLPPSRDELTKNQVSLTCLVKGFYPSDIAVEWESNGQPENNYKTTPPVLD
SDGSFFLYSKLTVDKSRWQQGNVFSCSVMHEALHNHYTQKSLSLSPGK
> hu13L1-CL (SEQ ID NO: 186) 
DIQMTQSPSSLSASVGDRVTITCRASQDISNYLNWYQQKPGKAPKLLIYYTSR
LQSGVPSRFSGSGSGTDFTLTISSLQPEDFATYYCQQGDTLPYTFGGGTKVEIK
RTVAAPSVFIFPPSDEQLKSGTASVVCLLNNFYPREAKVQWKVDNALQSGNSQ
ESVTEQDSKDSTYSLSSTLTLSKADYEKHKVYACEVTHQGLSSPVTKSFNRGE
C
> hu15H4-F2-1 (SEQ ID NO: 187) 
EFQLVQSGAEVKKPGASVKVSCKASGYSFTDHNMNWVRQAPGQRLEWMGL
INPNYGTADYNQKFKGRVTLTVDQSATTAYMELSSLRSEDTAVYYCARGSSLF
AYWGQGTLVTVSSASTKGPSVFPLAPSSKSTSGGTAALGCLVKDYFPEPVTVS
WNSGALTSGVHTFPAVLQSSGLYSLSSVVTVPSSSLGTQTYICNVNHKPSNTK
VDKKVEPKSCDKTHTCPPCPAPEAAGGPSVFLFPPKPKDTLMISRTPEVTCVV
 
VDVSHEDPEVKFNWYVDGVEVHNAKTKPREEQYNSTYRVVSVLTVLHQDW
LNGKEYKCKVSNKALPAPIEKTISKAKGQPREPQVYTLPPCRDELTKNQVSLW
CLVKGFYPSDIAVEWESNGQPENNYKTTPPVLDSDGSFFLYSKLTVDKSRWQQ
GNVFSCSVMHEALHNHYTQKSLSLSPGK
> hu15L2-CL (SEQ ID NO: 188) 
DIQMTQSPSSLSASVGDRVTITCKASQGVDTAVAWYQQKPGKSPKLLIYWAST
RHTGVPDRFSGSGSGTDFTLTISSLQPEDVATYYCQQYSRYPLTFGQGTKLEIK
RTVAAPSVFIFPPSDEQLKSGTASVVCLLNNFYPREAKVQWKVDNALQSGNSQ
ESVTEQDSKDSTYSLSSTLTLSKADYEKHKVYACEVTHQGLSSPVTKSFNRGE
C
> hu15H4-F2-3 (SEQ ID NO: 189) 
EFQLVQSGAEVKKPGASVKVSCKASGYSFTDHNMNWVRQAPGQRLEWMGL
INPNYGTADYNQKFKGRVTLTVDQSATTAYMELSSLRSEDTAVYYCARGSSLF
AYWGQGTLVTVSSASTKGPSVFPLAPSSKSTSGGTAALGCLVKDYFPEPVTVS
WNSGALTSGVHTFPAVLQSSGLYSLSSVVTVPSSSLGTQTYICNVNHKPSNTK
VDKKVEPKSCEVQLVESGGGLVQPGGSLKLSCAASGFTFNKYAMNWVRQAP
GKGLEWVARIRSKYNNYATYYADSVKDRFTISRDDSKNTAYLQMNNLKTEDT
AVYYCVRHGNFGNEYISYWAYWGQGTLVTVSSGGGGSGIPPKIECLPIDISIDE
GKVLTVASAFTGEPTPEVTWSTGGRKIHSQEQGRFHIENTDDSTTLTIKDVQKQ
DGGLYTLTLRNEFGSDSATVNIHIRSIDKTHTCPPCPAPEAAGGPSVFLFPPKPK
DTLMISRTPEVTCVVVDVSHEDPEVKFNWYVDGVEVHNAKTKPREEQYNST
YRVVSVLTVLHQDWLNGKEYKCKVSNKALPAPIEKTISKAKGQPREPQVCTL
PPSRDELTKNQVSLSCAVKGFYPSDIAVEWESNGQPENNYKTTPPVLDSDGSFF
LVSKLTVDKSRWQQGNVFSCSVMHEALHNHYTQKSLSLSPGK
> hu15H4-F4-1 (SEQ ID NO: 190) 
EFQLVQSGAEVKKPGASVKVSCKASGYSFTDHNMNWVRQAPGQRLEWMGL
INPNYGTADYNQKFKGRVTLTVDQSATTAYMELSSLRSEDTAVYYCARGSSLF
AYWGQGTLVTVSSASTKGPSVFPLAPSSKSTSGGTAALGCLVKDYFPEPVTVS
WNSGALTSGVHTFPAVLQSSGLYSLSSVVTVPSSSLGTQTYICNVNHKPSNTK
VDKKVEPKSCEVQLVESGGGLVQPGGSLKLSCAASGFTFNKYAMNWVRQAP
GKGLEWVARIRSKYNNYATYYADSVKDRFTISRDDSKNTAYLQMNNLKTEDT
AVYYCVRHGNFGNEYISYWAYWGQGTLVTVSSGGGGSGGGGSGGGGSQTVV
TQEPSLTVSPGGTVTLTCGSSTGAVTSGNYPNWVQQKPGQAPRGLIGGTKFLA
PGTPARFSGSLLGGKAALTLSGVQPEDEAEYYCVLWYSNRWVFGGGTKLTVL
GGGDKTHTCPPCPAPEAAGGPSVFLFPPKPKDTLMISRTPEVTCVVVDVSHED
PEVKFNWYVDGVEVHNAKTKPREEQYNSTYRVVSVLTVLHQDWLNGKEYK
CKVSNKALPAPIEKTISKAKGQPREPQVYTLPPSRDELTKNQVSLTCLVKGFYP
SDIAVEWESNGQPENNYKTTPPVLDSDGSFFLYSKLTVDKSRWQQGNVFSCSV
MHEALHNHYTQKSLSLSPGK
> hu15H4L2-F5-1 (SEQ ID NO: 191) 
EFQLVQSGAEVKKPGASVKVSCKASGYSFTDHNMNWVRQAPGQRLEWMGL
INPNYGTADYNQKFKGRVTLTVDQSATTAYMELSSLRSEDTAVYYCARGSSLF
AYWGQGTLVTVSSGGGGSGGGGSGGGGSDIQMTQSPSSLSASVGDRVTITCK
ASQGVDTAVAWYQQKPGKSPKLLIYWASTRHTGVPDRFSGSGSGTDFTLTISS
LQPEDVATYYCQQYSRYPLTFGQGTKLEIKSGGGGSEVQLVESGGGLVQPGGS
LKLSCAASGFTFNKYAMNWVRQAPGKGLEWVARIRSKYNNYATYYADSVKD
RFTISRDDSKNTAYLQMNNLKTEDTAVYYCVRHGNFGNEYISYWAYWGQGTL
VTVSSGGGGSGGGGSGGGGSQTVVTQEPSLTVSPGGTVTLTCGSSTGAVTSGN
YPNWVQQKPGQAPRGLIGGTKFLAPGTPARFSGSLLGGKAALTLSGVQPEDE
AEYYCVLWYSNRWVFGGGTKLTVLGGGDKTHTCPPCPAPEAAGGPSVFLFPP
KPKDTLMISRTPEVTCVVVDVSHEDPEVKFNWYVDGVEVHNAKTKPREEQY
NSTYRVVSVLTVLHQDWLNGKEYKCKVSNKALPAPIEKTISKAKGQPREPQV
CTLPPSRDELTKNQVSLSCAVKGFYPSDIAVEWESNGQPENNYKTTPPVLDSD
GSFFLVSKLTVDKSRWQQGNVFSCSVMHEALHNHYTQKSLSLSPGK
> hu15H6L3-F5-1 (SEQ ID NO: 192) 
EFQLVQSGAEVKKPGASVKVSCKASGYSFTDHNMNWVRQAPGQCLEWMGL
INPNYGTADYNQKFKGRVTLTVDQSATTAYMELSSLRSEDTAVYYCARGSSLF
AYWGQGTLVTVSSGGGGSGGGGSGGGGSDIQMTQSPSSLSASVGDRVTITCK
ASQGVDTAVAWYQQKPGKSPKLLIYWASTRHTGVPDRFSGSGSGTDFTLTISS
LQPEDVATYYCQQYSRYPLTFGCGTKLEIKSGGGGSEVQLVESGGGLVQPGGS
LKLSCAASGFTFNKYAMNWVRQAPGKGLEWVARIRSKYNNYATYYADSVKD
RFTISRDDSKNTAYLQMNNLKTEDTAVYYCVRHGNFGNEYISYWAYWGQGTL
VTVSSGGGGSGGGGSGGGGSQTVVTQEPSLTVSPGGTVTLTCGSSTGAVTSGN
YPNWVQQKPGQAPRGLIGGTKFLAPGTPARFSGSLLGGKAALTLSGVQPEDE
AEYYCVLWYSNRWVFGGGTKLTVLGGDKTHTCPPCPAPEAAGGPSVFLFPP
KPKDTLMISRTPEVTCVVVDVSHEDPEVKFNWYVDGVEVHNAKTKPREEQY
NSTYRVVSVLTVLHQDWLNGKEYKCKVSNKALPAPIEKTISKAKGQPREPQV
CTLPPSRDELTKNQVSLSCAVKGFYPSDIAVEWESNGQPENNYKTTPPVLDSD
GSFFLVSKLTVDKSRWQQGNVFSCSVMHEALHNHYTQKSLSLSPGK
> hu19H2-F1-1, also referred to as hu19H2-F2-1 (SEQ ID NO: 193) 
EVKLVESGGGLVQPGGSLRLSCAASGFTFSDYYMAWVRQAPGKGLEWVANI
NYDGSSTYYLDSLKSRFTISRDNAKNSLYLQMNSLRAEDTAVYYCARGGDY
WDAMDYWGQGTTVTVSSASTKGPSVFPLAPSSKSTSGGTAALGCLVKDYFPE
PVTVSWNSGALTSGVHTFPAVLQSSGLYSLSSVVTVPSSSLGTQTYICNVNHKP
SNTKVDKKVEPKSCDKTHTCPPCPAPEAAGGPSVFLFPPKPKDTLMISRTPEVT
CVVVDVSHEDPEVKFNWYVDGVEVHNAKTKPREEQYNSTYRVVSVLTVLHQ
DWLNGKEYKCKVSNKALPAPIEKTISKAKGQPREPQVYTLPPCRDELTKNQVS
LWCLVKGFYPSDIAVEWESNGQPENNYKTTPPVLDSDGSFFLYSKLTVDKSRW
QQGNVFSCSVMHEALHNHYTQKSLSLSPGK
> hu19L2-CL (SEQ ID NO: 194) 
DIQMTQSPSSLSASVGDRVTITCRASQDISKYLNWYQQKPGKAPKLLIYYTSSL
HSGVPSRFSGSGSGTDYTLTISSLQPEDFATYYCQQGNRLPRTFGGGTKVEIKR
TVAAPSVFIFPPSDEQLKSGTASVVCLLNNFYPREAKVQWKVDNALQSGNSQE
SVTEQDSKDSTYSLSSTLTLSKADYEKHKVYACEVTHQGLSSPVTKSFNRGEC
> hu19H2-F2-3 (SEQ ID NO: 195) 
EVKLVESGGGLVQPGGSLRLSCAASGFTFSDYYMAWVRQAPGKGLEWVANI
NYDGSSTYYLDSLKSRFTISRDNAKNSLYLQMNSLRAEDTAVYYCARGGDY
WDAMDYWGQGTTVTVSSASTKGPSVFPLAPSSKSTSGGTAALGCLVKDYFPE
PVTVSWNSGALTSGVHTFPAVLQSSGLYSLSSVVTVPSSSLGTQTYICNVNHKP
SNTKVDKKVEPKSCEVQLVESGGGLVQPGGSLKLSCAASGFTFNKYAMNWV
RQAPGKGLEWVARIRSKYNNYATYYADSVKDRFTISRDDSKNTAYLQMNNLK
TEDTAVYYCVRHGNFGNEYISYWAYWGQGTLVTVSSGGGGSGIPPKIECLPIDI
SIDEGKVLTVASAFTGEPTPEVTWSTGGRKIHSQEQGRFHIENTDDSTTLTIKD
VQKQDGGLYTLTLRNEFGSDSATVNIHIRSIDKTHTCPPCPAPEAAGGPSVFLFP
PKPKDTLMISRTPEVTCVVVDVSHEDPEVKFNWYVDGVEVHNAKTKPREEQ
YNSTYRVVSVLTVLHQDWLNGKEYKCKVSNKALPAPIEKTISKAKGQPREPQ
VCTLPPSRDELTKNQVSLSCAVKGFYPSDIAVEWESNGQPENNYKTTPPVLDS
DGSFFLVSKLTVDKSRWQQGNVFSCSVMHEALHNHYTQKSLSLSPGK
> hu19H2-F3-1 (SEQ ID NO: 196) 
EVKLVESGGGLVQPGGSLRLSCAASGFTFSDYYMAWVRQAPGKGLEWVANI
NYDGSSTYYLDSLKSRFTISRDNAKNSLYLQMNSLRAEDTAVYYCARGGDY
WDAMDYWGQGTTVTVSSASTKGPSVFPLAPSSKSTSGGTAALGCLVKDYFPE
PVTVSWNSGALTSGVHTFPAVLQSSGLYSLSSVVTVPSSSLGTQTYICNVNHKP
SNTKVDKKVEPKSCEVQLVESGGGLVQPGGSLKLSCAASGFTFNKYAMNWV
RQAPGKGLEWVARIRSKYNNYATYYADSVKDRFTISRDDSKNTAYLQMNNLK
TEDTAVYYCVRHGNFGNEYISYWAYWGQGTLVTVSSGGGGSGIPPKIECLPIDI
SIDEGKVLTVASAFTGEPTPEVTWSTGGRKIHSQEQGRFHIENTDDSTTLTIKD
VQKQDGGLYTLTLRNEFGSDSATVNIHIRSIDKTHTCPPCPAPEAAGGPSVFLFP
PKPKDTLMISRTPEVTCVVVDVSHEDPEVKFNWYVDGVEVHNAKTKPREEQ
YNSTYRVVSVLTVLHQDWLNGKEYKCKVSNKALPAPIEKTISKAKGQPREPQ
VYTLPPSRDELTKNQVSLTCLVKGFYPSDIAVEWESNGQPENNYKTTPPVLDS
DGSFFLYSKLTVDKSRWQQGNVFSCSVMHEALHNHYTQKSLSLSPGK
> hu19H2-F4-1 (SEQ ID NO: 197) 
EVKLVESGGGLVQPGGSLRLSCAASGFTFSDYYMAWVRQAPGKGLEWVANI
NYDGSSTYYLDSLKSRFTISRDNAKNSLYLQMNSLRAEDTAVYYCARGGDY
WDAMDYWGQGTTVTVSSASTKGPSVFPLAPSSKSTSGGTAALGCLVKDYFPE
PVTVSWNSGALTSGVHTFPAVLQSSGLYSLSSVVTVPSSSLGTQTYICNVNHKP
SNTKVDKKVEPKSCEVQLVESGGGLVQPGGSLKLSCAASGFTFNKYAMNWV
RQAPGKGLEWVARIRSKYNNYATYYADSVKDRFTISRDDSKNTAYLQMNNLK
TEDTAVYYCVRHGNFGNEYISYWAYWGQGTLVTVSSGGGGSGGGGSGGGGS
 
QTVVTQEPSLTVSPGGTVTLTCGSSTGAVTSGNYPNWVQQKPGQAPRGLIGGT
KFLAPGTPARFSGSLLGGKAALTLSGVQPEDEAEYYCVLWYSNRWVFGGGTK
LTVLGGGDKTHTCPPCPAPEAAGGPSVFLFPPKPKDTLMISRTPEVTCVVVDV
SHEDPEVKFNWYVDGVEVHNAKTKPREEQYNSTYRVVSVLTVLHQDWLNG
KEYKCKVSNKALPAPIEKTISKAKGQPREPQVYTLPPSRDELTKNQVSLTCLVK
GFYPSDIAVEWESNGQPENNYKTTPPVLDSDGSFFLYSKLTVDKSRWQQGNVF
SCSVMHEALHNHYTQKSLSLSPGK
> hu19H2L2-F5-1 (SEQ ID NO: 198)
EVKLVESGGGLVQPGGSLRLSCAASGFTFSDYYMAWVRQAPGKGLEWVANI
NYDGSSTYYLDSLKSRFTISRDNAKNSLYLQMNSLRAEDTAVYYCARGGDY
WDAMDYWGQGTTVTVSSGGGGSGGGGSGGGGSDIQMTQSPSSLSASVGDR
VTITCRASQDISKYLNWYQQKPGKAPKLLIYYTSSLHSGVPSRFSGSGSGTDY
TLTISSLQPEDFATYYCQQGNRLPRTFGGGTKVEIKSGGGGSEVQLVESGGGLV
QPGGSLKLSCAASGFTFNKYAMNWVRQAPGKGLEWVARIRSKYNNYATYYA
DSVKDRFTISRDDSKNTAYLQMNNLKTEDTAVYYCVRHGNFGNEYISYWAY
WGQGTLVTVSSGGGGSGGGGSGGGGSQTVVTQEPSLTVSPGGTVTLTCGSST
GAVTSGNYPNWVQQKPGQAPRGLIGGTKFLAPGTPARFSGSLLGGKAALTLS
GVQPEDEAEYYCVLWYSNRWVFGGGTKLTVLGGGDKTHTCPPCPAPEAAGG
PSVFLFPPKPKDTLMISRTPEVTCVVVDVSHEDPEVKFNWYVDGVEVHNAKT
KPREEQYNSTYRVVSVLTVLHQDWLNGKEYKCKVSNKALPAPIEKTISKAKG
QPREPQVCTLPPSRDELTKNQVSLSCAVKGFYPSDIAVEWESNGQPENNYKTT
PPVLDSDGSFFLVSKLTVDKSRWQQGNVFSCSVMHEALHNHYTQKSLSLSPG
K
> hu27H1-F4-1 (SEQ ID NO: 199) 
EVQLVESGGGLVQPGGSLRLSCAASGFTFSDYYMAWVRQAPGKGLEWVANI
NYDGSSTYYLDSLKSRFTISRDNAKNSLYLQMNSLRAEDTAVYYCARDLDGY
YGYFDVWGQGTTVTVSSASTKGPSVFPLAPSSKSTSGGTAALGCLVKDYFPEP
VTVSWNSGALTSGVHTFPAVLQSSGLYSLSSVVTVPSSSLGTQTYICNVNHKPS
NTKVDKKVEPKSCEVQLVESGGGLVQPGGSLKLSCAASGFTFNKYAMNWVR
QAPGKGLEWVARIRSKYNNYATYYADSVKDRFTISRDDSKNTAYLQMNNLKT
EDTAVYYCVRHGNFGNEYISYWAYWGQGTLVTVSSGGGGSGGGGSGGGGSQ
TVVTQEPSLTVSPGGTVTLTCGSSTGAVTSGNYPNWVQQKPGQAPRGLIGGTK
FLAPGTPARFSGSLLGGKAALTLSGVQPEDEAEYYCVLWYSNRWVFGGGTKL
TVLGGGDKTHTCPPCPAPEAAGGPSVFLFPPKPKDTLMISRTPEVTCVVVDVS
HEDPEVKFNWYVDGVEVHNAKTKPREEQYNSTYRVVSVLTVLHQDWLNGK
EYKCKVSNKALPAPIEKTISKAKGQPREPQVYTLPPSRDELTKNQVSLTCLVKG
FYPSDIAVEWESNGQPENNYKTTPPVLDSDGSFFLYSKLTVDKSRWQQGNVFS
CSVMHEALHNHYTQKSLSLSPGK
> hu27L6-CL (SEQ ID NO: 200) 
QIVLTQSPDFQSVTPKEKVTIQCSASSSVSYMYWYQQKPDQSPKPWIYRTSNL
ASGVPSRFSGSGSGTDYTLTINSLEAEDAATYYCQQYHSYPLTFGQGTKLEIKR
TVAAPSVFIFPPSDEQLKSGTASVVCLLNNFYPREAKVQWKVDNALQSGNSQE
SVTEQDSKDSTYSLSSTLTLSKADYEKHKVYACEVTHQGLSSPVTKSFNRGEC
> hu31H1-F1-1 (SEQ ID NO: 201) 
EVQLVESGGGLVQPGGSLRLSCAASGFTFSDYYMAWVRQAPGKGLEWVASIN
YDGSSTYYLDSLKSRFTISRDNAKNSLYLQMNSLRAEDTAVYYCAGSDGYWA
WFAYWGQGTLVTVSSASTKGPSVFPLAPSSKSTSGGTAALGCLVKDYFPEPVT
VSWNSGALTSGVHTFPAVLQSSGLYSLSSVVTVPSSSLGTQTYICNVNHKPSNT
KVDKKVEPKSCDKTHTCPPCPAPEAAGGPSVFLFPPKPKDTLMISRTPEVTCV
VVDVSHEDPEVKFNWYVDGVEVHNAKTKPREEQYNSTYRVVSVLTVLHQD
WLNGKEYKCKVSNKALPAPIEKTISKAKGQPREPQVYTLPPCRDELTKNQVSL
WCLVKGFYPSDIAVEWESNGQPENNYKTTPPVLDSDGSFFLYSKLTVDKSRW
QQGNVFSCSVMHEALHNHYTQKSLSLSPGK
> hu31Ll-CL (SEQ ID NO: 202) 
DIQMTQSPSSLSASVGDRVTITCRASGNIHNYLAWYQQKPGKAPKLLIYNAET
LADGVPSRFSGSGSGTDFTLTISSLQPEDFATYYCQHFWSTPPTFGGGTKVEIK
RTVAAPSVFIFPPSDEQLKSGTASVVCLLNNFYPREAKVQWKVDNALQSGNSQ
ESVTEQDSKDSTYSLSSTLTLSKADYEKHKVYACEVTHQGLSSPVTKSFNRGE
C
> hu43H3-F3-1 (SEQ ID NO: 203) 
EVQFVESGGGLVQPGGSLRLSCAASGFTFSNYWMNWLRQAPGKGLEWVAQV
RLKSDNYATHYAESVKGRFTISREDAKNSAYLQMNSLRAEDTAVYYCSENYY
GPAGPFDFWGQGTTVTVSSASTKGPSVFPLAPSSKSTSGGTAALGCLVKDYFP
EPVTVSWNSGALTSGVHTFPAVLQSSGLYSLSSVVTVPSSSLGTQTYICNVNHK
PSNTKVDKKVEPKSCEVQLVESGGGLVQPGGSLKLSCAASGFTFNKYAMNW
VRQAPGKGLEWVARIRSKYNNYATYYADSVKDRFTISRDDSKNTAYLQMNNL
KTEDTAVYYCVRHGNFGNEYISYWAYWGQGTLVTVSSGGGGSGIPPKIECLPI
DISIDEGKVLTVASAFTGEPTPEVTWSTGGRKIHSQEQGRFHIENTDDSTTLTIK
DVQKQDGGLYTLTLRNEFGSDSATVNIHIRSIDKTHTCPPCPAPEAAGGPSVFL
FPPKPKDTLMISRTPEVTCVVVDVSHEDPEVKFNWYVDGVEVHNAKTKPREE
QYNSTYRVVSVLTVLHQDWLNGKEYKCKVSNKALPAPIEKTISKAKGQPREP
QVYTLPPSRDELTKNQVSLTCLVKGFYPSDIAVEWESNGQPENNYKTTPPVLD
SDGSFFLYSKLTVDKSRWQQGNVFSCSVMHEALHNHYTQKSLSLSPGK
> hu43L2-CL (SEQ ID NO: 204) 
DIQMTQSPSSLSASVGDRVTITCKASQNVHTSVAWYQQKPGKSPKLLIYSASN
RYSGVPSRFSGSGSGTDFTLTISSLQPEDVATYYCQQYSSYPLTFGQGTKLEIKR
TVAAPSVFIFPPSDEQLKSGTASVVCLLNNFYPREAKVQWKVDNALQSGNSQE
SVTEQDSKDSTYSLSSTLTLSKADYEKHKVYACEVTHQGLSSPVTKSFNRGEC
> hu43H3-F4-1 (SEQ ID NO: 205) 
EVQFVESGGGLVQPGGSLRLSCAASGFTFSNYWMNWLRQAPGKGLEWVAQV
RLKSDNYATHYAESVKGRFTISREDAKNSAYLQMNSLRAEDTAVYYCSENYY
GPAGPFDFWGQGTTVTVSSASTKGPSVFPLAPSSKSTSGGTAALGCLVKDYFP
EPVTVSWNSGALTSGVHTFPAVLQSSGLYSLSSVVTVPSSSLGTQTYICNVNHK
PSNTKVDKKVEPKSCEVQLVESGGGLVQPGGSLKLSCAASGFTFNKYAMNW
VRQAPGKGLEWVARIRSKYNNYATYYADSVKDRFTISRDDSKNTAYLQMNNL
KTEDTAVYYCVRHGNFGNEYISYWAYWGQGTLVTVSSGGGGSGGGGSGGGG
SQTVVTQEPSLTVSPGGTVTLTCGSSTGAVTSGNYPNWVQQKPGQAPRGLIGG
TKFLAPGTPARFSGSLLGGKAALTLSGVQPEDEAEYYCVLWYSNRWVFGGGT
KLTVLGGGDKTHTCPPCPAPEAAGGPSVFLFPPKPKDTLMISRTPEVTCVVVD
VSHEDPEVKFNWYVDGVEVHNAKTKPREEQYNSTYRVVSVLTVLHQDWLN
GKEYKCKVSNKALPAPIEKTISKAKGQPREPQVYTLPPSRDELTKNQVSLTCLV
KGFYPSDIAVEWESNGQPENNYKTTPPVLDSDGSFFLYSKLTVDKSRWQQGN
VFSCSVMHEALHNHYTQKSLSLSPGK
> hu46H2-F1-1, also referred to as hu46H2-F2-1 (SEQ ID NO: 206) 
EVQLVESGGGLVKPGGSLRLSCAASGFTFSNYGMSWVRQAPGKGLEWVSTIG
SGGGYTYYSDSVKGRFTISRDNAKNSLYLQMNSLRAEDTAVYYCVRHPQIGY
WGQGTLVTVSSASTKGPSVFPLAPSSKSTSGGTAALGCLVKDYFPEPVTVSWN
SGALTSGVHTFPAVLQSSGLYSLSSVVTVPSSSLGTQTYICNVNHKPSNTKVDK
KVEPKSCDKTHTCPPCPAPEAAGGPSVFLFPPKPKDTLMISRTPEVTCVVVDVS
HEDPEVKFNWYVDGVEVHNAKTKPREEQYNSTYRVVSVLTVLHQDWLNGK
EYKCKVSNKALPAPIEKTISKAKGQPREPQVYTLPPCRDELTKNQVSLWCLVK
GFYPSDIAVEWESNGQPENNYKTTPPVLDSDGSFFLYSKLTVDKSRWQQGNVF
SCSVMHEALHNHYTQKSLSLSPGK
> hu46L2-CL (SEQ ID NO: 207) 
DIQMTQSPSSLSASVGDRVTITCKASQSVGTAVAWYQQKPGKAPKLLIYSASSR
YTGVPSRFSGSGSGTDFTLTISSLQPEDFADYFCHQYSSYPLTFGGGTKVEIKRT
VAAPSVFIFPPSDEQLKSGTASVVCLLNNFYPREAKVQWKVDNALQSGNSQES
VTEQDSKDSTYSLSSTLTLSKADYEKHKVYACEVTHQGLSSPVTKSFNRGEC
> hu46H2-F2-3 (SEQ ID NO: 208) 
EVQLVESGGGLVKPGGSLRLSCAASGFTFSNYGMSWVRQAPGKGLEWVSTIG
SGGGYTYYSDSVKGRFTISRDNAKNSLYLQMNSLRAEDTAVYYCVRHPQIGY
WGQGTLVTVSSASTKGPSVFPLAPSSKSTSGGTAALGCLVKDYFPEPVTVSWN
SGALTSGVHTFPAVLQSSGLYSLSSVVTVPSSSLGTQTYICNVNHKPSNTKVDK
KVEPKSCEVQLVESGGGLVQPGGSLKLSCAASGFTFNKYAMNWVRQAPGKG
LEWVARIRSKYNNYATYYADSVKDRFTISRDDSKNTAYLQMNNLKTEDTAVY
YCVRHGNFGNEYISYWAYWGQGTLVTVSSGGGGSGIPPKIECLPIDISIDEGKV
LTVASAFTGEPTPEVTWSTGGRKIHSQEQGRFHIENTDDSTTLTIKDVQKQDGG
LYTLTLRNEFGSDSATVNIHIRSIDKTHTCPPCPAPEAAGGPSVFLFPPKPKDTL
MISRTPEVTCVVVDVSHEDPEVKFNWYVDGVEVHNAKTKPREEQYNSTYRV
 
VSVLTVLHQDWLNGKEYKCKVSNKALPAPIEKTISKAKGQPREPQVCTLPPSR
DELTKNQVSLSCAVKGFYPSDIAVEWESNGQPENNYKTTPPVLDSDGSFFLVS
KLTVDKSRWQQGNVFSCSVMHEALHNHYTQKSLSLSPGK
> hu46H2-F4-1 (SEQ ID NO: 209) 
EVQLVESGGGLVKPGGSLRLSCAASGFTFSNYGMSWVRQAPGKGLEWVSTIG
SGGGYTYYSDSVKGRFTISRDNAKNSLYLQMNSLRAEDTAVYYCVRHPQIGY
WGQGTLVTVSSASTKGPSVFPLAPSSKSTSGGTAALGCLVKDYFPEPVTVSWN
SGALTSGVHTFPAVLQSSGLYSLSSVVTVPSSSLGTQTYICNVNHKPSNTKVDK
KVEPKSCEVQLVESGGGLVQPGGSLKLSCAASGFTFNKYAMNWVRQAPGKG
LEWVARIRSKYNNYATYYADSVKDRFTISRDDSKNTAYLQMNNLKTEDTAVY
YCVRHGNFGNEYISYWAYWGQGTLVTVSSGGGGSGGGGSGGGGSQTVVTQE
PSLTVSPGGTVTLTCGSSTGAVTSGNYPNWVQQKPGQAPRGLIGGTKFLAPGT
PARFSGSLLGGKAALTLSGVQPEDEAEYYCVLWYSNRWVFGGGTKLTVLGG
GDKTHTCPPCPAPEAAGGPSVFLFPPKPKDTLMISRTPEVTCVVVDVSHEDPE
VKFNWYVDGVEVHNAKTKPREEQYNSTYRVVSVLTVLHQDWLNGKEYKCK
VSNKALPAPIEKTISKAKGQPREPQVYTLPPSRDELTKNQVSLTCLVKGFYPSDI
AVEWESNGQPENNYKTTPPVLDSDGSFFLYSKLTVDKSRWQQGNVFSCSVMH
EALHNHYTQKSLSLSPGK
> 73VH-F1-1 (SEQ ID NO: 284) 
QVQLQQSGPELVKPGASVKISCKASGYAFSTSWMNWVKERPGKGLEWIGRIY
PGDGDTKYNGKFQGKATLTADKSSSTAYMKLSSLTSEDSAVYFCVRSIYYLYY
FDYWGQGTTLTVSSASTKGPSVFPLAPSSKSTSGGTAALGCLVKDYFPEPVTV
SWNSGALTSGVHTFPAVLQSSGLYSLSSVVTVPSSSLGTQTYICNVNHKPSNTK
VDKKVEPKSCDKTHTCPPCPAPEAAGGPSVFLFPPKPKDTLMISRTPEVTCVV
VDVSHEDPEVKFNWYVDGVEVHNAKTKPREEQYNSTYRVVSVLTVLHQDW
LNGKEYKCKVSNKALPAPIEKTISKAKGQPREPQVYTLPPCRDELTKNQVSLW
CLVKGFYPSDIAVEWESNGQPENNYKTTPPVLDSDGSFFLYSKLTVDKSRWQQ
GNVFSCSVMHEALHNHYTQKSLSLSPGK
> 73VL-CL (SEQ ID NO: 285) 
DIQMTQPTSSLSASLGDRVTISCSTSQDIYNNLSWFQQKPDGTVRLLIYYTSSL
HSGVPSRFSGSGSGTDYSLTISNLEPEDIATYYCQQYNNLPFTFGGGTNLEIRRT
VAAPSVFIFPPSDEQLKSGTASVVCLLNNFYPREAKVQWKVDNALQSGNSQES
VTEQDSKDSTYSLSSTLTLSKADYEKHKVYACEVTHQGLSSPVTKSFNRGEC
> 73VH-F3-1 (SEQ ID NO: 286)

QVQLQQSGPELVKPGASVKISCKASGYAFSTSWMNWVKERPGKGLEWIGRIY
PGDGDTKYNGKFQGKATLTADKSSSTAYMKLSSLTSEDSAVYFCVRSIYYLYY
FDYWGQGTTLTVSSASTKGPSVFPLAPSSKSTSGGTAALGCLVKDYFPEPVTV
SWNSGALTSGVHTFPAVLQSSGLYSLSSVVTVPSSSLGTQTYICNVNHKPSNTK
VDKKVEPKSCEVQLVESGGGLVQPGGSLKLSCAASGFTFNKYAMNWVRQAP
GKGLEWVARIRSKYNNYATYYADSVKDRFTISRDDSKNTAYLQMNNLKTEDT

AVYYCVRHGNFGNEYISYWAYWGQGTLVTVSSGGGGSGIPPKIECLPIDISIDE
GKVLTVASAFTGEPTPEVTWSTGGRKIHSQEQGRFHIENTDDSTTLTIKDVQKQ
DGGLYTLTLRNEFGSDSATVNIHIRSIDKTHTCPPCPAPEAAGGPSVFLFPPKPK
DTLMISRTPEVTCVVVDVSHEDPEVKFNWYVDGVEVHNAKTKPREEQYNST
YRVVSVLTVLHQDWLNGKEYKCKVSNKALPAPIEKTISKAKGQPREPQVYTL
PPSRDELTKNQVSLTCLVKGFYPSDIAVEWESNGQPENNYKTTPPVLDSDGSFF
LYSKLTVDKSRWQQGNVFSCSVMHEALHNHYTQKSLSLSPGK.

The positive control molecule used in the present disclosure was Aca-Mab, the amino acid sequence of which is set forth in SEQ ID NO: 382 of WO2017134158A1.

### Test Examples

Cho-human PSMA and Cho-monkey PSMA overexpressing cell strains were obtained by using methods described in other previous patents. Hamster ovary cell Cho-K1, human prostate cancer cell Lncap (ATCC), human prostate cancer cell 22RV1 (ATCC), and human prostate cancer cell PC-3 (Cell Bank of the Chinese Academy of Sciences) were purchased.

### Test Example 1. Binding Activity of Anti-PSMA Antibodies to PSMA

To test the ability of the PSMA-targeting humanized antibodies of the present disclosure to bind to PSMA antigen, the ELISA method was used in this test example to measure the binding of the PSMA-targeting humanized antibodies to human PSMA antigen (purchased from R&D SYSTEM, 4234-ZN-010). The specific steps are as follows: A 96-well plate was coated with PSMA antigen at 1 µg/well and placed at 4 °C overnight. After the supernatant was discarded, the plate was blocked with 5% skim milk for 2 h. Different concentrations of humanized antibodies were prepared using 1% BSA. The initial concentration was 10 µg/mL and was serially diluted 4-fold to obtain 12 concentrations. After the blocked 96-well plate was washed twice, the prepared PSMA antigen was added, and the plate was incubated at 4 °C for 1 h. After four washes, the plate was incubated with Strepidation-HRP secondary antibody (1:10,000) at 4 °C for 1 h. After color development with TMB, the reaction was stopped with 0.1 M sulfuric acid. The ELISA results are shown in the table below.

**Table 18. ELISA binding of humanized antibodies to PSMA antigen**

| Antibody | ELISA binding | | Antibody | ELISA binding | |
|---|---|---|---|---|---|
| hu2H1L1 | Top | 0.9750 | Chi 13 | Top | 2.107 |
| | EC₅₀ (µg/mL) | 0.03540 | | EC₅₀ (µg/mL) | 0.04091 |
| hu15H4L2 | Top | 2.234 | hu19H2L2 | Top | 1.577 |
| | EC₅₀ (µg/mL) | 0.04212 | | EC₅₀ (µg/mL) | 0.08368 |
| hu27H1L6 | Top | 0.5533 | hu31H1L1 | Top | 1.765 |
| | EC₅₀ (µg/mL) | 0.3980 | | EC₅₀ (µg/mL) | 0.08640 |
| hu41H2L2 | Top | 0.4209 | hu43H3L2 | Top | 0.2453 |
| | EC₅₀ (µg/mL) | 0.6095 | | EC₅₀ (µg/mL) | 1.062 |
| hu46H2L2 | Top | 1.674 | hu50L2H1 | Top | 1.296 |
| | EC₅₀ (µg/mL) | 0.03648 | | EC₅₀ (µg/mL) | 0.04209 |
| hu71L2H3 | Top | 0.9521 | chi73 | Top | 2.078 |
| | EC₅₀ (µg/mL) | 0.05890 | | EC₅₀ (µg/mL) | 0.02903 |

| | | | | | |
|---|---|---|---|---|---|
| Note: Top represents the maximum absorbance. | | | | | |

The results show that the PSMA humanized antibodies and chimeric antibodies obtained by screening in the present disclosure have good binding activity to PSMA antigen.

### Test Example 2. Binding Activity of Antibodies to PSMA

The binding activity of antibodies to the stably transfected cell strain Cho-hPSMA, which overexpresses human PSMA, was measured by flow cytometry in this test example. The full-length gene encoding human PSMA was cloned into the mammalian cell expression vector pCDH. HEK293T cells (ATCC^{®} CRL-11268) were co-transfected with three plasmids-pVSV-G, pCMV-dR8.91, and pCDH-human PSMA-to package viruses. After 48 h of transfection, viruses were collected to infect CHO-K1 cells. After two weeks of pressure screening, cell subcloning was performed, and a cell strain highly expressing PSMA, Cho-hPSMA, was obtained by FACS.

Experiment 1: Cho-hPSMA was cultured in F12 medium with 10% FBS. The medium to which cells were inoculated was incubated in a 37 °C, 5% CO₂ incubator for 2 days, and cells were added to a cell plate at 1 × 10⁵ cells/well and washed by centrifugation. Antibodies were serially diluted, and the antibody solutions were added to the cell plate at 100 µL/well. The plate was incubated at 4 °C for 1 h and then washed. An AF488-Goat Anti-human IgG Fc fluorescent secondary antibody dilution (1 :400) was added to the cell plate at 100 µL/well, and the plate was incubated at 4 °C for 1 h and then washed. PBS was added to the cell plate at 100 µL/well before plate reading. The cell-binding activity of the antibodies is shown in the table below.

**Table 19-1. The binding activity of humanized antibodies and chimeric antibodies to Cho-hPSMA**

| Antibody | EC₅₀ (µg/mL) | Antibody | EC₅₀ (µg/mL) |
|---|---|---|---|
| hu13H1L1 | 0.9474 | hu13H3L2 | 1.100 |
| hu13H2L1 | 1.055 | hu13H1L3 | 1.012 |
| hu13H3L1 | 1.052 | hu13H2L3 | 1.142 |
| hu13H1L2 | 0.9808 | hu13H3L3 | 1.495 |
| hu13H2L2 | 1.088 | Chi 13 | 1.213 |

Experiment 2: Cells were cultured and inoculated onto a cell plate using the same method as in Experiment 1. The antibodies were serially diluted, and the antibody solutions were added to the cell plate at 100 µL/well. The plate was incubated at 4 °C for 1 h and then washed. An HRP-Goat Anti-human IgG (H+L) secondary antibody dilution (1:8000) was added to the cell plate at 100 µL/well, and the plate was incubated at 4 °C for 1 h and then washed by centrifugation. After color development with TMB, the reaction was stopped with 0.1 M sulfuric acid, and OD450 values were measured. The cell-binding activity of the antibodies is shown in the table below.

**Table 19-2. The binding activity of humanized antibodies and chimeric antibodies to Cho-hPSMA (EC₅₀, µg/mL)**

| 1 | | | | | | | |
|---|---|---|---|---|---|---|---|
| hu15H1L1 | 0.06832 | hu15H4L1 | 0.06901 | hu15H2L2 | 0.08745 | hu15H5L2 | 0.08181 |
| hu15H2L1 | 0.08709 | hu15H5L1 | 0.08469 | hu15H3L2 | 0.08206 | | |
| hu15H3L1 | 0.07307 | hu15H1L2 | 0.07495 | hu15H4L2 | 0.07946 | | |

| 2 | | | | | | | |
|---|---|---|---|---|---|---|---|
| hu19H1L1 | 0.09453 | hu19H2L1 | 0.09187 | hu19H1L2 | 0.1117 | hu19H2L2 | 0.08892 |

| 3 | | | | | | | |
|---|---|---|---|---|---|---|---|
| hu27H1L1 | 1.170 | hu27H1L3 | 1.399 | hu27H1L5 | 2.389 | | |
| hu27H1L2 | 1.649 | hu27H1L4 | 1.725 | hu27H1L6 | 2.766 | | |

| 4 | | | | | | | |
|---|---|---|---|---|---|---|---|
| hu31H1L1 | 0.1416 | hu31H1L2 | 0.1203 | hu31H1L3 | 0.124 | Chi31 | 0.141 |

| 5 | | | | | | | |
|---|---|---|---|---|---|---|---|
| hu41H1L1 | 1.077 | hu41H1L3 | 0.9854 | hu41H2L2 | 1.097 | | |
| hu41H1L2 | 0.9824 | hu41H2L1 | 1.221 | hu41H2L3 | 1.205 | | |

| 6 | | | | | | | |
|---|---|---|---|---|---|---|---|
| hu43H1L1 | 6.221 | hu43H2L1 | 6.167 | hu43H3L1 | 2.831 | hu43H3L2 | 4.032 |

| 7 | | | | | | | |
|---|---|---|---|---|---|---|---|
| hu46H1L1 | 0.1359 | hu46H4L1 | 0.08426 | hu46H3L2 | 0.06169 | hu46H2L3 | 0.08049 |
| hu46H2L1 | 0.07879 | hu46H1L2 | 0.08133 | hu46H4L2 | 0.07903 | hu46H3L3 | 0.09118 |
| hu46H3L1 | 0.05969 | hu46H2L2 | 0.02016 | hu46H1L3 | 0.1229 | | |

| 8 | | | | | | | |
|---|---|---|---|---|---|---|---|
| hu50H1L1 | 0.06790 | hu50H1L2 | 0.06026 | hu50H1L3 | 0.1085 | | |
| hu50H2L1 | 0.0487 | hu50H2L2 | 0.04769 | hu50H2L3 | 0.1045 | | |

| 9 | | | | | | | |
|---|---|---|---|---|---|---|---|
| hu71H1L1 | 0.1196 | hu71H3L1 | 0.08603 | hu71H1L2 | 0.1099 | hu71H3L2 | 0.1120 |
| hu71H2L1 | 0.06314 | hu71H4L1 | 0.1005 | hu71H2L2 | 0.1164 | hu71H4L2 | 0.08670 |

Experiment 3: The binding activity of bispecific antibodies was measured using the method of experiment 1. The binding activity of antibodies to cells at various concentrations is shown in the table below.

**Table 19-3. The binding activity (fluorescence intensity) of bispecific antibodies to Cho-hPSMA**

| 1 | | | | | | | | | | | | |
|---|---|---|---|---|---|---|---|---|---|---|---|---|
| Antibody concentration (nM) | 15-F2 | 15-F4 | | 19-F1 | 19-F2 | 19-F4 | | 46-F1 | 46-F2 | 46-F4 | | Aca-Mab |
| 100 | 26855 | 32222 | | 35366 | 30316 | 30823 | | 31578 | 23038 | 21294 | | 10883 |
| 20 | 30717 | 31433 | | 30714 | 28549 | 29310 | | 28645 | 23056 | 20779 | | 4390 |
| 4 | 27811 | 21967 | | 13384 | 21430 | 20428 | | 12287 | 19426 | 18745 | | 1284 |
| 0.8 | 15001 | 8285 | | 3669 | 7748 | 6914 | | 3929 | 8326 | 9489 | | 646 |

| 2 | | | | | | | | | | | | |
|---|---|---|---|---|---|---|---|---|---|---|---|---|
| Antibody concentration (µg/mL) | 19-F1 | | 31-F1 | | 15-F5-1 | | 19-F5 | | 15-F5-2 | | Aca-Mab | |
| 100 | 11971 | | 16453 | | 10654 | | 9213 | | 10364 | | 7996 | |
| 20 | 15633 | | 16224 | | 15039 | | 12101 | | 14282 | | 6390 | |
| 4 | 9297 | | 7969 | | 12359 | | 8811 | | 10813 | | 2421 | |
| 0.8 | 3091 | | 2516 | | 5370 | | 3239 | | 4627 | | 687 | |

| 3 | | | | | | | | | | | | |
|---|---|---|---|---|---|---|---|---|---|---|---|---|
| Antibody concentration (µg/mL) | 73-F1 | | 73-F3 | | Aca-Mab | | | | | | | |
| 40.00 | 1638 | | 612 | | 413 | | | | | | | |
| 5.71 | 1533 | | 553 | | 302 | | | | | | | |
| 0.82 | 810 | | 455 | | 94 | | | | | | | |
| 0.12 | 121 | | 125 | | 26 | | | | | | | |

Experiment 4: The binding activity of bispecific antibodies was measured using the method of experiment 2. The binding activity of antibodies to cells at various concentrations is shown in the table below.

**Table 19-4. The binding activity (absorbance) of bispecific antibodies to Cho-hPSMA**

| Antibody concentration (nM) | 27-F4 | 43-F3 | 43-F4 | 2-F3 | 2-F4 | Aca-Mab |
|---|---|---|---|---|---|---|
| 100 | NA | 1.3903 | 1.4031 | 1.8077 | 1.3578 | 0.768 |
| 25 | 1.0463 | 0.7209 | 1.0401 | 1.63 | 1.5745 | 0.4532 |
| 6.25 | 0.7315 | 0.4406 | 0.5267 | 1.1226 | 1.2698 | 0.3044 |
| 1.5625 | 0.4194 | 0.2266 | 0.2541 | 0.5479 | 0.5673 | 0.1901 |

Experiment 5: Antibodies were affinity-captured using a Protein A biosensor chip, and human PSMA was then allowed to flow over the chip surface. The association and dissociation curves were obtained by real-time detection of reaction signals using a Biacore T200 instrument at 25 °C. After the dissociation in each experimental cycle was complete, the biosensor chip was regenerated by washing with Glycine 1.5. Data fitting was performed using a 1:1 Model. The human PSMA antigen was purchased from Aero (Cat. No. PSA-H52H3; Lys44-Ala750), and the hCD3D&CD3E complex antigen was purchased from Sino Biological (Cat. No. CT038-H2508H). The association and dissociation of each antibody are shown in the table below.

**Table 19-5. The binding activity of bispecific antibodies to human PSMA as measured by surface plasmon resonance**

| Bispecific antibody | Detection antigen | ka (1/Ms) | kd (1/s) | KD (M) |
|---|---|---|---|---|
| Aca-Mab | Human PSMA | 9.29E+03 | 1.31E-03 | 1.41E-07 |
| 15-F5-2 | | 5.00E+04 | 1.22E-04 | 2.43E-09 |
| 19-F1 | | 7.22E+03 | 1.11E-05 | 1.54E-09 |
| 31-F1 | | 9.89E+03 | 1.84E-05 | 1.86E-09 |

The results show that the anti-PSMA antibodies and bispecific antibodies of the present disclosure have good binding activity to PSMA, and the binding activity is stronger than that of Aca-mab.

### Test Example 3. In Vitro Cytotoxicity of Bispecific Antibodies of the Present Disclosure

In this test example, the killing activity of the bispecific antibodies of the present disclosure as T cell engager molecules against tumor cells was investigated. The target-specific cytotoxic activity of the bispecific antibodies of the present disclosure was tested using the PSMA-expressing cell line Lncap (human prostate cancer cells, metastatic) as the target cells. Fresh PBMCs (purchased from Xuanfeng Biotech Co.) were centrifuged at 300 g for 10 min. The supernatant was discarded, and the cells were resuspended in phenol red-free 1640 + 4% FBS. After another centrifugation, the cells were resuspended, counted, adjusted to a density of 1.5 × 10⁶ cells/mL, and added at 50 µL/well. The target cells were collected, centrifuged at 1000 rpm for 3 min, resuspended, counted, adjusted to a density of 3 × 10⁵ cells/mL, and added at 25 µL/well, with an E:T Ratio of 10:1. The antibodies were diluted with phenol red-free complete medium. The initial concentration was 400 nM (4× final concentration) and was diluted 8-fold to obtain 10 gradients. The antibodies were added at 25 µL/well. The cells were then incubated in a 37 °C, 5% CO₂ incubator for 48 h. Before the assay, 10 µL of medium was pipetted from two wells containing only target cells, and 10 µL of Lysis Solution (10×) was then added. After 45 min of lysis, the culture plate was taken out and centrifuged at 1000 rpm for 3 min, and 50 µL of the supernatant was pipetted to a new 96-well plate (#3590). 50 µL of dissolved CytoTox 96° Reagent was added in a 1:1 ratio. After 0.5 h of incubation at room temperature, 50 µL of stop solution was added, and the absorbance (490 nm) was measured using a FlexStation 3 (Molecular Devices).

**Table 20. The cytotoxic activity of bispecific antibodies against Lncap**

| 1 | | | 2 | | |
|---|---|---|---|---|---|
| Antibody | EC₅₀ (pM) | Maximum lysis rate% | Antibody | EC₅₀ (pM) | Maximum lysis rate% |
| 2-F2 | 8.400 | 48.73 | 15-F4 | 9.633 | 63.20 |
| 2-F3 | 0.6299 | 48.22 | 19-F3 | 4.960 | 65.24 |
| 2-F4 | 2.039 | 48.60 | 19-F4 | 10.85 | 64.91 |
| 43-F3 | 7.395 | 49.49 | 46-F4 | 2.854 | 63.54 |
| 43-F4 | 6.208 | 47.96 | Aca-Mab | 77.63 | 58.96 |
| Aca-Mab | 25.44 | 48.61 | | | |

| 3 | | | 4 | | |
|---|---|---|---|---|---|
| Antibody | EC₅₀ (pM) | Maximum lysis rate% | Antibody | EC₅₀ (pM) | Maximum lysis rate% |
| 13-F3 | 5.12 | 56.3 | 15-F5-1 | 12.1 | 64.95 |
| Aca-Mab | 18.01 | 54.4 | Aca-Mab | 25.06 | 55.09 |

The results show that the bispecific antibodies of the present disclosure exhibited relatively strong tumor-killing activity against PSMA-expressing Lncap cells, with their EC₅₀ values significantly lower than that of the control antibody Aca-Mab.

### Test Example 4. Cytokine Release Levels of Bispecific Antibodies of the Present Disclosure

CD3 T-cell engager molecules can cause cytokine storms. Therefore, when developing CD3 T-cell engager molecules, it is necessary to maintain cytokine levels, especially IL6, which is irrelevant to efficacy and can cause side effects, at relatively low levels.

In this test example, the levels of cytokines IFNγ and IL6 released during the killing of the PSMA-expressing cell line Lncap by the bispecific antibodies of the present disclosure were investigated. Fresh PBMCs (purchased from Xuanfeng Biotech Co.) were centrifuged at 300 g for 10 min. The supernatant was discarded, and the cells were resuspended in phenol red-free 1640 + 4% FBS. After another centrifugation, the cells were resuspended, counted, adjusted to a density of 1.5 × 10⁶ cells/mL, and added at 50 µL/well. The target cells were collected, centrifuged at 1000 rpm for 3 min, resuspended, counted, adjusted to a density of 3 × 10⁵ cells/mL, and added at 25 µL/well, with an E:T Ratio of 10:1. The antibodies were diluted with phenol red-free complete medium. The initial concentration was 400 nM (4× final concentration) and was diluted 8-fold to obtain 10 gradients. The antibodies were added at 25 µL/well. The cells were then incubated in a 37 °C, 5% CO₂ incubator for 48 h.

The IL6 assay was performed using the ELISA method. The cell sample described above was centrifuged at 1000 rpm for 3 min, and 50 µL of the supernatant was collected and diluted 6-fold with a general specimen diluent. The IL6 standard was also diluted 6-fold. The diluted samples or different concentrations of the standard were added to an assay plate (100 µL/well), and the reaction wells were sealed with plate-sealing adhesive paper. The plate was incubated at 37 °C for 90 min and then washed 5 times. A biotinylated antibody working solution was added (100 µL/well), and the reaction wells were sealed with new plate-sealing adhesive paper. The plate was incubated at 37 °C for 60 min and then washed 5 times. An enzyme conjugate working solution was added (100 µL/well), and the reaction wells were sealed with new plate-sealing adhesive paper. The plate was incubated at 37 °C for 30 min and then washed 5 times. The chromogenic substrate (TMB) was added at 100 µL/well, and the plate was incubated in the dark at 37 °C for 8 min. The reaction stop solution was added at 100 µL/well, and the OD450 values were measured immediately after thorough mixing (within 3 min).

The IFNγ assay was performed using the HTRF method. The cell sample described above was centrifuged at 1000 rpm for 3 min, and 50 µL of the supernatant was collected. The assay kit was equilibrated to room temperature, and the two assay antibodies in the kit were diluted (20-fold) with the assay buffer. 16 µL of the 20-fold diluted sample and the IFNγ standard were added to a 384-well plate, and 4 µL of the corresponding diluted assay antibody was added. A sealing material was attached, and the plate was shaken for thorough mixing, centrifuged at 1000 rpm for 1 min, incubated overnight at room temperature, and then centrifuged at 1000 rpm for 1 min. The sealing material was removed. The absorbance values at 665 nm and 620 nm were measured using a PHERAstar multi-mode microplate reader, and data were processed and analyzed using Graphpad Prism 5.

**Table 21-1. IFN-γ release in the Lncap cytotoxic activity experiment**

| 1 | | | | | | |
|---|---|---|---|---|---|---|
| Antibody concentration (pM) | 19-F1 | | 31-F1 | | Aca-Mab | |
| | IFNγ (pg/mL) | | | | | |
| 100000 | 5063.369 | | 6289.517 | | 16648.99 | |
| 10000 | 3823.259 | | 6619.214 | | 30450.18 | |
| 1000 | 2355.021 | | 11581.7 | | 15781.93 | |
| 100 | 1807.322 | | 1536.317 | | 3726.629 | |

| 2 | | | | | | |
|---|---|---|---|---|---|---|
| Antibody concentration (pM) | 19-F2 | | 19-F1 | | Aca-Mab | |
| | IFNγ (pg/mL) | | | | | |
| 100000 | 18087.21 | | 13565.2 | | 29375.2 | |
| 12500 | 10796.82 | | 6239.503 | | 29600.08 | |
| 1562.5 | 9965.275 | | 7621.483 | | 21258.31 | |
| 195.3125 | 214.1729 | | 1236.21 | | 5732.93 | |

| 3 | | | | | | |
|---|---|---|---|---|---|---|
| | 43-F4 | 43-F3 | 27-F4 | Aca-Mab | | |
| Antibody concentration (pM) | IFNγ (pg/mL) | | | | | |
| 100000 | 15786.95 | 20680.98 | 18217.46 | 24224.85 | | |
| 10000 | 17714.85 | 13421.09 | 16968.92 | 19892.93 | | |
| 1000 | 10776.15 | 14309.2 | 12062.14 | 17766.12 | | |
| 100 | 12380.72 | 8434.848 | 9088.648 | 9582.432 | | |

**Table 21-2. IL-6 release in the Lncap cytotoxic activity experiment**

| Antibody concentration (pM) | 1 | | 2 | |
|---|---|---|---|---|
| | 19-F1 | Aca-Mab | 15-F5-2 | Aca-Mab |
| | IL-6 (pg/mL) | | | |
| 100000 | 63.105 | 334.3985 | 92.3355 | 263.0121 |
| 10000 | 150.5496 | 766.2513 | 112.4726 | 344.7813 |
| 1000 | 87.07533 | 208.426 | 76.17301 | 163.1947 |

The results show that the bispecific antibodies of the present disclosure caused lower levels of IL6 and IFNγ released than Aca-Mab, suggesting that the bispecific antibodies of the present disclosure have better safety.

### Biological Evaluations of In Vivo Activity

### Test Example 5. Efficacy of Bispecific Antibodies of the Present Disclosure in 22RV1 Subcutaneous Graft Tumor Model

In the present disclosure, the anti-tumor activity of the PSMA-CD3 bispecific antibodies was evaluated using a human prostate cancer 22RV1 cell human PBMC NCG mouse xenograft tumor model.

NCG mice, male, 4-5 weeks old, were purchased from Jiangsu GemPharmatech Co., Ltd.

Human prostate cancer 22RV1 cells were purchased from ATCC.

22RV1 cells were added to RPMI-1640 medium containing 10% FBS and cultured in a 5% CO₂, 37 °C incubator with saturated humidity. 22RV1 cells in the logarithmic growth phase were collected and resuspended in RPMI-1640 medium containing 50% Matrigel, and the cell concentration was adjusted to 2 × 10⁷/mL. Under sterile conditions, 0.1 mL of the cell suspension was inoculated subcutaneously into the right side of the back of the mice, with an inoculation concentration of 2 × 10⁶/0.1 mL/mouse. One day after tumor cell inoculation, 0.1 mL of human PBMC suspension was intraperitoneally injected into the mice under sterile conditions, with an injection concentration of 8 × 10⁶/0.1 mL/mouse. When the mean tumor volume reached about 100 mm³, the animals were randomly grouped according to tumor volume, with the tumor volume difference between groups being less than 10% of the mean. The day of grouping was denoted by Day 0, and administration was started according to animal body weight. Administration was performed immediately after grouping. Intraperitoneal administration was performed twice weekly, and 6 successive administrations were performed (IP, BIW×3).

Tumor growth inhibition rate (%) = (1 - (Ti - T0)/(Ci - C0)) × 100%, where Ti and Ci are the tumor volumes of a treatment group and the control group at the end of the experiment, and T0 and C0 are the tumor volumes at the beginning of the experiment.

**Table 22. The efficacy of bispecific antibodies in the 22RV1 subcutaneous graft tumor model**

| Administration group | Dose (mg/kg) | Tumor volume (Day 21) mm³ | Tumor growth inhibition rate% |
|---|---|---|---|
| Blank (PBS) | / | 1977.87 | / |
| 31-F1 | 0.75 | 415.94 | 83.18 |
| 31-F1 | 3 | 464.92 | 80.57 |
| 43-F4 | 1 | 345.77 | 86.91 |
| 43-F4 | 4 | 181.52 | 95.66 |

The experimental results show that the bispecific antibodies 31-F1 and 43-F4 of the present disclosure exhibited good efficacy at the tested doses. No drug-related animal deaths or other significant drug-related toxic and side effects were observed during the experiment.

### Test Example 6. Efficacy of Bispecific Antibodies of the Present Disclosure in C4-2 Subcutaneous Graft Tumor Model

In the present disclosure, the anti-tumor activity of the PSMA-CD3 bispecific antibodies was evaluated using a human prostate cancer C4-2 cell human PBMC NCG mouse xenograft tumor model.

NCG mice, male, 7-8 weeks old, were purchased from Jiangsu GemPharmatech Co., Ltd.

Human prostate cancer C4-2 cells were purchased from ATCC.

C4-2 cells were cultured in DMEM/Ham's F12K (4:1) medium containing 10% fetal bovine serum in a 5% CO₂, 37 °C incubator with saturated humidity. C4-2 cells in the logarithmic growth phase were collected and resuspended in RPMI-1640 medium containing 50% Matrigel, and the cell concentration was adjusted to 2 × 10⁷/mL. Under sterile conditions, 0.1 mL of the cell suspension was inoculated subcutaneously into the right side of the back of the mice, with an inoculation concentration of 2 × 10⁶/0.1 mL/mouse. At day 3 after tumor cell inoculation, 0.1 mL of human PBMC suspension was intraperitoneally injected into the mice under sterile conditions, with an injection concentration of 1 × 10⁷/0.1 mL/mouse. At day 12 after tumor cell inoculation (the mean tumor volume reached about 51.60 mm³), the animals were randomly grouped according to tumor volume, with the tumor volume difference between groups being less than 10% of the mean, and administration was started according to animal body weight. Administration was performed immediately after grouping. Intraperitoneal administration was performed twice weekly, and 4 successive administrations were performed (IP, BIW×2).

**Table 23. The efficacy of bispecific antibodies in the C4-2 subcutaneous graft tumor model (day 10 post-administration)**

| Administration group | Dose (mg/kg) | Tumor volume (mm³) | Tumor growth inhibition rate% |
|---|---|---|---|
| Blank (PBS) | / | 1882.65 | / |
| Aca-Mab | 1 | 363.89 | 82.96 |
| 15-F5-1 | 1 | 111.27 | 96.74 |
| 15-F5-1 | 0.2 | 112.05 | 96.69 |
| 15-F5-2 | 0.2 | 170.32 | 93.50 |

The experimental results show that the tumor inhibition effects of the bispecific antibodies 15-F5-1 and 15-F5-2 on the C4-2 model is stronger than that of Aca-Mab; even low doses of the bispecific antibodies can achieve significantly stronger tumor inhibiting effects than high doses of Aca-Mab.

## Claims

1. An antigen-binding molecule that specifically binds to PSMA and CD3, comprising:
at least one antigen-binding moiety that specifically binds to PSMA, and
at least one antigen-binding moiety that specifically binds to CD3, wherein
the antigen-binding moiety that specifically binds to PSMA comprises a heavy chain variable region PSMA-VH and a light chain variable region PSMA-VL, and
the antigen-binding moiety that specifically binds to CD3 comprises a heavy chain variable region CD3-VH and a light chain variable region CD3-VL, wherein
(i) PSMA-HCDR1, PSMA-HCDR2, and PSMA-HCDR3 in the PSMA-VH comprise the amino acid sequences of PSMA-HCDR1, PSMA-HCDR2, and PSMA-HCDR3 in SEQ ID NO: 77, respectively, and
PSMA-LCDR1, PSMA-LCDR2, and PSMA-LCDR3 in the PSMA-VL comprise the amino acid sequences of PSMA-LCDR1, PSMA-LCDR2, and PSMA-LCDR3 in SEQ ID NO: 78, respectively, or
(ii) PSMA-HCDR1, PSMA-HCDR2, and PSMA-HCDR3 in the PSMA-VH comprise the amino acid sequences of PSMA-HCDR1, PSMA-HCDR2, and PSMA-HCDR3 in SEQ ID NO: 73, respectively, and
PSMA-LCDR1, PSMA-LCDR2, and PSMA-LCDR3 in the PSMA-VL comprise the amino acid sequences of PSMA-LCDR1, PSMA-LCDR2, and PSMA-LCDR3 in SEQ ID NO: 74, respectively, or
(iii) PSMA-HCDR1, PSMA-HCDR2, and PSMA-HCDR3 in the PSMA-VH comprise the amino acid sequences of PSMA-HCDR1, PSMA-HCDR2, and PSMA-HCDR3 in SEQ ID NO: 75, respectively, and
PSMA-LCDR1, PSMA-LCDR2, and PSMA-LCDR3 in the PSMA-VL comprise the amino acid sequences of PSMA-LCDR1, PSMA-LCDR2, and PSMA-LCDR3 in SEQ ID NO: 76, respectively, or
(iv) PSMA-HCDR1, PSMA-HCDR2, and PSMA-HCDR3 in the PSMA-VH comprise the amino acid sequences of PSMA-HCDR1, PSMA-HCDR2, and PSMA-HCDR3 in SEQ ID NO: 79, respectively, and
PSMA-LCDR1, PSMA-LCDR2, and PSMA-LCDR3 in the PSMA-VL comprise the amino acid sequences of PSMA-LCDR1, PSMA-LCDR2, and PSMA-LCDR3 in SEQ ID NO: 80, respectively, or
(v) PSMA-HCDR1, PSMA-HCDR2, and PSMA-HCDR3 in the PSMA-VH comprise the amino acid sequences of PSMA-HCDR1, PSMA-HCDR2, and PSMA-HCDR3 in SEQ ID NO: 81, respectively, and
PSMA-LCDR1, PSMA-LCDR2, and PSMA-LCDR3 in the PSMA-VL comprise the amino acid sequences of PSMA-LCDR1, PSMA-LCDR2, and PSMA-LCDR3 in SEQ ID NO: 82, respectively, or
(vi) PSMA-HCDR1, PSMA-HCDR2, and PSMA-HCDR3 in the PSMA-VH comprise the amino acid sequences of PSMA-HCDR1, PSMA-HCDR2, and PSMA-HCDR3 in SEQ ID NO: 83, respectively, and
PSMA-LCDR1, PSMA-LCDR2, and PSMA-LCDR3 in the PSMA-VL comprise the amino acid sequences of PSMA-LCDR1, PSMA-LCDR2, and PSMA-LCDR3 in SEQ ID NO: 84, respectively, or
(vii) PSMA-HCDR1, PSMA-HCDR2, and PSMA-HCDR3 in the PSMA-VH comprise the amino acid sequences of PSMA-HCDR1, PSMA-HCDR2, and PSMA-HCDR3 in SEQ ID NO: 85, respectively, and
PSMA-LCDR1, PSMA-LCDR2, and PSMA-LCDR3 in the PSMA-VL comprise the amino acid sequences of PSMA-LCDR1, PSMA-LCDR2, and PSMA-LCDR3 in SEQ ID NO: 86, respectively, or
(viii) PSMA-HCDR1, PSMA-HCDR2, and PSMA-HCDR3 in the PSMA-VH comprise the amino acid sequences of PSMA-HCDR1, PSMA-HCDR2, and PSMA-HCDR3 in SEQ ID NO: 87, respectively, and
PSMA-LCDR1, PSMA-LCDR2, and PSMA-LCDR3 in the PSMA-VL comprise the amino acid sequences of PSMA-LCDR1, PSMA-LCDR2, and PSMA-LCDR3 in SEQ ID NO: 88, respectively, or
(ix) PSMA-HCDR1, PSMA-HCDR2, and PSMA-HCDR3 in the PSMA-VH comprise the amino acid sequences of PSMA-HCDR1, PSMA-HCDR2, and PSMA-HCDR3 in SEQ ID NO: 89, respectively, and
PSMA-LCDR1, PSMA-LCDR2, and PSMA-LCDR3 in the PSMA-VL comprise the amino acid sequences of PSMA-LCDR1, PSMA-LCDR2, and PSMA-LCDR3 in SEQ ID NO: 90, respectively, or
(x) PSMA-HCDR1, PSMA-HCDR2, and PSMA-HCDR3 in the PSMA-VH comprise the amino acid sequences of PSMA-HCDR1, PSMA-HCDR2, and PSMA-HCDR3 in SEQ ID NO: 91, respectively, and
PSMA-LCDR1, PSMA-LCDR2, and PSMA-LCDR3 in the PSMA-VL comprise the amino acid sequences of PSMA-LCDR1, PSMA-LCDR2, and PSMA-LCDR3 in SEQ ID NO: 92 or 152, respectively, or
(xi) PSMA-HCDR1, PSMA-HCDR2, and PSMA-HCDR3 in the PSMA-VH comprise the amino acid sequences of PSMA-HCDR1, PSMA-HCDR2, and PSMA-HCDR3 in SEQ ID NO: 93, respectively, and
PSMA-LCDR1, PSMA-LCDR2, and PSMA-LCDR3 in the PSMA-VL comprise the amino acid sequences of PSMA-LCDR1, PSMA-LCDR2, and PSMA-LCDR3 in SEQ ID NO: 94, respectively, or
(xii) PSMA-HCDR1, PSMA-HCDR2, and PSMA-HCDR3 in the PSMA-VH comprise the amino acid sequences of PSMA-HCDR1, PSMA-HCDR2, and PSMA-HCDR3 in SEQ ID NO: 95, respectively, and
PSMA-LCDR1, PSMA-LCDR2, and PSMA-LCDR3 in the PSMA-VL comprise the amino acid sequences of PSMA-LCDR1, PSMA-LCDR2, and PSMA-LCDR3 in SEQ ID NO: 96, respectively;
preferably,
(i) the PSMA-VH has: a PSMA-HCDR1 comprising the amino acid sequence of SEQ ID NO: 13, a PSMA-HCDR2 comprising the amino acid sequence of SEQ ID NO: 14, and a PSMA-HCDR3 comprising the amino acid sequence of SEQ ID NO: 15, and
the PSMA-VL has: a PSMA-LCDR1 comprising the amino acid sequence of SEQ ID NO: 16, a PSMA-LCDR2 comprising the amino acid sequence of SEQ ID NO: 17, and
a PSMA-LCDR3 comprising the amino acid sequence of SEQ ID NO: 18, or
(ii) the PSMA-VH has: a PSMA-HCDR1 comprising the amino acid sequence of SEQ ID NO: 1, a PSMA-HCDR2 comprising the amino acid sequence of SEQ ID NO: 2, and
a PSMA-HCDR3 comprising the amino acid sequence of SEQ ID NO: 3, and
the PSMA-VL has: a PSMA-LCDR1 comprising the amino acid sequence of SEQ ID NO: 4, a PSMA-LCDR2 comprising the amino acid sequence of SEQ ID NO: 5, and a PSMA-LCDR3 comprising the amino acid sequence of SEQ ID NO: 6, or
(iii) the PSMA-VH has: a PSMA-HCDR1 comprising the amino acid sequence of SEQ ID NO: 7, a PSMA-HCDR2 comprising the amino acid sequence of SEQ ID NO: 8, and
a PSMA-HCDR3 comprising the amino acid sequence of SEQ ID NO: 9, and
the PSMA-VL has: a PSMA-LCDR1 comprising the amino acid sequence of SEQ ID NO: 10, a PSMA-LCDR2 comprising the amino acid sequence of SEQ ID NO: 11, and a PSMA-LCDR3 comprising the amino acid sequence of SEQ ID NO: 12, or
(iv) the PSMA-VH has: a PSMA-HCDR1 comprising the amino acid sequence of SEQ ID NO: 19, a PSMA-HCDR2 comprising the amino acid sequence of SEQ ID NO: 20, and a PSMA-HCDR3 comprising the amino acid sequence of SEQ ID NO: 21, and
the PSMA-VL has: a PSMA-LCDR1 comprising the amino acid sequence of SEQ ID NO: 22, a PSMA-LCDR2 comprising the amino acid sequence of SEQ ID NO: 23, and
a PSMA-LCDR3 comprising the amino acid sequence of SEQ ID NO: 24, or
(v) the PSMA-VH has: a PSMA-HCDR1 comprising the amino acid sequence of SEQ ID NO: 25, a PSMA-HCDR2 comprising the amino acid sequence of SEQ ID NO: 26, and a PSMA-HCDR3 comprising the amino acid sequence of SEQ ID NO: 27, and
the PSMA-VL has: a PSMA-LCDR1 comprising the amino acid sequence of SEQ ID NO: 28, a PSMA-LCDR2 comprising the amino acid sequence of SEQ ID NO: 29, and
a PSMA-LCDR3 comprising the amino acid sequence of SEQ ID NO: 30, or
(vi) the PSMA-VH has: a PSMA-HCDR1 comprising the amino acid sequence of SEQ ID NO: 31, a PSMA-HCDR2 comprising the amino acid sequence of SEQ ID NO: 32, and a PSMA-HCDR3 comprising the amino acid sequence of SEQ ID NO: 33, and
the PSMA-VL has: a PSMA-LCDR1 comprising the amino acid sequence of SEQ ID NO: 34, a PSMA-LCDR2 comprising the amino acid sequence of SEQ ID NO: 35, and
a PSMA-LCDR3 comprising the amino acid sequence of SEQ ID NO: 36, or
(vii) the PSMA-VH has: a PSMA-HCDR1 comprising the amino acid sequence of SEQ ID NO: 37, a PSMA-HCDR2 comprising the amino acid sequence of SEQ ID NO: 38, and a PSMA-HCDR3 comprising the amino acid sequence of SEQ ID NO: 39, and
the PSMA-VL has: a PSMA-LCDR1 comprising the amino acid sequence of SEQ ID NO: 40, a PSMA-LCDR2 comprising the amino acid sequence of SEQ ID NO: 41, and
a PSMA-LCDR3 comprising the amino acid sequence of SEQ ID NO: 42, or
(viii) the PSMA-VH has: a PSMA-HCDR1 comprising the amino acid sequence of SEQ ID NO: 43, a PSMA-HCDR2 comprising the amino acid sequence of SEQ ID NO: 44, and a PSMA-HCDR3 comprising the amino acid sequence of SEQ ID NO: 45, and
the PSMA-VL has: a PSMA-LCDR1 comprising the amino acid sequence of SEQ ID NO: 46, a PSMA-LCDR2 comprising the amino acid sequence of SEQ ID NO: 47, and
a PSMA-LCDR3 comprising the amino acid sequence of SEQ ID NO: 48, or
(ix) the PSMA-VH has: a PSMA-HCDR1 comprising the amino acid sequence of SEQ ID NO: 49, a PSMA-HCDR2 comprising the amino acid sequence of SEQ ID NO: 50, and a PSMA-HCDR3 comprising the amino acid sequence of SEQ ID NO: 51, and
the PSMA-VL has: a PSMA-LCDR1 comprising the amino acid sequence of SEQ ID NO: 52, a PSMA-LCDR2 comprising the amino acid sequence of SEQ ID NO: 53, and
a PSMA-LCDR3 comprising the amino acid sequence of SEQ ID NO: 54, or
(x) the PSMA-VH has: a PSMA-HCDR1 comprising the amino acid sequence of SEQ ID NO: 55, a PSMA-HCDR2 comprising the amino acid sequence of SEQ ID NO: 56, and a PSMA-HCDR3 comprising the amino acid sequence of SEQ ID NO: 57, and
the PSMA-VL has: a PSMA-LCDR1 comprising the amino acid sequence of SEQ ID NO: 58 or 159, a PSMA-LCDR2 comprising the amino acid sequence of SEQ ID NO: 59, and a PSMA-LCDR3 comprising the amino acid sequence of SEQ ID NO: 60, or
(xi) the PSMA-VH has: a PSMA-HCDR1 comprising the amino acid sequence of SEQ ID NO: 61, a PSMA-HCDR2 comprising the amino acid sequence of SEQ ID NO: 62, and a PSMA-HCDR3 comprising the amino acid sequence of SEQ ID NO: 63, and
the PSMA-VL has: a PSMA-LCDR1 comprising the amino acid sequence of SEQ ID NO: 64, a PSMA-LCDR2 comprising the amino acid sequence of SEQ ID NO: 65, and
a PSMA-LCDR3 comprising the amino acid sequence of SEQ ID NO: 66, or
(xii) the PSMA-VH has: a PSMA-HCDR1 comprising the amino acid sequence of SEQ ID NO: 67, a PSMA-HCDR2 comprising the amino acid sequence of SEQ ID NO: 68, and a PSMA-HCDR3 comprising the amino acid sequence of SEQ ID NO: 69, and
the PSMA-VL has: a PSMA-LCDR1 comprising the amino acid sequence of SEQ ID NO: 70, a PSMA-LCDR2 comprising the amino acid sequence of SEQ ID NO: 71, and
a PSMA-LCDR3 comprising the amino acid sequence of SEQ ID NO: 72;
more preferably,
the antigen-binding molecule binds to human PSMA at 25 °C with a KD of less than 1 × 10⁻⁸ M, as measured by surface plasmon resonance.

2. The antigen-binding molecule according to claim 1, wherein:
(i) the PSMA-VH comprises the amino acid sequence of SEQ ID NO: 112, 110, 77, 107, 108, 109, or 111, and the PSMA-VL comprises the amino acid sequence of SEQ ID NO: 115, 114, 78, or 113, or
(ii) the PSMA-VH comprises the amino acid sequence of SEQ ID NO: 73 or 99, and the PSMA-VL comprises the amino acid sequence of SEQ ID NO: 74 or 100, or
(iii) the PSMA-VH comprises the amino acid sequence of SEQ ID NO: 75, 101, 102, or 103, and the PSMA-VL comprises the amino acid sequence of SEQ ID NO: 76, 104, 105, or 106, or
(iv) the PSMA-VH comprises the amino acid sequence of SEQ ID NO: 79, 116, or 117, and the PSMA-VL comprises the amino acid sequence of SEQ ID NO: 80, 118, or 119, or
(v) the PSMA-VH comprises the amino acid sequence of SEQ ID NO: 81 or 120, and the PSMA-VL comprises the amino acid sequence of SEQ ID NO: 82, 121, 122, 123, 124, 125, or 126, or
(vi) the PSMA-VH comprises the amino acid sequence of SEQ ID NO: 127 or 83, and the PSMA-VL comprises the amino acid sequence of SEQ ID NO: 128, 84, 129, or 130, or
(vii) the PSMA-VH comprises the amino acid sequence of SEQ ID NO: 85, 131, or 132, and the PSMA-VL comprises the amino acid sequence of SEQ ID NO: 86, 133, 134, or 135, or
(viii) the PSMA-VH comprises the amino acid sequence of SEQ ID NO: 87, 136, 137, or 138, and the PSMA-VL comprises the amino acid sequence of SEQ ID NO: 88, 139, or 140, or
(ix) the PSMA-VH comprises the amino acid sequence of SEQ ID NO: 89, 141, 142, 143, or 144, and the PSMA-VL comprises the amino acid sequence of SEQ ID NO: 90, 145, 146, or 147, or
(x) the PSMA-VH comprises the amino acid sequence of SEQ ID NO: 91, 148, or 149, and the PSMA-VL comprises the amino acid sequence of SEQ ID NO: 92, 150, 151, or 152, or
(xi) the PSMA-VH comprises the amino acid sequence of SEQ ID NO: 93, 153, 154, 155, or 156, and the PSMA-VL comprises the amino acid sequence of SEQ ID NO: 94, 157, or 158; or
(xii) the PSMA-VH comprises the amino acid sequence of SEQ ID NO: 95, 277, 278, or 279, and the PSMA-VL comprises the amino acid sequence of SEQ ID NO: 96, 280, 281, 282, or 283;
preferably,
(i) the PSMA-VH comprises the amino acid sequence of SEQ ID NO: 112, and the PSMA-VL comprises the amino acid sequence of SEQ ID NO: 115, or
the PSMA-VH comprises the amino acid sequence of SEQ ID NO: 110, and the PSMA-VL comprises the amino acid sequence of SEQ ID NO: 114, or
the PSMA-VH comprises the amino acid sequence of SEQ ID NO: 77, and the PSMA-VL comprises the amino acid sequence of SEQ ID NO: 78, or
the PSMA-VH comprises the amino acid sequence of SEQ ID NO: 107, and the PSMA-VL comprises the amino acid sequence of SEQ ID NO: 113, or
the PSMA-VH comprises the amino acid sequence of SEQ ID NO: 108, and the PSMA-VL comprises the amino acid sequence of SEQ ID NO: 113, or
the PSMA-VH comprises the amino acid sequence of SEQ ID NO: 109, and the PSMA-VL comprises the amino acid sequence of SEQ ID NO: 113, or
the PSMA-VH comprises the amino acid sequence of SEQ ID NO: 110, and the PSMA-VL comprises the amino acid sequence of SEQ ID NO: 113, or
the PSMA-VH comprises the amino acid sequence of SEQ ID NO: 111, and the PSMA-VL comprises the amino acid sequence of SEQ ID NO: 113, or
the PSMA-VH comprises the amino acid sequence of SEQ ID NO: 107, and the PSMA-VL comprises the amino acid sequence of SEQ ID NO: 114, or
the PSMA-VH comprises the amino acid sequence of SEQ ID NO: 108, and the PSMA-VL comprises the amino acid sequence of SEQ ID NO: 114, or
the PSMA-VH comprises the amino acid sequence of SEQ ID NO: 109, and the PSMA-VL comprises the amino acid sequence of SEQ ID NO: 114, or
the PSMA-VH comprises the amino acid sequence of SEQ ID NO: 111, and the PSMA-VL comprises the amino acid sequence of SEQ ID NO: 114, or
(ii) the PSMA-VH comprises the amino acid sequence of SEQ ID NO: 73, and the PSMA-VL comprises the amino acid sequence of SEQ ID NO: 74, or
the PSMA-VH comprises the amino acid sequence of SEQ ID NO: 99, and the PSMA-VL comprises the amino acid sequence of SEQ ID NO: 100, or
(iii) the PSMA-VH comprises the amino acid sequence of SEQ ID NO: 75, and the PSMA-VL comprises the amino acid sequence of SEQ ID NO: 76, or
the PSMA-VH comprises the amino acid sequence of SEQ ID NO: 101, and the PSMA-VL comprises the amino acid sequence of SEQ ID NO: 104, or
the PSMA-VH comprises the amino acid sequence of SEQ ID NO: 102, and the PSMA-VL comprises the amino acid sequence of SEQ ID NO: 104, or
the PSMA-VH comprises the amino acid sequence of SEQ ID NO: 103, and the PSMA-VL comprises the amino acid sequence of SEQ ID NO: 104, or
the PSMA-VH comprises the amino acid sequence of SEQ ID NO: 101, and the PSMA-VL comprises the amino acid sequence of SEQ ID NO: 105, or
the PSMA-VH comprises the amino acid sequence of SEQ ID NO: 102, and the PSMA-VL comprises the amino acid sequence of SEQ ID NO: 105, or
the PSMA-VH comprises the amino acid sequence of SEQ ID NO: 103, and the PSMA-VL comprises the amino acid sequence of SEQ ID NO: 105, or
the PSMA-VH comprises the amino acid sequence of SEQ ID NO: 101, and the PSMA-VL comprises the amino acid sequence of SEQ ID NO: 106, or
the PSMA-VH comprises the amino acid sequence of SEQ ID NO: 102, and the PSMA-VL comprises the amino acid sequence of SEQ ID NO: 106, or
the PSMA-VH comprises the amino acid sequence of SEQ ID NO: 103, and the PSMA-VL comprises the amino acid sequence of SEQ ID NO: 106, or
(iv) the PSMA-VH comprises the amino acid sequence of SEQ ID NO: 79, and the PSMA-VL comprises the amino acid sequence of SEQ ID NO: 80, or
the PSMA-VH comprises the amino acid sequence of SEQ ID NO: 116, and the PSMA-VL comprises the amino acid sequence of SEQ ID NO: 118, or
the PSMA-VH comprises the amino acid sequence of SEQ ID NO: 117, and the PSMA-VL comprises the amino acid sequence of SEQ ID NO: 118, or
the PSMA-VH comprises the amino acid sequence of SEQ ID NO: 116, and the PSMA-VL comprises the amino acid sequence of SEQ ID NO: 119, or
the PSMA-VH comprises the amino acid sequence of SEQ ID NO: 117, and the PSMA-VL comprises the amino acid sequence of SEQ ID NO: 119, or
(v) the PSMA-VH comprises the amino acid sequence of SEQ ID NO: 81, and the PSMA-VL comprises the amino acid sequence of SEQ ID NO: 82, or
the PSMA-VH comprises the amino acid sequence of SEQ ID NO: 120, and the PSMA-VL comprises the amino acid sequence of SEQ ID NO: 121, or
the PSMA-VH comprises the amino acid sequence of SEQ ID NO: 120, and the PSMA-VL comprises the amino acid sequence of SEQ ID NO: 122, or
the PSMA-VH comprises the amino acid sequence of SEQ ID NO: 120, and the PSMA-VL comprises the amino acid sequence of SEQ ID NO: 123, or
the PSMA-VH comprises the amino acid sequence of SEQ ID NO: 120, and the PSMA-VL comprises the amino acid sequence of SEQ ID NO: 124, or
the PSMA-VH comprises the amino acid sequence of SEQ ID NO: 120, and the PSMA-VL comprises the amino acid sequence of SEQ ID NO: 125, or
the PSMA-VH comprises the amino acid sequence of SEQ ID NO: 120, and the PSMA-VL comprises the amino acid sequence of SEQ ID NO: 126, or
(vi) the PSMA-VH comprises the amino acid sequence of SEQ ID NO: 83, and the PSMA-VL comprises the amino acid sequence of SEQ ID NO: 84, or
the PSMA-VH comprises the amino acid sequence of SEQ ID NO: 127, and the PSMA-VL comprises the amino acid sequence of SEQ ID NO: 128, or
the PSMA-VH comprises the amino acid sequence of SEQ ID NO: 127, and the PSMA-VL comprises the amino acid sequence of SEQ ID NO: 129, or
the PSMA-VH comprises the amino acid sequence of SEQ ID NO: 127, and the PSMA-VL comprises the amino acid sequence of SEQ ID NO: 130, or
(vii) the PSMA-VH comprises the amino acid sequence of SEQ ID NO: 85, and the PSMA-VL comprises the amino acid sequence of SEQ ID NO: 86, or
the PSMA-VH comprises the amino acid sequence of SEQ ID NO: 131, and the PSMA-VL comprises the amino acid sequence of SEQ ID NO: 133, or
the PSMA-VH comprises the amino acid sequence of SEQ ID NO: 131, and the PSMA-VL comprises the amino acid sequence of SEQ ID NO: 134, or
the PSMA-VH comprises the amino acid sequence of SEQ ID NO: 131, and the PSMA-VL comprises the amino acid sequence of SEQ ID NO: 135, or
the PSMA-VH comprises the amino acid sequence of SEQ ID NO: 132, and the PSMA-VL comprises the amino acid sequence of SEQ ID NO: 133, or
the PSMA-VH comprises the amino acid sequence of SEQ ID NO: 132, and the PSMA-VL comprises the amino acid sequence of SEQ ID NO: 134, or
the PSMA-VH comprises the amino acid sequence of SEQ ID NO: 132, and the PSMA-VL comprises the amino acid sequence of SEQ ID NO: 135, or
(viii) the PSMA-VH comprises the amino acid sequence of SEQ ID NO: 87, and the PSMA-VL comprises the amino acid sequence of SEQ ID NO: 88, or
the PSMA-VH comprises the amino acid sequence of SEQ ID NO: 136, and the PSMA-VL comprises the amino acid sequence of SEQ ID NO: 139, or
the PSMA-VH comprises the amino acid sequence of SEQ ID NO: 137, and the PSMA-VL comprises the amino acid sequence of SEQ ID NO: 139, or
the PSMA-VH comprises the amino acid sequence of SEQ ID NO: 138, and the PSMA-VL comprises the amino acid sequence of SEQ ID NO: 139, or
the PSMA-VH comprises the amino acid sequence of SEQ ID NO: 138, and the PSMA-VL comprises the amino acid sequence of SEQ ID NO: 140, or
(ix) the PSMA-VH comprises the amino acid sequence of SEQ ID NO: 89, and the PSMA-VL comprises the amino acid sequence of SEQ ID NO: 90, or
the PSMA-VH comprises the amino acid sequence of SEQ ID NO: 141, and the PSMA-VL comprises the amino acid sequence of SEQ ID NO: 145, or
the PSMA-VH comprises the amino acid sequence of SEQ ID NO: 142, and the PSMA-VL comprises the amino acid sequence of SEQ ID NO: 145, or
the PSMA-VH comprises the amino acid sequence of SEQ ID NO: 143, and the PSMA-VL comprises the amino acid sequence of SEQ ID NO: 145, or
the PSMA-VH comprises the amino acid sequence of SEQ ID NO: 144, and the PSMA-VL comprises the amino acid sequence of SEQ ID NO: 145, or
the PSMA-VH comprises the amino acid sequence of SEQ ID NO: 141, and the PSMA-VL comprises the amino acid sequence of SEQ ID NO: 146, or
the PSMA-VH comprises the amino acid sequence of SEQ ID NO: 142, and the PSMA-VL comprises the amino acid sequence of SEQ ID NO: 146, or
the PSMA-VH comprises the amino acid sequence of SEQ ID NO: 143, and the PSMA-VL comprises the amino acid sequence of SEQ ID NO: 146, or
the PSMA-VH comprises the amino acid sequence of SEQ ID NO: 144, and the PSMA-VL comprises the amino acid sequence of SEQ ID NO: 146, or
the PSMA-VH comprises the amino acid sequence of SEQ ID NO: 141, and the PSMA-VL comprises the amino acid sequence of SEQ ID NO: 147, or
the PSMA-VH comprises the amino acid sequence of SEQ ID NO: 142, and the PSMA-VL comprises the amino acid sequence of SEQ ID NO: 147, or
the PSMA-VH comprises the amino acid sequence of SEQ ID NO: 143, and the PSMA-VL comprises the amino acid sequence of SEQ ID NO: 147, or
(x) the PSMA-VH comprises the amino acid sequence of SEQ ID NO: 91, and the PSMA-VL comprises the amino acid sequence of SEQ ID NO: 92, or
the PSMA-VH comprises the amino acid sequence of SEQ ID NO: 148, and the PSMA-VL comprises the amino acid sequence of SEQ ID NO: 150, or
the PSMA-VH comprises the amino acid sequence of SEQ ID NO: 149, and the PSMA-VL comprises the amino acid sequence of SEQ ID NO: 150, or
the PSMA-VH comprises the amino acid sequence of SEQ ID NO: 148, and the PSMA-VL comprises the amino acid sequence of SEQ ID NO: 151, or
the PSMA-VH comprises the amino acid sequence of SEQ ID NO: 149, and the PSMA-VL comprises the amino acid sequence of SEQ ID NO: 151, or
the PSMA-VH comprises the amino acid sequence of SEQ ID NO: 148, and the PSMA-VL comprises the amino acid sequence of SEQ ID NO: 152, or
the PSMA-VH comprises the amino acid sequence of SEQ ID NO: 149, and the PSMA-VL comprises the amino acid sequence of SEQ ID NO: 152, or
(xi) the PSMA-VH comprises the amino acid sequence of SEQ ID NO: 93, and the PSMA-VL comprises the amino acid sequence of SEQ ID NO: 94; or
the PSMA-VH comprises the amino acid sequence of SEQ ID NO: 153, and the PSMA-VL comprises the amino acid sequence of SEQ ID NO: 157; or
the PSMA-VH comprises the amino acid sequence of SEQ ID NO: 154, and the PSMA-VL comprises the amino acid sequence of SEQ ID NO: 157; or
the PSMA-VH comprises the amino acid sequence of SEQ ID NO: 155, and the PSMA-VL comprises the amino acid sequence of SEQ ID NO: 157; or
the PSMA-VH comprises the amino acid sequence of SEQ ID NO: 156, and the PSMA-VL comprises the amino acid sequence of SEQ ID NO: 157; or
the PSMA-VH comprises the amino acid sequence of SEQ ID NO: 153, and the PSMA-VL comprises the amino acid sequence of SEQ ID NO: 158; or
the PSMA-VH comprises the amino acid sequence of SEQ ID NO: 154, and the PSMA-VL comprises the amino acid sequence of SEQ ID NO: 158; or
the PSMA-VH comprises the amino acid sequence of SEQ ID NO: 155, and the PSMA-VL comprises the amino acid sequence of SEQ ID NO: 158; or
the PSMA-VH comprises the amino acid sequence of SEQ ID NO: 156, and the PSMA-VL comprises the amino acid sequence of SEQ ID NO: 158; or
(xii) the PSMA-VH comprises the amino acid sequence of SEQ ID NO: 95, and the PSMA-VL comprises the amino acid sequence of SEQ ID NO: 96;
more preferably,
(i) the PSMA-VH comprises the amino acid sequence of SEQ ID NO: 112, and the PSMA-VL comprises the amino acid sequence of SEQ ID NO: 115, or
the PSMA-VH comprises the amino acid sequence of SEQ ID NO: 110, and the PSMA-VL comprises the amino acid sequence of SEQ ID NO: 114, or
(vi) the PSMA-VH comprises the amino acid sequence of SEQ ID NO: 127, and the PSMA-VL comprises the amino acid sequence of SEQ ID NO: 128.

3. The antigen-binding molecule according to claim 1 or 2, wherein
the antigen-binding moiety that specifically binds to CD3 comprises a heavy chain variable region CD3-VH and a light chain variable region CD3-VL, wherein CD3-HCDR1, CD3-HCDR2, and CD3-HCDR3 in the CD3-VH comprises the amino acid sequences of CD3-HCDR1, CD3-HCDR2, and CD3-HCDR3 in SEQ ID NO: 166, respectively; and CD3-LCDR1, CD3-LCDR2, and CD3-LCDR3 in the CD3-VL comprises the amino acid sequences of CD3-LCDR1, CD3-LCDR2, and CD3-LCDR3 in SEQ ID NO: 167, respectively;
preferably,
the CD3-VH has: a CD3-HCDR1 comprising the amino acid sequence of SEQ ID NO: 160, a CD3-HCDR2 comprising the amino acid sequence of SEQ ID NO: 161, and a CD3-HCDR3 comprising the amino acid sequence of SEQ ID NO: 162; and the CD3-VL has: a CD3-LCDR1 comprising the amino acid sequence of SEQ ID NO: 163, a CD3-LCDR2 comprising the amino acid sequence of SEQ ID NO: 164, and a CD3-LCDR3 comprising the amino acid sequence of SEQ ID NO: 165;
more preferably,
the CD3-VH comprises the amino acid sequence of SEQ ID NO: 166, and the CD3-VL comprises the amino acid sequence of SEQ ID NO: 167.

4. The antigen-binding molecule according to any one of claims 1 to 3, wherein the antigen-binding molecule comprises an Fc region; the Fc region is preferably an IgG Fc region, and the Fc region is more preferably an IgG₁ Fc region;
preferably, the Fc region comprises one or more amino acid substitutions capable of reducing binding of the Fc region to Fcy receptors;
more preferably, the Fc region is a human IgG₁ Fc region, and the amino acid residues at positions 234 and 235 are A, as numbered according to the EU index.

5. The antigen-binding molecule according to any one of claims 1 to 4, wherein the antigen-binding molecule comprises an Fc region comprising a first subunit Fc1 and a second subunit Fc2 capable of associating with each other, and the Fc1 and Fc2 each independently have one or more amino acid substitutions that reduce homodimerization of the Fc region;
preferably, the Fc1 has a knob structure according to the knob-into-hole technique, and the Fc2 has a hole structure according to the knob-into-hole technique; or
the Fc2 has a knob structure according to the knob-into-hole technique, and the Fc1 has a hole structure according to the knob-into-hole technique;
more preferably,
1) the amino acid residue at position 366 of the Fc1 is W; and
the amino acid residue at position 366 of the Fc2 is S, the amino acid residue at position 368 is A, and the amino acid residue at position 407 is V, as numbered according to the EU index; or
2) the amino acid residue at position 366 of the Fc2 is W; and
the amino acid residue at position 366 of the Fc1 is S, the amino acid residue at position 368 is A, and the amino acid residue at position 407 is V, as numbered according to the EU index.

6. The antigen-binding molecule according to claim 5, wherein the antigen-binding molecule comprises:
one antigen-binding moiety that specifically binds to PSMA, and
one antigen-binding moiety that specifically binds to CD3;
the antigen-binding moiety that specifically binds to PSMA and the antigen-binding moiety that specifically binds to CD3 are scFvs;
preferably,
the antigen-binding molecule comprises:
one first chain having a structure represented by formula (a), and
one second chain having a structure represented by formula (b):
formula (a) [PSMA-VH]-[linker 1]-[PSMA-VL]-[linker 2]-[CD3-VH]-[linker 3]-[CD3-VL]-[linker 4]-[Fc2], and
formula (b) [Fc1];
the structures represented by formula (a) and formula (b) are arranged from N-terminus to C-terminus, and the linker 1, linker 2, linker 3, and linker 4 are identical or different peptide linkers;
more preferably,
the antigen-binding molecule has: one first chain comprising the amino acid sequence of SEQ ID NO: 192 and one second chain comprising the amino acid sequence of SEQ ID NO: 169; or
the antigen-binding molecule has: one first chain comprising the amino acid sequence of SEQ ID NO: 191 and one second chain comprising the amino acid sequence of SEQ ID NO: 169; or
the antigen-binding molecule has: one first chain comprising the amino acid sequence of SEQ ID NO: 198 and one second chain comprising the amino acid sequence of SEQ ID NO: 169.

7. The antigen-binding molecule according to any one of claims 1 to 5, wherein the antigen-binding moiety that specifically binds to PSMA or the antigen-binding moiety that specifically binds to CD3 each independently comprises a Titin chain and an Obscurin chain;
preferably,
the Titin chain comprises an amino acid sequence selected from the group consisting of SEQ ID NO: 210 to SEQ ID NO: 228, and the Obscurin chain comprises an amino acid sequence selected from the group consisting of SEQ ID NO: 229 to SEQ ID NO: 269;
more preferably,
the Titin chain comprises the amino acid sequence of SEQ ID NO: 226, and the Obscurin chain comprises the amino acid sequence of SEQ ID NO: 264.

8. The antigen-binding molecule according to claim 7, wherein the antigen-binding molecule comprises one antigen-binding moiety that specifically binds to PSMA and one antigen-binding moiety that specifically binds to CD3;
the antigen-binding moiety that specifically binds to PSMA is a Fab;
the antigen-binding moiety that specifically binds to CD3 is a substituted Fab comprising a Titin chain and an Obscurin chain;
preferably,
the antigen-binding molecule comprises one first chain having a structure represented by formula (c), one second chain having a structure represented by formula (d), one third chain having a structure represented by formula (e), and one fourth chain having a structure represented by formula (f):
formula (c) [PSMA-VH]-[CH1]-[Fc1],
formula (d) [PSMA-VL]-[CL],
formula (e) [CD3-VH]-[linker 5]-[Obscurin chain]-[Fc2], and
formula (f) [CD3-VL]-[linker 6]-[Titin chain];
the structures represented by formulas (c), (d), (e), and (f) are arranged from N-terminus to C-terminus, and the linker 5 and the linker 6 are identical or different peptide linkers;
more preferably,
the antigen-binding molecule has: one first chain comprising the amino acid sequence of SEQ ID NO: 201, one second chain comprising the amino acid sequence of SEQ ID NO: 202, one third chain comprising the amino acid sequence of SEQ ID NO: 171, and one fourth chain comprising the amino acid sequence of SEQ ID NO: 172, or
the antigen-binding molecule has: one first chain comprising the amino acid sequence of SEQ ID NO: 193, one second chain comprising the amino acid sequence of SEQ ID NO: 194, one third chain comprising the amino acid sequence of SEQ ID NO: 171, and one fourth chain comprising the amino acid sequence of SEQ ID NO: 172, or
the antigen-binding molecule has: one first chain comprising the amino acid sequence of SEQ ID NO: 206, one second chain comprising the amino acid sequence of SEQ ID NO: 207, one third chain comprising the amino acid sequence of SEQ ID NO: 171, and one fourth chain comprising the amino acid sequence of SEQ ID NO: 172, or
the antigen-binding molecule has: one first chain comprising the amino acid sequence of SEQ ID NO: 284, one second chain comprising the amino acid sequence of SEQ ID NO: 285, one third chain comprising the amino acid sequence of SEQ ID NO: 171, and one fourth chain comprising the amino acid sequence of SEQ ID NO: 172.

9. The antigen-binding molecule according to claim 7, wherein the antigen-binding molecule comprises two antigen-binding moieties that specifically bind to PSMA and one antigen-binding moiety that specifically binds to CD3;
the antigen-binding moieties that specifically bind to PSMA are Fabs; the antigen-binding moiety that specifically binds to CD3 is a substituted Fab comprising a Titin chain and an Obscurin chain;
preferably,
the antigen-binding molecule comprises one first chain having a structure represented by formula (c), two second chains having a structure represented by formula (d), one third chain having a structure represented by formula (g), and one fourth chain having a structure represented by formula (h):
formula (c) [PSMA-VH]-[CH1]-[Fc1],
formula (d) [PSMA-VL]-[CL],
formula (g) [PSMA-VH]-[CH1]-[CD3-VH]-[linker 7]-[Titin chain]-[Fc2], and
formula (h) [CD3-VL]-[linker 8]-[Obscurin chain];
the structures represented by formulas (c), (d), (g), and (h) are arranged from N-terminus to C-terminus, and the linker 7 and the linker 8 are identical or different peptide linkers;
more preferably,
the antigen-binding molecule has: one first chain comprising the amino acid sequence of SEQ ID NO: 180, two second chains comprising the amino acid sequence of SEQ ID NO: 181, one third chain comprising the amino acid sequence of SEQ ID NO: 182, and one fourth chain comprising the amino acid sequence of SEQ ID NO: 174, or
the antigen-binding molecule has: one first chain comprising the amino acid sequence of SEQ ID NO: 187, two second chains comprising the amino acid sequence of SEQ ID NO: 188, one third chain comprising the amino acid sequence of SEQ ID NO: 189, and one fourth chain comprising the amino acid sequence of SEQ ID NO: 174, or
the antigen-binding molecule has: one first chain comprising the amino acid sequence of SEQ ID NO: 193, two second chains comprising the amino acid sequence of SEQ ID NO: 194, one third chain comprising the amino acid sequence of SEQ ID NO: 195, and one fourth chain comprising the amino acid sequence of SEQ ID NO: 174, or
the antigen-binding molecule has: one first chain comprising the amino acid sequence of SEQ ID NO: 206, two second chains comprising the amino acid sequence of SEQ ID NO: 207, one third chain comprising the amino acid sequence of SEQ ID NO: 208, and one fourth chain comprising the amino acid sequence of SEQ ID NO: 174.

10. The antigen-binding molecule according to claim 7, wherein the antigen-binding molecule comprises two antigen-binding moieties that specifically bind to PSMA and two antigen-binding moieties that specifically bind to CD3; the antigen-binding moieties that specifically bind to PSMA are Fabs, and the antigen-binding moieties that specifically bind to CD3 are substituted Fabs comprising a Titin chain and an Obscurin chain;
preferably,
the antigen-binding molecule comprises two first chains having a structure represented by formula (j), two second chains having a structure represented by formula (d), and two third chains having a structure represented by formula (h):
formula (j) [PSMA-VH]-[CH1]-[CD3-VH]-[finker 9]-[Titin chain]-[one subunit of Fc],
formula (d) [PSMA-VL]-[CL], and
formula (h) [CD3-VL]-[linker 8]-[Obscurin chain];
the structures represented by formulas (j), (d), and (h) are arranged from N-terminus to C-terminus, and the linker 8 and the linker 9 are identical or different peptide linkers;
more preferably,
the antigen-binding molecule has: two first chains comprising the amino acid sequence of SEQ ID NO: 183, two second chains comprising the amino acid sequence of SEQ ID NO: 181, and two third chains comprising the amino acid sequence of SEQ ID NO: 174, or
the antigen-binding molecule has: two first chains comprising the amino acid sequence of SEQ ID NO: 185, two second chains comprising the amino acid sequence of SEQ ID NO: 186, and two third chains comprising the amino acid sequence of SEQ ID NO: 174, or
the antigen-binding molecule has: two first chains comprising the amino acid sequence of SEQ ID NO: 196, two second chains comprising the amino acid sequence of SEQ ID NO: 194, and two third chains comprising the amino acid sequence of SEQ ID NO: 174, or
the antigen-binding molecule has: two first chains comprising the amino acid sequence of SEQ ID NO: 203, two second chains comprising the amino acid sequence of SEQ ID NO: 204, and two third chains comprising the amino acid sequence of SEQ ID NO: 174, or
the antigen-binding molecule has: two first chains comprising the amino acid sequence of SEQ ID NO: 286, two second chains comprising the amino acid sequence of SEQ ID NO: 285, and two third chains comprising the amino acid sequence of SEQ ID NO: 174.

11. The antigen-binding molecule according to any one of claims 1 to 4, wherein the antigen-binding molecule comprises two antigen-binding moieties that specifically bind to PSMA and two antigen-binding moieties that specifically bind to CD3; the antigen-binding moieties that specifically bind to PSMA are Fabs, and the antigen-binding moieties that specifically bind to CD3 are scFvs;
preferably,
the antigen-binding molecule comprises two first chains having a structure represented by formula (k) and two second chains having a structure represented by formula (d):
formula (k) [PSMA-VH]-[CH1]-[CD3-VH]-[linker 10]-[CD3-VL]-[linker 11]-[one subunit of Fc], and
formula (d) [PSMA-VL]-[CL];
the structures represented by formulas (k) and (d) are arranged from N-terminus to C-terminus, and the linker 10 and the linker 11 are identical or different peptide linkers;
more preferably,
the antigen-binding molecule has: two first chains comprising the amino acid sequence of SEQ ID NO: 184 and two second chains comprising the amino acid sequence of SEQ ID NO: 181, or
the antigen-binding molecule has: two first chains comprising the amino acid sequence of SEQ ID NO: 190 and two second chains comprising the amino acid sequence of SEQ ID NO: 188, or
the antigen-binding molecule has: two first chains comprising the amino acid sequence of SEQ ID NO: 197 and two second chains comprising the amino acid sequence of SEQ ID NO: 194, or
the antigen-binding molecule has: two first chains comprising the amino acid sequence of SEQ ID NO: 199 and two second chains comprising the amino acid sequence of SEQ ID NO: 200, or
the antigen-binding molecule has: two first chains comprising the amino acid sequence of SEQ ID NO: 205 and two second chains comprising the amino acid sequence of SEQ ID NO: 204, or
the antigen-binding molecule has: two first chains comprising the amino acid sequence of SEQ ID NO: 209 and two second chains comprising the amino acid sequence of SEQ ID NO: 207.

12. An isolated antibody capable of specifically binding to PSMA, wherein the antibody comprises a heavy chain variable region PSMA-VH and a light chain variable region PSMA-VL, wherein
(i) PSMA-HCDR1, PSMA-HCDR2, and PSMA-HCDR3 in the PSMA-VH comprise the amino acid sequences of PSMA-HCDR1, PSMA-HCDR2, and PSMA-HCDR3 in SEQ ID NO: 77, respectively, and PSMA-LCDR1, PSMA-LCDR2, and PSMA-LCDR3 in the PSMA-VL comprise the amino acid sequences of PSMA-LCDR1, PSMA-LCDR2, and PSMA-LCDR3 in SEQ ID NO: 78, respectively, or
(ii) PSMA-HCDR1, PSMA-HCDR2, and PSMA-HCDR3 in the PSMA-VH comprise the amino acid sequences of PSMA-HCDR1, PSMA-HCDR2, and PSMA-HCDR3 in SEQ ID NO: 73, respectively, and PSMA-LCDR1, PSMA-LCDR2, and PSMA-LCDR3 in the PSMA-VL comprise the amino acid sequences of PSMA-LCDR1, PSMA-LCDR2, and PSMA-LCDR3 in SEQ ID NO: 74, respectively, or
(iii) PSMA-HCDR1, PSMA-HCDR2, and PSMA-HCDR3 in the PSMA-VH comprise the amino acid sequences of PSMA-HCDR1, PSMA-HCDR2, and PSMA-HCDR3 in SEQ ID NO: 75, respectively, and PSMA-LCDR1, PSMA-LCDR2, and PSMA-LCDR3 in the PSMA-VL comprise the amino acid sequences of PSMA-LCDR1, PSMA-LCDR2, and PSMA-LCDR3 in SEQ ID NO: 76, respectively, or
(iv) PSMA-HCDR1, PSMA-HCDR2, and PSMA-HCDR3 in the PSMA-VH comprise the amino acid sequences of PSMA-HCDR1, PSMA-HCDR2, and PSMA-HCDR3 in SEQ ID NO: 79, respectively, and PSMA-LCDR1, PSMA-LCDR2, and PSMA-LCDR3 in the PSMA-VL comprise the amino acid sequences of PSMA-LCDR1, PSMA-LCDR2, and PSMA-LCDR3 in SEQ ID NO: 80, respectively, or
(v) PSMA-HCDR1, PSMA-HCDR2, and PSMA-HCDR3 in the PSMA-VH comprise the amino acid sequences of PSMA-HCDR1, PSMA-HCDR2, and PSMA-HCDR3 in SEQ ID NO: 81, respectively, and PSMA-LCDR1, PSMA-LCDR2, and PSMA-LCDR3 in the PSMA-VL comprise the amino acid sequences of PSMA-LCDR1, PSMA-LCDR2, and PSMA-LCDR3 in SEQ ID NO: 82, respectively, or
(vi) PSMA-HCDR1, PSMA-HCDR2, and PSMA-HCDR3 in the PSMA-VH comprise the amino acid sequences of PSMA-HCDR1, PSMA-HCDR2, and PSMA-HCDR3 in SEQ ID NO: 83, respectively, and PSMA-LCDR1, PSMA-LCDR2, and PSMA-LCDR3 in the PSMA-VL comprise the amino acid sequences of PSMA-LCDR1, PSMA-LCDR2, and PSMA-LCDR3 in SEQ ID NO: 84, respectively, or
(vii) PSMA-HCDR1, PSMA-HCDR2, and PSMA-HCDR3 in the PSMA-VH comprise the amino acid sequences of PSMA-HCDR1, PSMA-HCDR2, and PSMA-HCDR3 in SEQ ID NO: 85, respectively, and PSMA-LCDR1, PSMA-LCDR2, and PSMA-LCDR3 in the PSMA-VL comprise the amino acid sequences of PSMA-LCDR1, PSMA-LCDR2, and PSMA-LCDR3 in SEQ ID NO: 86, respectively, or
(viii) PSMA-HCDR1, PSMA-HCDR2, and PSMA-HCDR3 in the PSMA-VH comprise the amino acid sequences of PSMA-HCDR1, PSMA-HCDR2, and PSMA-HCDR3 in SEQ ID NO: 87, respectively, and PSMA-LCDR1, PSMA-LCDR2, and PSMA-LCDR3 in the PSMA-VL comprise the amino acid sequences of PSMA-LCDR1, PSMA-LCDR2, and PSMA-LCDR3 in SEQ ID NO: 88, respectively, or
(ix) PSMA-HCDR1, PSMA-HCDR2, and PSMA-HCDR3 in the PSMA-VH comprise the amino acid sequences of PSMA-HCDR1, PSMA-HCDR2, and PSMA-HCDR3 in SEQ ID NO: 89, respectively, and PSMA-LCDR1, PSMA-LCDR2, and PSMA-LCDR3 in the PSMA-VL comprise the amino acid sequences of PSMA-LCDR1, PSMA-LCDR2, and PSMA-LCDR3 in SEQ ID NO: 90, respectively, or
(x) PSMA-HCDR1, PSMA-HCDR2, and PSMA-HCDR3 in the PSMA-VH comprise the amino acid sequences of PSMA-HCDR1, PSMA-HCDR2, and PSMA-HCDR3 in SEQ ID NO: 91, respectively, and PSMA-LCDR1, PSMA-LCDR2, and PSMA-LCDR3 in the PSMA-VL comprise the amino acid sequences of PSMA-LCDR1, PSMA-LCDR2, and PSMA-LCDR3 in SEQ ID NO: 92 or 152, respectively, or
(xi) PSMA-HCDR1, PSMA-HCDR2, and PSMA-HCDR3 in the PSMA-VH comprise the amino acid sequences of PSMA-HCDR1, PSMA-HCDR2, and PSMA-HCDR3 in SEQ ID NO: 93, respectively, and PSMA-LCDR1, PSMA-LCDR2, and PSMA-LCDR3 in the PSMA-VL comprise the amino acid sequences of PSMA-LCDR1, PSMA-LCDR2, and PSMA-LCDR3 in SEQ ID NO: 94, respectively, or
(xii) PSMA-HCDR1, PSMA-HCDR2, and PSMA-HCDR3 in the PSMA-VH comprise the amino acid sequences of PSMA-HCDR1, PSMA-HCDR2, and PSMA-HCDR3 in SEQ ID NO: 95, respectively, and PSMA-LCDR1, PSMA-LCDR2, and PSMA-LCDR3 in the PSMA-VL comprise the amino acid sequences of PSMA-LCDR1, PSMA-LCDR2, and PSMA-LCDR3 in SEQ ID NO: 96, respectively;
preferably,
(i) the PSMA-VH has: a PSMA-HCDR1 comprising the amino acid sequence of SEQ ID NO: 13, a PSMA-HCDR2 comprising the amino acid sequence of SEQ ID NO: 14, and a PSMA-HCDR3 comprising the amino acid sequence of SEQ ID NO: 15, and the PSMA-VL has: a PSMA-LCDR1 comprising the amino acid sequence of SEQ ID NO: 16, a PSMA-LCDR2 comprising the amino acid sequence of SEQ ID NO: 17, and a PSMA-LCDR3 comprising the amino acid sequence of SEQ ID NO: 18, or
(ii) the PSMA-VH has: a PSMA-HCDR1 comprising the amino acid sequence of SEQ ID NO: 1, a PSMA-HCDR2 comprising the amino acid sequence of SEQ ID NO: 2, and a PSMA-HCDR3 comprising the amino acid sequence of SEQ ID NO: 3, and the PSMA-VL has: a PSMA-LCDR1 comprising the amino acid sequence of SEQ ID NO: 4, a PSMA-LCDR2 comprising the amino acid sequence of SEQ ID NO: 5, and a PSMA-LCDR3 comprising the amino acid sequence of SEQ ID NO: 6, or
(iii) the PSMA-VH has: a PSMA-HCDR1 comprising the amino acid sequence of SEQ ID NO: 7, a PSMA-HCDR2 comprising the amino acid sequence of SEQ ID NO: 8, and a PSMA-HCDR3 comprising the amino acid sequence of SEQ ID NO: 9, and the PSMA-VL has: a PSMA-LCDR1 comprising the amino acid sequence of SEQ ID NO: 10, a PSMA-LCDR2 comprising the amino acid sequence of SEQ ID NO: 11, and a PSMA-LCDR3 comprising the amino acid sequence of SEQ ID NO: 12, or
(iv) the PSMA-VH has: a PSMA-HCDR1 comprising the amino acid sequence of SEQ ID NO: 19, a PSMA-HCDR2 comprising the amino acid sequence of SEQ ID NO: 20, and a PSMA-HCDR3 comprising the amino acid sequence of SEQ ID NO: 21, and the PSMA-VL has: a PSMA-LCDR1 comprising the amino acid sequence of SEQ ID NO: 22, a PSMA-LCDR2 comprising the amino acid sequence of SEQ ID NO: 23, and a PSMA-LCDR3 comprising the amino acid sequence of SEQ ID NO: 24, or
(v) the PSMA-VH has: a PSMA-HCDR1 comprising the amino acid sequence of SEQ ID NO: 25, a PSMA-HCDR2 comprising the amino acid sequence of SEQ ID NO: 26, and a PSMA-HCDR3 comprising the amino acid sequence of SEQ ID NO: 27, and the PSMA-VL has: a PSMA-LCDR1 comprising the amino acid sequence of SEQ ID NO: 28, a PSMA-LCDR2 comprising the amino acid sequence of SEQ ID NO: 29, and a PSMA-LCDR3 comprising the amino acid sequence of SEQ ID NO: 30, or
(vi) the PSMA-VH has: a PSMA-HCDR1 comprising the amino acid sequence of SEQ ID NO: 31, a PSMA-HCDR2 comprising the amino acid sequence of SEQ ID NO: 32, and a PSMA-HCDR3 comprising the amino acid sequence of SEQ ID NO: 33, and the PSMA-VL has: a PSMA-LCDR1 comprising the amino acid sequence of SEQ ID NO: 34, a PSMA-LCDR2 comprising the amino acid sequence of SEQ ID NO: 35, and a PSMA-LCDR3 comprising the amino acid sequence of SEQ ID NO: 36, or
(vii) the PSMA-VH has: a PSMA-HCDR1 comprising the amino acid sequence of SEQ ID NO: 37, a PSMA-HCDR2 comprising the amino acid sequence of SEQ ID NO: 38, and a PSMA-HCDR3 comprising the amino acid sequence of SEQ ID NO: 39, and the PSMA-VL has: a PSMA-LCDR1 comprising the amino acid sequence of SEQ ID NO: 40, a PSMA-LCDR2 comprising the amino acid sequence of SEQ ID NO: 41, and a PSMA-LCDR3 comprising the amino acid sequence of SEQ ID NO: 42, or
(viii) the PSMA-VH has: a PSMA-HCDR1 comprising the amino acid sequence of SEQ ID NO: 43, a PSMA-HCDR2 comprising the amino acid sequence of SEQ ID NO: 44, and a PSMA-HCDR3 comprising the amino acid sequence of SEQ ID NO: 45, and the PSMA-VL has: a PSMA-LCDR1 comprising the amino acid sequence of SEQ ID NO: 46, a PSMA-LCDR2 comprising the amino acid sequence of SEQ ID NO: 47, and a PSMA-LCDR3 comprising the amino acid sequence of SEQ ID NO: 48, or
(ix) the PSMA-VH has: a PSMA-HCDR1 comprising the amino acid sequence of SEQ ID NO: 49, a PSMA-HCDR2 comprising the amino acid sequence of SEQ ID NO: 50, and a PSMA-HCDR3 comprising the amino acid sequence of SEQ ID NO: 51, and the PSMA-VL has: a PSMA-LCDR1 comprising the amino acid sequence of SEQ ID NO: 52, a PSMA-LCDR2 comprising the amino acid sequence of SEQ ID NO: 53, and a PSMA-LCDR3 comprising the amino acid sequence of SEQ ID NO: 54, or
(x) the PSMA-VH has: a PSMA-HCDR1 comprising the amino acid sequence of SEQ ID NO: 55, a PSMA-HCDR2 comprising the amino acid sequence of SEQ ID NO: 56, and a PSMA-HCDR3 comprising the amino acid sequence of SEQ ID NO: 57, and the PSMA-VL has: a PSMA-LCDR1 comprising the amino acid sequence of SEQ ID NO: 58 or 159, a PSMA-LCDR2 comprising the amino acid sequence of SEQ ID NO: 59, and a PSMA-LCDR3 comprising the amino acid sequence of SEQ ID NO: 60, or
(xi) the PSMA-VH has: a PSMA-HCDR1 comprising the amino acid sequence of SEQ ID NO: 61, a PSMA-HCDR2 comprising the amino acid sequence of SEQ ID NO: 62, and a PSMA-HCDR3 comprising the amino acid sequence of SEQ ID NO: 63, and the PSMA-VL has: a PSMA-LCDR1 comprising the amino acid sequence of SEQ ID NO: 64, a PSMA-LCDR2 comprising the amino acid sequence of SEQ ID NO: 65, and a PSMA-LCDR3 comprising the amino acid sequence of SEQ ID NO: 66, or
(xii) the PSMA-VH has: a PSMA-HCDR1 comprising the amino acid sequence of SEQ ID NO: 67, a PSMA-HCDR2 comprising the amino acid sequence of SEQ ID NO: 68, and a PSMA-HCDR3 comprising the amino acid sequence of SEQ ID NO: 69, and the PSMA-VL has: a PSMA-LCDR1 comprising the amino acid sequence of SEQ ID NO: 70, a PSMA-LCDR2 comprising the amino acid sequence of SEQ ID NO: 71, and a PSMA-LCDR3 comprising the amino acid sequence of SEQ ID NO: 72;
more preferably,
the antibody binds to human PSMA at 25 °C with a KD of less than 1 × 10⁻⁸ M, as measured by surface plasmon resonance.

13. The isolated antibody according to claim 12, wherein
(i) the PSMA-VH comprises the amino acid sequence of SEQ ID NO: 112, 110, 77, 107, 108, 109, or 111, and the PSMA-VL comprises the amino acid sequence of SEQ ID NO: 115, 114, 78, or 113, or
(ii) the PSMA-VH comprises the amino acid sequence of SEQ ID NO: 73 or 99, and the PSMA-VL comprises the amino acid sequence of SEQ ID NO: 74 or 100, or
(iii) the PSMA-VH comprises the amino acid sequence of SEQ ID NO: 75, 101, 102, or 103, and the PSMA-VL comprises the amino acid sequence of SEQ ID NO: 76, 104, 105, or 106, or
(iv) the PSMA-VH comprises the amino acid sequence of SEQ ID NO: 79, 116, or 117, and the PSMA-VL comprises the amino acid sequence of SEQ ID NO: 80, 118, or 119, or
(v) the PSMA-VH comprises the amino acid sequence of SEQ ID NO: 81 or 120, and the PSMA-VL comprises the amino acid sequence of SEQ ID NO: 82, 121, 122, 123, 124, 125, or 126, or
(vi) the PSMA-VH comprises the amino acid sequence of SEQ ID NO: 127 or 83, and the PSMA-VL comprises the amino acid sequence of SEQ ID NO: 128, 84, 129, or 130, or
(vii) the PSMA-VH comprises the amino acid sequence of SEQ ID NO: 85, 131, or 132, and the PSMA-VL comprises the amino acid sequence of SEQ ID NO: 86, 133, 134, or 135, or
(viii) the PSMA-VH comprises the amino acid sequence of SEQ ID NO: 87, 136, 137, or 138, and the PSMA-VL comprises the amino acid sequence of SEQ ID NO: 88, 139, or 140, or
(ix) the PSMA-VH comprises the amino acid sequence of SEQ ID NO: 89, 141, 142, 143, or 144, and the PSMA-VL comprises the amino acid sequence of SEQ ID NO: 90, 145, 146, or 147, or
(x) the PSMA-VH comprises the amino acid sequence of SEQ ID NO: 91, 148, or 149, and the PSMA-VL comprises the amino acid sequence of SEQ ID NO: 92, 150, 151, or 152, or
(xi) the PSMA-VH comprises the amino acid sequence of SEQ ID NO: 93, 153, 154, 155, or 156, and the PSMA-VL comprises the amino acid sequence of SEQ ID NO: 94, 157, or 158, or
(xii) the PSMA-VH comprises the amino acid sequence of SEQ ID NO: 95, 277, 278, or 279, and the PSMA-VL comprises the amino acid sequence of SEQ ID NO: 96, 280, 281, 282, or 283;
preferably,
(i) the PSMA-VH comprises the amino acid sequence of SEQ ID NO: 112, and the PSMA-VL comprises the amino acid sequence of SEQ ID NO: 115, or
the PSMA-VH comprises the amino acid sequence of SEQ ID NO: 110, and the PSMA-VL comprises the amino acid sequence of SEQ ID NO: 114, or
the PSMA-VH comprises the amino acid sequence of SEQ ID NO: 77, and the PSMA-VL comprises the amino acid sequence of SEQ ID NO: 78, or
the PSMA-VH comprises the amino acid sequence of SEQ ID NO: 107, and the PSMA-VL comprises the amino acid sequence of SEQ ID NO: 113, or
the PSMA-VH comprises the amino acid sequence of SEQ ID NO: 108, and the PSMA-VL comprises the amino acid sequence of SEQ ID NO: 113, or
the PSMA-VH comprises the amino acid sequence of SEQ ID NO: 109, and the PSMA-VL comprises the amino acid sequence of SEQ ID NO: 113, or
the PSMA-VH comprises the amino acid sequence of SEQ ID NO: 110, and the PSMA-VL comprises the amino acid sequence of SEQ ID NO: 113, or
the PSMA-VH comprises the amino acid sequence of SEQ ID NO: 111, and the PSMA-VL comprises the amino acid sequence of SEQ ID NO: 113, or
the PSMA-VH comprises the amino acid sequence of SEQ ID NO: 107, and the PSMA-VL comprises the amino acid sequence of SEQ ID NO: 114, or
the PSMA-VH comprises the amino acid sequence of SEQ ID NO: 108, and the PSMA-VL comprises the amino acid sequence of SEQ ID NO: 114, or
the PSMA-VH comprises the amino acid sequence of SEQ ID NO: 109, and the PSMA-VL comprises the amino acid sequence of SEQ ID NO: 114, or
the PSMA-VH comprises the amino acid sequence of SEQ ID NO: 111, and the PSMA-VL comprises the amino acid sequence of SEQ ID NO: 114, or
(ii) the PSMA-VH comprises the amino acid sequence of SEQ ID NO: 73, and the PSMA-VL comprises the amino acid sequence of SEQ ID NO: 74, or
the PSMA-VH comprises the amino acid sequence of SEQ ID NO: 99, and the PSMA-VL comprises the amino acid sequence of SEQ ID NO: 100, or
(iii) the PSMA-VH comprises the amino acid sequence of SEQ ID NO: 75, and the PSMA-VL comprises the amino acid sequence of SEQ ID NO: 76, or
the PSMA-VH comprises the amino acid sequence of SEQ ID NO: 101, and the PSMA-VL comprises the amino acid sequence of SEQ ID NO: 104, or
the PSMA-VH comprises the amino acid sequence of SEQ ID NO: 102, and the PSMA-VL comprises the amino acid sequence of SEQ ID NO: 104, or
the PSMA-VH comprises the amino acid sequence of SEQ ID NO: 103, and the PSMA-VL comprises the amino acid sequence of SEQ ID NO: 104, or
the PSMA-VH comprises the amino acid sequence of SEQ ID NO: 101, and the PSMA-VL comprises the amino acid sequence of SEQ ID NO: 105, or
the PSMA-VH comprises the amino acid sequence of SEQ ID NO: 102, and the PSMA-VL comprises the amino acid sequence of SEQ ID NO: 105, or
the PSMA-VH comprises the amino acid sequence of SEQ ID NO: 103, and the PSMA-VL comprises the amino acid sequence of SEQ ID NO: 105, or
the PSMA-VH comprises the amino acid sequence of SEQ ID NO: 101, and the PSMA-VL comprises the amino acid sequence of SEQ ID NO: 106, or
the PSMA-VH comprises the amino acid sequence of SEQ ID NO: 102, and the PSMA-VL comprises the amino acid sequence of SEQ ID NO: 106, or
the PSMA-VH comprises the amino acid sequence of SEQ ID NO: 103, and the PSMA-VL comprises the amino acid sequence of SEQ ID NO: 106, or
(iv) the PSMA-VH comprises the amino acid sequence of SEQ ID NO: 79, and the PSMA-VL comprises the amino acid sequence of SEQ ID NO: 80, or
the PSMA-VH comprises the amino acid sequence of SEQ ID NO: 116, and the PSMA-VL comprises the amino acid sequence of SEQ ID NO: 118, or
the PSMA-VH comprises the amino acid sequence of SEQ ID NO: 117, and the PSMA-VL comprises the amino acid sequence of SEQ ID NO: 118, or
the PSMA-VH comprises the amino acid sequence of SEQ ID NO: 116, and the PSMA-VL comprises the amino acid sequence of SEQ ID NO: 119, or
the PSMA-VH comprises the amino acid sequence of SEQ ID NO: 117, and the PSMA-VL comprises the amino acid sequence of SEQ ID NO: 119, or
(v) the PSMA-VH comprises the amino acid sequence of SEQ ID NO: 81, and the PSMA-VL comprises the amino acid sequence of SEQ ID NO: 82, or
the PSMA-VH comprises the amino acid sequence of SEQ ID NO: 120, and the PSMA-VL comprises the amino acid sequence of SEQ ID NO: 121, or
the PSMA-VH comprises the amino acid sequence of SEQ ID NO: 120, and the PSMA-VL comprises the amino acid sequence of SEQ ID NO: 122, or
the PSMA-VH comprises the amino acid sequence of SEQ ID NO: 120, and the PSMA-VL comprises the amino acid sequence of SEQ ID NO: 123, or
the PSMA-VH comprises the amino acid sequence of SEQ ID NO: 120, and the PSMA-VL comprises the amino acid sequence of SEQ ID NO: 124, or
the PSMA-VH comprises the amino acid sequence of SEQ ID NO: 120, and the PSMA-VL comprises the amino acid sequence of SEQ ID NO: 125, or
the PSMA-VH comprises the amino acid sequence of SEQ ID NO: 120, and the PSMA-VL comprises the amino acid sequence of SEQ ID NO: 126, or
(vi) the PSMA-VH comprises the amino acid sequence of SEQ ID NO: 83, and the PSMA-VL comprises the amino acid sequence of SEQ ID NO: 84, or
the PSMA-VH comprises the amino acid sequence of SEQ ID NO: 127, and the PSMA-VL comprises the amino acid sequence of SEQ ID NO: 128, or
the PSMA-VH comprises the amino acid sequence of SEQ ID NO: 127, and the PSMA-VL comprises the amino acid sequence of SEQ ID NO: 129, or
the PSMA-VH comprises the amino acid sequence of SEQ ID NO: 127, and the PSMA-VL comprises the amino acid sequence of SEQ ID NO: 130, or
(vii) the PSMA-VH comprises the amino acid sequence of SEQ ID NO: 85, and the PSMA-VL comprises the amino acid sequence of SEQ ID NO: 86, or
the PSMA-VH comprises the amino acid sequence of SEQ ID NO: 131, and the PSMA-VL comprises the amino acid sequence of SEQ ID NO: 133, or
the PSMA-VH comprises the amino acid sequence of SEQ ID NO: 131, and the PSMA-VL comprises the amino acid sequence of SEQ ID NO: 134, or
the PSMA-VH comprises the amino acid sequence of SEQ ID NO: 131, and the PSMA-VL comprises the amino acid sequence of SEQ ID NO: 135, or
the PSMA-VH comprises the amino acid sequence of SEQ ID NO: 132, and the PSMA-VL comprises the amino acid sequence of SEQ ID NO: 133, or
the PSMA-VH comprises the amino acid sequence of SEQ ID NO: 132, and the PSMA-VL comprises the amino acid sequence of SEQ ID NO: 134, or
the PSMA-VH comprises the amino acid sequence of SEQ ID NO: 132, and the PSMA-VL comprises the amino acid sequence of SEQ ID NO: 135, or
(viii) the PSMA-VH comprises the amino acid sequence of SEQ ID NO: 87, and the PSMA-VL comprises the amino acid sequence of SEQ ID NO: 88, or
the PSMA-VH comprises the amino acid sequence of SEQ ID NO: 136, and the PSMA-VL comprises the amino acid sequence of SEQ ID NO: 139, or
the PSMA-VH comprises the amino acid sequence of SEQ ID NO: 137, and the PSMA-VL comprises the amino acid sequence of SEQ ID NO: 139, or
the PSMA-VH comprises the amino acid sequence of SEQ ID NO: 138, and the PSMA-VL comprises the amino acid sequence of SEQ ID NO: 139, or
the PSMA-VH comprises the amino acid sequence of SEQ ID NO: 138, and the PSMA-VL comprises the amino acid sequence of SEQ ID NO: 140, or
(ix) the PSMA-VH comprises the amino acid sequence of SEQ ID NO: 89, and the PSMA-VL comprises the amino acid sequence of SEQ ID NO: 90, or
the PSMA-VH comprises the amino acid sequence of SEQ ID NO: 141, and the PSMA-VL comprises the amino acid sequence of SEQ ID NO: 145, or
the PSMA-VH comprises the amino acid sequence of SEQ ID NO: 142, and the PSMA-VL comprises the amino acid sequence of SEQ ID NO: 145, or
the PSMA-VH comprises the amino acid sequence of SEQ ID NO: 143, and the PSMA-VL comprises the amino acid sequence of SEQ ID NO: 145, or
the PSMA-VH comprises the amino acid sequence of SEQ ID NO: 144, and the PSMA-VL comprises the amino acid sequence of SEQ ID NO: 145, or
the PSMA-VH comprises the amino acid sequence of SEQ ID NO: 141, and the PSMA-VL comprises the amino acid sequence of SEQ ID NO: 146, or
the PSMA-VH comprises the amino acid sequence of SEQ ID NO: 142, and the PSMA-VL comprises the amino acid sequence of SEQ ID NO: 146, or
the PSMA-VH comprises the amino acid sequence of SEQ ID NO: 143, and the PSMA-VL comprises the amino acid sequence of SEQ ID NO: 146, or
the PSMA-VH comprises the amino acid sequence of SEQ ID NO: 144, and the PSMA-VL comprises the amino acid sequence of SEQ ID NO: 146, or
the PSMA-VH comprises the amino acid sequence of SEQ ID NO: 141, and the PSMA-VL comprises the amino acid sequence of SEQ ID NO: 147, or
the PSMA-VH comprises the amino acid sequence of SEQ ID NO: 142, and the PSMA-VL comprises the amino acid sequence of SEQ ID NO: 147, or
the PSMA-VH comprises the amino acid sequence of SEQ ID NO: 143, and the PSMA-VL comprises the amino acid sequence of SEQ ID NO: 147, or
(x) the PSMA-VH comprises the amino acid sequence of SEQ ID NO: 91, and the PSMA-VL comprises the amino acid sequence of SEQ ID NO: 92, or
the PSMA-VH comprises the amino acid sequence of SEQ ID NO: 148, and the PSMA-VL comprises the amino acid sequence of SEQ ID NO: 150, or
the PSMA-VH comprises the amino acid sequence of SEQ ID NO: 149, and the PSMA-VL comprises the amino acid sequence of SEQ ID NO: 150, or
the PSMA-VH comprises the amino acid sequence of SEQ ID NO: 148, and the PSMA-VL comprises the amino acid sequence of SEQ ID NO: 151, or
the PSMA-VH comprises the amino acid sequence of SEQ ID NO: 149, and the PSMA-VL comprises the amino acid sequence of SEQ ID NO: 151, or
the PSMA-VH comprises the amino acid sequence of SEQ ID NO: 148, and the PSMA-VL comprises the amino acid sequence of SEQ ID NO: 152, or
the PSMA-VH comprises the amino acid sequence of SEQ ID NO: 149, and the PSMA-VL comprises the amino acid sequence of SEQ ID NO: 152, or
(xi) the PSMA-VH comprises the amino acid sequence of SEQ ID NO: 93, and the PSMA-VL comprises the amino acid sequence of SEQ ID NO: 94; or
the PSMA-VH comprises the amino acid sequence of SEQ ID NO: 153, and the PSMA-VL comprises the amino acid sequence of SEQ ID NO: 157; or
the PSMA-VH comprises the amino acid sequence of SEQ ID NO: 154, and the PSMA-VL comprises the amino acid sequence of SEQ ID NO: 157; or
the PSMA-VH comprises the amino acid sequence of SEQ ID NO: 155, and the PSMA-VL comprises the amino acid sequence of SEQ ID NO: 157; or
the PSMA-VH comprises the amino acid sequence of SEQ ID NO: 156, and the PSMA-VL comprises the amino acid sequence of SEQ ID NO: 157; or
the PSMA-VH comprises the amino acid sequence of SEQ ID NO: 153, and the PSMA-VL comprises the amino acid sequence of SEQ ID NO: 158; or
the PSMA-VH comprises the amino acid sequence of SEQ ID NO: 154, and the PSMA-VL comprises the amino acid sequence of SEQ ID NO: 158; or
the PSMA-VH comprises the amino acid sequence of SEQ ID NO: 155, and the PSMA-VL comprises the amino acid sequence of SEQ ID NO: 158; or
the PSMA-VH comprises the amino acid sequence of SEQ ID NO: 156, and the PSMA-VL comprises the amino acid sequence of SEQ ID NO: 158; or
(xii) the PSMA-VH comprises the amino acid sequence of SEQ ID NO: 95, and the PSMA-VL comprises the amino acid sequence of SEQ ID NO: 96;
more preferably,
(i) the PSMA-VH comprises the amino acid sequence of SEQ ID NO: 112, and the PSMA-VL comprises the amino acid sequence of SEQ ID NO: 115, or
the PSMA-VH comprises the amino acid sequence of SEQ ID NO: 110, and the PSMA-VL comprises the amino acid sequence of SEQ ID NO: 114, or
(vi) the PSMA-VH comprises the amino acid sequence of SEQ ID NO: 127, and the PSMA-VL comprises the amino acid sequence of SEQ ID NO: 128.

14. The isolated antibody according to claim 12 or 13, wherein the antibody is a bispecific antibody;
preferably, the bispecific antibody specifically binds to PSMA and CD3.

15. A pharmaceutical composition, comprising:
a therapeutically effective amount of the antigen-binding molecule according to any one of claims 1 to 11 or the isolated antibody according to any one of claims 12 to 14, and one or more pharmaceutically acceptable carriers, diluents, buffers, or excipients;
wherein preferably, the pharmaceutical composition also comprises at least one second therapeutic agent;
more preferably, the second therapeutic agent is an antibody capable of specifically binding to CD28.

16. An isolated nucleic acid, encoding the antigen-binding molecule according to any one of claims 1 to 11 or the isolated antibody according to any one of claims 12 to 14.

17. A host cell, comprising the isolated nucleic acid according to claim 16.

18. A method for treating a disease, wherein the method comprises:
a step of administering to a subject a therapeutically effective amount of the antigen-binding molecule according to any one of claims 1 to 11, or the isolated antibody according to any one of claims 12 to 14, or the pharmaceutical composition according to claim 15;
the disease is a proliferative disease, a tumor, or an immune disease;
preferably, the disease is selected from the group consisting of any one of the following: prostate cancer, lung cancer, endometrial cancer, kidney cancer, bladder cancer, colorectal cancer, and gastric cancer.
